# EUROPEAN PATENT APPLICATION

(11) **EP 2 581 376 A1**
(43) Date of publication of application: **17.04.2013**
(21) Application number: 11774274.2
(22) Date of filing: 29.04.2011
(51) Int. Cl.: C07D 471/16, C07D 471/22, A61K 31/4375, A61P 31/14

(54) **MATRINIC ACID/ MATRINE DERIVATIVES AND PREPARATION METHODS AND USES THEREOF**

(30) Priority: 30.04.2010 CN 201010160550
(71) Applicant: Institute of Medicinal Biotechnology, Chinese Academy of Medical Sciences, Beijing 100050 (CN)
(72) Inventor: JIANG, Jiandong, Beijing 100050 (CN); SONG, Danqing, Beijing 100050 (CN); DU, Nana, Beijing 100050 (CN); PENG, Zonggen, Beijing 100050 (CN); WANG, Yuping, Beijing 100050 (CN); GAO, Limei, Beijing 100050 (CN); HAN, Yanxing, Beijing 100050 (CN); LI, Xin, Beijing 100050 (CN); LI, Chunxin, Beijing 100050 (CN)
(74) Representative: Böhm, Brigitte
(86) International application number: PCT/CN2011/000762
(87) International publication number: WO 2011/134283

(57) **Abstract**

The present invention relates to a N-substituted matrinic acid derivative or matrine derivative, and its preparation method and uses. Specifically, the present invention relates to a compound of Formula (I) or (II) (wherein all the definitions of substituted groups are those mentioned in the specification), or a pharmaceutically acceptable salt, geometric isomer, stereoisomer, solvate, ester or prodrug thereof. The present invention further relates to a method for preparing the compound of the present invention, a pharmaceutical composition containing the compound, and uses thereof in manufacture of a medicament. The compound of the present invention can be used for prophylaxis and/or treatment of a disease or disorder associated with viral infection such as hepatitis B and/orhepatitis C and/orAIDS.

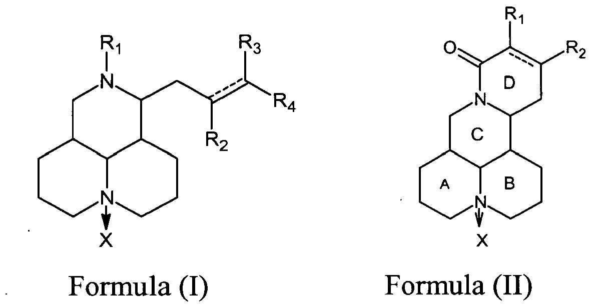

## Description

### Technical Field

The present invention relates to a group of novel compounds, specifically to matrinic acid/matrine derivatives, especially N-substituted matrinic acid and substituted matrine derivatives as well as preparation methods and uses thereof, particularly to their uses in prophylaxis and/or treatment of viral diseases, such as hepatitis B and/or hepatitis C and/or AIDS.

### Background Art

At the beginning of 1930s, the chemical components of a leguminous plant, *Sophora flavescens* Ait, were studied with the emphasis on alkaloids thereof. At present, in domestic researches, the extracted, separated, identified alkaloids are mainly Matrine (abbreviated in the text as MT, C₁₅H₂₄N₂O), Oxymatrine (also called as kurorinone, abbreviated in the text as OMT, C₁₅H₂₄N₂O₂). In addition, *Sophora flavescens* Ait further comprises some flavanoids such as kurarinol etc. The chemical structure formulas of matrine and oxymatrine are as follows:

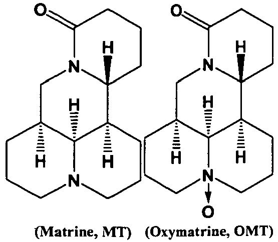

In the structure of matrine or oxymatrine, the bond linking carbonyl and nitrogen of the ring may subject to ring-opening under certain conditions to form matrinic acid or oxymatrinic acid. Some Japanese scholars synthesized oxymatrinic acid for researches related to pains in 1950s, and the structure formula of matrinic acid is as follows:

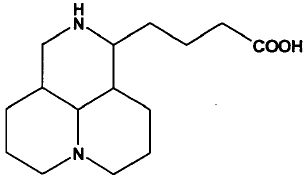

A disease associated with viral infection is one of the most serious diseases affecting human health. So far, although many antiviral drugs have been found, there is still in lack of effective clinical therapeutic regime. Hence, it is still in need to provide a novel antiviral drug for those skilled in the art.

### Contents of the Invention

The inventors use host heat shock homologous protein 70 (Hsc70) as action target, design a group of novel Hsc70 downregulating agents, i.e., N-substituted matrinic acid derivatives or matrine derivatives as well as analogs thereof. The compounds targeting Hsc70 have advantages of broad spectrum of antiviral activity, not prone to generation of drug resistance, and high safety. In the present invention, matrinic acid/matrine derivatives of Formula (I) or (II) at post-transcriptional level can down-regulate the gene expression of liver cell Hsc70, then exhibit good inhibition activity to viruses of hepatitis B and/or hepatitis C and AIDS, and thus can be used for prophylaxis and/or treatment of viral diseases such as hepatitis B and/or hepatitis C and/or AIDS. The present invention is fulfilled based on the above findings.

### Brief Description of the Invention:

Hence, in the first aspect, the present invention provides a compound of Formula (I) or Formula (II), or a pharmaceutically acceptable salt, geometric isomer, stereoisomer, solvate, ester or prodrug thereof, 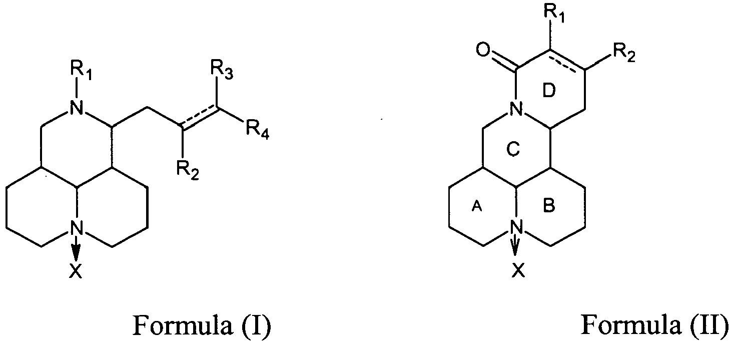 wherein:
X is unsubstituted or O;
R₁ is selected from
   (1)C₁₋₆ alkyl-CO-, C₂₋₆ alkenyl-CO-, aryl-C₁₋₆ alkyl-CO-, aryl-C₂₋₆ alkenyl-CO-, aryloxy-C₁₋₆ alkyl-CO-, or aryloxy-C₂₋₆ alkenyl-CO-, wherein the alkyl, alkenyl and aryl is optionally substituted with 1-3 groups independently selected from the group consisting of: hydroxyl, halogen, nitro and C₁₋₆alkyl,
   (2)C₁₋₆ alkyl-SO₂- or aryl-SO₂-, wherein the alkyl and aryl is optionally substituted with 1-3 groups independently selected from the group consisting of: hydroxyl, halogen, nitro and C₁₋₆alkyl,
   (3)one or two C₁₋₆ alkyl, or one or two C₂₋₆ alkenyl, wherein the alkyl and alkenyl are optionally substituted with 1-3 groups independently selected from the group consisting of: hydroxyl, halogen and C₁₋₆alkyl,
   (4) aryl-C₁₋₆ alkyl-, or heteroaryl-C₁₋₆ alkyl-, wherein the aryl and heteroaryl are optionally substituted with 1-3 groups independently selected from the group consisting of: C₁₋₆ alkyloxy, nitro, halogen, cyano, C₁₋₆ alkyl, C₂₋₆ alkenyl, and wherein the ring of the heteroaryl contains 1 or 2 heteroatoms selected from N, O and S,
   (5) aryl, which is optionally substituted with 1-3 groups independently selected from the group consisting of: C₁₋₆ alkyl, nitro, halogen and cyano,
   (6) C₁₋₁₂ alkyl, C₅₋₁₂ aryl, C₂₋₁₂ alkenyl, C₂₋₁₂ alkynyl, C₇₋₁₂ alkenylaryl, C₇-C₁₂ alkynylaryl, or C₆-C₁₂alkylaryl, which is optionally substituted with 1-3 groups independently selected from the group consisting of: hydroxyl, C₁₋₆ alkyloxy and halogen;
R₂ represents 1 or 2 groups selected from the group consisting of: -H, hydroxyl, C₁₋₁₂ alkyloxy, C₁₋₁₂ alkyl optionally substituted with nitro or amino, and C₂₋₁₂ ester group;
If present, R₃ represents 1 or 2 groups selected from the group consisting of: -H, hydroxyl, C₁₋₁₂ alkyloxy and C₂₋₁₂ ester group;
If present, R₄ is selected from the group consisting of: -COOH, -CHO, -CH=NOH, -CH=N-NH₂, hydroxyl-C₁₋₆ alkyl-, C₂-C₁₂ ester group, C₂-C₁₂ acylamino, C₂-C₁₂ ether group, C₂-C₁₂ oxime ether group; --------- represents single bond or double bond.
The compound according to any of the first aspect of the present invention is a compound of Formula (Ia), or a pharmaceutically acceptable salt, geometric isomer, stereoisomer, solvate, ester or prodrug thereof, 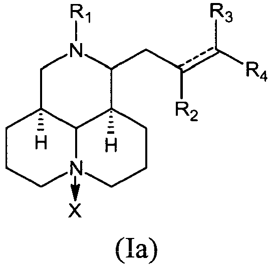 wherein:
   X, R₁, R₂, R₃ and R₄ have definitions identical to those of Formula (I).

The compound according to any of the second aspect of the present invention is a compound of Formula (Ib), or a pharmaceutically acceptable salt, geometric isomer, stereoisomer, solvate, ester or prodrug thereof, 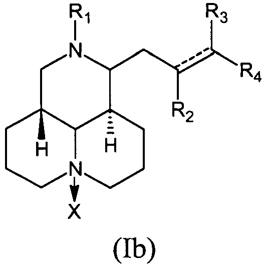 wherein:
X, R₁, R₂, R₃ and R₄ have definitions identical to those of Formula (I).

In the compound according to any of the first aspect of the present invention, X is unsubstituted.

In the compound according to any of the first aspect of the present invention, X is oxygen.

In the compound according to any of the first aspect of the present invention, R₁ is selected from:
(1) C₁₋₆ alkyl-CO-, C₂₋₆ alkenyl-CO-, aryl-C₁₋₆ alkyl-CO-, aryl-C₂₋₆ alkenyl-CO-, aryloxy-C₁₋₆ alkyl-CO-, or aryloxy-C₂₋₆ alkenyl-CO-, wherein the alkyl, alkenyl and aryl are optionally substituted with 1-3 groups independently selected from the group consisting of: hydroxyl, halogen, nitro and C₁₋₆ alkyl,
(2) C₁₋₆ alkyl-SO-, or aryl-SO₂-, wherein the alkyl and aryl is optionally substituted with 1-3 groups independently selected from the group consisting of: hydroxyl, halogen, nitro and C₁₋₆ alkyl,
(3) one or two C₁₋₆alkyl, or one or two C₂₋₆ alkenyl, wherein the alkyl and alkenyl are optionally substituted with 1-3 groups independently selected from the group consisting of: hydroxyl, halogen and C₁₋₆ alkyl,
(4) aryl-C₁₋₆ alkyl-, or heteroaryl-C₁₋₆ alkyl-, wherein the aryl and heteroaryl are optionally substituted with 1-3 groups independently selected from the group consisting of: C₁₋₆ alkyloxy, nitro, halogen, cyano, C₁₋₆ alkyl, C₂₋₆ alkenyl, and wherein the ring of the heteroaryl contains 1 or 2 heteroatoms selected from N, O and S,
(5) aryl, which is optionally substituted with 1-3 groups independently selected from the group consisting of: C₁₋₆ alkyl, nitro, halogen and cyano,
(6) C₁₋₁₂ alkyl, C₅₋₁₂ aryl, C₂₋₁₂ alkenyl, C₂₋₁₂ alkynyl, C₇₋₁₂ alkenylaryl, C₇-C₁₂ alkynylaryl, or C₆-C₁₂alkylaryl, which is optionally substituted with 1-3 groups independently selected from the group consisting of: hydroxyl, C₁₋₆ alkyloxy and halogen.

In the compound according to any of the first aspect of the present invention, R₂ represents 1 or 2 groups selected from the group consisting of: -H, hydroxyl, C₁₋₁₂ alkyloxy, C₁₋₁₂ alkyl optionally substituted with nitro or amino, and C₂₋₁₂ ester group.

In the compound according to any of the first aspect of the present invention, if present, R₃ represents 1 or 2 groups selected from the group consisting of: -H, hydroxyl, C₁₋₁₂ alkyloxy and C₂₋₁₂ ester group.

In the compound according to any of the first aspect of the present invention,if present, R₄ is selected from the group consisting of: -COOH, hydroxyl-C₁₋₆ alkyl-, C₂-C₁₂ ester group, C₂-C₁₂ acylamino, C₂-C₁₂ alcohol group, C₂-C₁₂ ether group, C₂-C₁₂ aldehyde group, C₂-C₁₂ hydrazone group, C₂-C₁₂ oxime group and C₂-C₁₂ oxime ether group;

In the compound according to any of the first aspect of the present invention, --------- represents single bond.

In the compound according to any of the first aspect of the present invention, --------- represents double bond.

In the compound according to any of the first aspect of the present invention:
X is unsubstituted or O;
R₁ is selected from
   (1)C₁₋₄ alkyl-CO-, C₂₋₄ alkenyl-CO-, phenyl-C₁₋₄ alkyl-CO-, phenyl-C₂₋₄ alkenyl-CO-, phenoxy-C₁₋₄ alkyl-CO-, or phenoxy-C₂₋₄ alkenyl-CO-, wherein the alkyl, alkenyl and phenyl are optionally substituted with 1-3 groups independently selected from the group consisting of: hydroxyl, halogen, nitro and C₁₋₄ alkyl,
   (2) C₁₋₄ alkyl-SO₂-, or phenyl-SO₂-, wherein the alkyl and phenyl are optionally substituted with 1-2 groups independently selected from the group consisting of: hydroxyl, halogen, nitro and C₁₋₄alkyl,
   (3) one or two C₁₋₄ alkyl, or one or two C₂₋₄ alkenyl, wherein the alkyl and alkenyl are optionally substituted with 1-2 groups independently selected from the group consisting of: hydroxyl, halogen and C₁₋₄ alkyl,
   (4) phenyl-C₁₋₄ alkyl-, or heteroaryl-C₁₋₆ alkyl-, wherein the phenyl and heteroaryl are optionally substituted with 1-2 groups independently selected from the group consisting of: C₁₋₄ alkyloxy, nitro, halogen, cyano, C₁₋₄ alkyl, C₂₋₄ alkenyl, and wherein the heteroaryl is selected from pyridyl and thiazolyl,
   (5) phenyl, which is optionally substituted with 1-2 groups independently selected from the group consisting of: C₁₋₄ alkyl, nitro, halogen and cyano,
   (6) C₁₋₆ alkyl, C₅₋₈ aryl, C₂₋₆ alkenyl, C₂₋₆ alkynyl, C₂₋₆ alkenylaryl-, C₂-C₆ alkynyl- aryl-, or C₁-C₆ alkyl- aryl-, which is optionally substituted with 1-3 groups selected from the group consisting of: hydroxyl, C₁₋₄ alkyloxy and halogen;
R₂ represents 1 or 2 groups selected from the group consisting of: -H, hydroxyl, C₁₋₆ alkyloxy, C₁₋₆ alkyl optionally substituted with nitro or amino, and C₂₋₆ ester group;
If present, R₃ represents 1 or 2 groups selected from the group consisting of: -H, hydroxyl, C₁₋₆ alkyloxy and C₂₋₆ ester group;
If present, R₄ is selected from the group consisting of: -COOH, -CHO, -CH=NOH, -CH=N-NH₂, hydroxyl-C₁₋₆ alkyl-, C₂-C₁₂ ester group, C₂-C₁₂ acylamino, C₂-C₁₂ ether group and C₂-C₁₂ oxime ether group;
--------- represents single bond or double bond.

In the compound according to any of the first aspect of the present invention:
X is unsubstituted or O;
R₁ is selected from
   (1) C₁₋₄ alkyl-CO-, C₂₋₄ alkenyl-CO-, phenyl-C₁₋₄ alkyl-CO-, phenyl-C₂₋₄ alkenyl-CO-, phenoxy-C₁₋₄ alkyl-CO-, or phenoxy-C₂₋₄ alkenyl-CO-, wherein the alkyl, alkenyl and phenyl are optionally substituted with 1-2 groups independently selected from the group consisting of: hydroxyl, halogen, nitro and C₁₋₄ alkyl,
   (2) C₁₋₄ alkyl-SO₂-, or phenyl-SO₂-, wherein the alkyl and phenyl are optionally substituted with 1-2 groups independently selected from the group consisting of: hydroxyl, halogen, nitro and C₁₋₄ alkyl,
   (3) one or two C₁₋₄ alkyl, or one or two C₂₋₄ alkenyl, wherein the alkyl and alkenyl are optionally substituted with 1-2 groups independently selected from the group consisting of: hydroxyl, halogen and C₁₋₄ alkyl,
   (4) phenyl-C₁₋₄ alkyl-, or heteroaryl-C₁₋₆ alkyl-, wherein the phenyl and heteroaryl are optionally substituted with 1-2 groups independently selected from the group consisting of: C₁₋₄ alkyloxy, nitro, halogen, cyano, C₁₋₄ alkyl, C₂₋₄ alkenyl, and wherein the heteroaryl is selected from pyridyl and thiazolyl,
   (5) phenyl, which is optionally substituted with 1-2 groups independently selected from the group consisting of: C₁₋₄ alkyl, nitro, halogen and cyano;
R₂ represents 1 or 2 groups selected from the group consisting of: -H, hydroxyl, C₁₋₄ alkyloxy, C₁₋₄ alkyl optionally substituted with nitro or amino, and C₂₋₄ ester group;
If present, R₃ represents 1 or 2 groups selected from the group consisting of: -H, hydroxyl, C₁₋₄ alkyloxy and C₂₋₄ ester group;
If present, R₄ is selected from the group consisting of: -COOH, -CHO, -CH=NOH, -CH=N-NH₂, hydroxyl-C₁₋₆ alkyl-, C₂-C₁₂ ester group, C₂-C₁₂ acylamino, C₂-C₁₂ ether group and C₂-C₁₂ oxime ether group;
--------- represents single bond or double bond.

The compound according to any of the first aspect of the present invention is a compound of Formula (Ia-1): 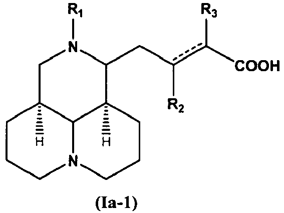 or a pharmaceutically acceptable salt, geometric isomer, stereoisomer, solvate, ester or prodrug thereof, wherein:
R₁ is selected from
   (1) C₁₋₄ alkyl-CO-, C₂₋₄ alkenyl-CO-, phenyl-C₁₋₄ alkyl-CO-, phenyl-C₂₋₄ alkenyl-CO-, phenoxy-C₁₋₄ alkyl-CO-, or phenoxy-C₂₋₄ alkenyl-CO-, wherein the alkyl, alkenyl and phenyl are optionally substituted with 1-2 groups independently selected from the group consisting of: hydroxyl, halogen, nitro and C₁₋₄ alkyl,
   (2) C₁₋₄ alkyl-SO₂-, or phenyl-SO₂-, wherein the alkyl and phenyl are optionally substituted with 1-2 groups independently selected from the group consisting of: hydroxyl, halogen, nitro and C₁₋₄ alkyl,
   (3) one or two C₁₋₄ alkyl, or one or two C₂₋₄ alkenyl, wherein the alkyl and alkenyl are optionally substituted with 1-2 groups independently selected from the group consisting of: hydroxyl, halogen and C₁₋₄ alkyl,
   (4) phenyl-C₁₋₄ alkyl-, or heteroaryl-C₁₋₆ alkyl-, wherein the phenyl and heteroaryl are optionally substituted with 1-2 groups independently selected from the group consisting of: C₁₋₄ alkyloxy, nitro, halogen, cyano, C₁₋₄ alkyl, C₂₋₄ alkenyl, and wherein the heteroaryl is selected from pyridyl and thiazolyl,
   (5) phenyl, which is optionally substituted with 1-2 groups independently selected from the group consisting of: C₁₋₄ alkyl, nitro, halogen and cyano;
R₂ represents 1 or 2 groups selected from the group consisting of: -H, hydroxyl, C₁₋₄ alkyloxy, C₁₋₄ alkyl optionally substituted with nitro or amino, and C₂₋₄ ester group;
R₃ represents 1 or 2 groups selected from the group consisting of: -H, hydroxyl, C₁₋₄ alkyloxy and C₂₋₄ ester group;
R₄ is selected from the group consisting of: -COOH, -CHO, -CH=NOH, -CH=N-NH₂, hydroxyl-C₁₋₆ alkyl-, C₂-C₁₂ ester group, C₂-C₁₂ acylamino, C₂-C₁₂ ether group and C₂-C₁₂ oxime ether group;
--------- represents single bond or double bond.

The compound according to any of the first aspect of the present invention is a compound of Formula (Ia-2): 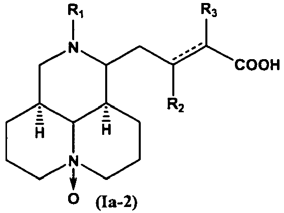 or a pharmaceutically acceptable salt, geometric isomer, stereoisomer, solvate, ester or prodrug thereof, wherein:
R₁ is selected from
   (1) C₁₋₄ alkyl-CO-, C₂₋₄ alkenyl-CO-, phenyl-C₁₋₄ alkyl-CO-, phenyl-C₂₋₄ alkenyl-CO-, phenoxy-C₁₋₄ alkyl-CO-, or phenoxy-C₂₋₄ alkenyl-CO-, wherein the alkyl, alkenyl and phenyl are optionally substituted with 1-2 groups independently selected from the group consisting of: hydroxyl, halogen, nitro and C₁₋₄ alkyl,
   (2) C₁₋₄ alkyl-SO₂-, or phenyl-SO₂-, wherein the alkyl and phenyl are optionally substituted with 1-2 groups independently selected from the group consisting of: hydroxyl, halogen, nitro and C₁₋₄ alkyl,
   (3) one or two C₁₋₄ alkyl, or one or two C₂₋₄ alkenyl, wherein the alkyl and alkenyl are optionally substituted with 1-2 groups independently selected from the group consisting of: hydroxyl, halogen and C₁₋₄ alkyl,
   (4) phenyl-C₁₋₄ alkyl-, or heteroaryl-C₁₋₆ alkyl-, wherein the phenyl and heteroaryl are optionally substituted with 1-2 groups independently selected from the group consisting of: C₁₋₄ alkyloxy, nitro, halogen, cyano, C₁₋₄ alkyl, C₂₋₄ alkenyl, and wherein the heteroaryl is selected from pyridyl and thiazolyl,
   (5) phenyl, which is optionally substituted with 1-2 groups independently selected from the group consisting of: C₁₋₄ alkyl, nitro, halogen and cyano;
R₂ represents 1 or 2 groups selected from the group consisting of: -H, hydroxyl, C₁₋₄ alkyloxy, C₁₋₄ alkyl optionally substituted with nitro or amino, and C₂₋₄ ester group;
R₃ represents 1 or 2 groups selected from the group consisting of: -H, hydroxyl, C₁₋₄ alkyloxy and C₂₋₄ ester group;
R₄ is selected from the group consisting of: -COOH, -CHO, -CH=NOH, -CH=N-NH₂, hydroxyl-C₁₋₆ alkyl-, C₂-C₁₂ ester group, C₂-C₁₂ acylamino, C₂-C₁₂ ether group and C₂-C₁₂ oxime ether group;
--------- represents single bond or double bond.

In the compound according to any of the first aspect of the present invention, R₁ is selected from R1 groups as shown in Tables 1 to 3. In the compound according to any of the first aspect of the present invention, R₂ is selected from R2 groups as shown in Tables 1 to 3,

In the compound according to any of the first aspect of the present invention, R₁ is selected from R1 groups as shown in Tables 1 to 3, or is selected from following groups:
CLCH₂CO-, CH₃CO-, OHCH₂CO-, CH₃CHOHCO-, (*m*-CH₃C₆H₄O)CH₂CO-, C₆H₅CH=CHCO-, CH₃(*m*-CH₃C₆H_{4O})CH₂CO-, C₆H₅CO-, *m*-NO₂C₆H₄CO- 2-CH₃-5-NO₂C₆H₃CO-, *p*-CH₃C₆H₄SO₂-, C₆H₅SO₂-, CH₃SO₂-,
CH₃CH₃⁺-, CH₃CH₂-, CH₃CH₂CH₂-, (CH₂CH₂CH)CH₂-, (CH₂CH₂CH)CH₂-, OHCH₂CH₂-, C₆H₅CH₂-, *p*-CH₃OC₆H₄CH₂-, *p*-CH₃OC₆H₄CH₂-, *p*-NO₂C₆H₄CH₂-, *o*-ClC₆H₄CH₂-, *m*-ClC₆H₄CH₂-, p-ClC₆H₄CH₂-, 3-Cl-4-ClC₆H₃CH₂-, *p*-BrC₆H₄CH₂-, 2-Cl-4-ClC₆H₃CH₂,*p*-FC₆H₄CH₂-, *m*-FC₆H₄CH₂-, *p*-CNC₆H₄CH₂-, *o*-FC₆H₄CH₂-, (*p*-CH₂=CH)C₆H₄CH₂-, *m*-NO₂C₆H₄CH₂-, *o*-CH₃C₆H₄CH₂-, *m*-CH₃C₆H₄CH₂-, *p*-CH₃C₆H₄CH₂-, *m*-CH₃OC₆H₄CH₂-, 2-F-4-BrC₆H₃CH₂-, 2-C₅H₄NCH₂-, 4-C₃H₂SNCH₂-, CH₃CH₂CH₂-, CH₃CH₂CH₂-, CH₃CH₂CH₂CH₂CH₂-, CH₃CH₂CH₂CH₂CH₂-, *p*-NO₂C₆H₅-, 2-CH₃C₆H₅-, 2-CH₃C₆H₅-, 4-BrC₆H₅-, 4-BrC₆H₅-, 3,5₋(CH₃)₂C₆H₅-, *p*-CNC₆H₅-, *p*-CNC₆H₅-.

The compound according to any of the first aspect of the present invention, which is selected from :
N-acetyl matrinic acid;
N-p-tosyl matrinic acid;
N-chloroacetyl matrinic acid;
N-(2-m-methylphenoxy)acetyl matrinic acid;
N-(2-hydroxyl)acetyl matrinic acid;
N-(2-hydroxyl)propionyl matrinic acid;
N-(2-m-methylphenoxy)propionyl matrinic acid;
N-benzoyl matrinic acid;
N-(3-nitrobenzoyl) matrinic acid;
N-(2-methyl-5-nitrobenzoyl) matrinic acid;
N-benzyl matrinic acid;
N-benzyl oxy matrinic acid;
N-benzenesulfonyl matrinic acid;
N-cinnamoyl matrinic acid;
N,N'-dimethyl matrinic acid;
N,N'-dimethyl oxy matrinic acid;
N-ethyl matrinic acid;
N-propyl matrinic acid;
N-cyclopropylmethyl matrinic acid;
N-cyclopropylmethyl oxy matrinic acid;
N-hydroxylethyl matrinic acid;
N-(4-methoxybenzyl) matrinic acid;
N-(4-methoxybenzyl) oxy matrinic acid;
N-(4-nitrobenzyl) matrinic acid;
N-(2-chlorobenzyl) matrinic acid;
N-(3-chlorobenzyl) matrinic acid;
N-(4-chlorobenzyl) matrinic acid;
N-(3,4-dichlorobenzyl) matrinic acid;
N-(4-bromobenzyl) matrinic acid;
N-(2,4-dichlorobenzyl) matrinic acid;
N-(4-fluorobenzyl) matrinic acid;
N-(3-fluorobenzyl) matrinic acid;
N-(4-cyanobenzyl) matrinic acid;
N-(2-fluorobenzyl) matrinic acid;
N-(4-ethenylbenzyl) matrinic acid;
N-(3-nitrobenzyl) matrinic acid;
N-(2-methylbenzyl) matrinic acid;
N-(3-methylbenzyl) matrinic acid;
N-(4-methylbenzyl) matrinic acid;
N-(3-methoxy)benzyl matrinic acid;
N-(2-pyridinylbenzyl) matrinic acid;
N-(3-pyridinylbenzyl) matrinic acid;
N-(4-pyridinylbenzyl) matrinic acid;
N-(4-thiazolylbenzyl) matrinic acid;
N-(1-naphthylbenzyl) matrinic acid;
N-(4-methylbenzoyl) matrinic acid;
N-(4-methoxybenzoyl) matrinic acid;
N-(4-cyanobenzoyl) matrinic acid;
N-(4-fluorobenzoyl) matrinic acid;
N-(4-trifluoromethylbenzoyl) matrinic acid;
N-p-methoxybenzenesulfonyl matrinic acid;
N-(4-trifluoromethoxybenzyl) matrinic acid;
Sophocarpinic acid;
N-benzoyl sophocarpinic acid;
N-benzyl sophocarpinic acid;
N-acetyloxy sophocarpinic acid;
N-propyl sophocarpinic acid;
N-(2-fluoro-4-bromobenzyl) sophocarpinic acid;
N-pivaloyl sophocarpinic acid;
N-pentyl sophocarpinic acid;
N-(4-cyanobenzyl) sophocarpinic acid;
N-(4-nitrobenzyl) sophocarpinic acid;
N-(2-methylbenzyl) sophocarpinic acid;
N-(4-bromobenzyl) sophocarpinic acid;
N-(4-trifluoromethylbenzyl) sophocarpinic acid;
N-(p-trifluoromethylbenzenesulfonyl) sophocarpinic acid;
N-(m-cyanobenzenesulfonyl) sophocarpinic acid;
N-benzyl-13-hydroxyl matrinic acid;
N-4-cyanobenzyl-13-hydroxyl matrinic acid;
N-2-methylbenzyl-13-hydroxyl matrinic acid;
N-4-bromobenzyl-13-hydroxyl matrinic acid;
N-3, 5-dimethylbenzyl-13-hydroxyl matrinic acid;
N-4-trifluoromethylbenzyl-13-hydroxyl matrinic acid;
N-acetyl-(5R)-matrinic acid;
N-tert-butyryl-(5R)-matrinic acid;
N-bromoacetyl-(5R)-matrinic acid;
N-formylethyl ester group-(5R)-matrinic acid;
N-formylmethyl ester group-(5R)-matrinic acid;
N-formylbenzyl ester group-(5R)-matrinic acid;
N-benzenesulfonyl-(5R)-matrinic acid;
N-p-tosyl-(5R)-matrinic acid;
N-benzoyl(5R)-matrinic acid;
N-(3-nitrobenzoyl)-(5R)-matrinic acid;
N-(p-methylbenzoyl)-(5R)-matrinic acid;
N-(p-methoxybenzoyl)-(5R)-matrinic acid;
N-(p-fluorobenzoyl)-(5R)-matrinic acid;
N-(2-methyl-5-nitrobenzoyl) -(5R)-matrinic acid;
N-(3-nitro-4-fluorobenzoyl) -(5R)-matrinic acid;
N-(3-nitro-4-methoxybenzoyl)-(5R)-matrinic acid,
13-hydroxy matrine;
13-methoxy matrine;
13-benzyloxy matrine;
13-ethoxy matrine;
13-benzoyloxy matrine;
13-nitromethyl matrine;
13-methylaminomatrine;
13,14-dihydroxyl matrine;
13,14-dimethoxy matrine;
13,14-dibenzyloxy matrine;
13,14-diacetyloxy matrine;
13,14-di(4-fluoro-3-nitro) benzoyloxy matrine;
14-hydroxyl-13-acetyloxy matrine;
14-hydroxyl-13-chloroacetyloxy matrine;
14-hydroxyl-13-2-chloropropionyloxy matrine;
14-hydroxyl-13-benzoyloxy matrine;
14-hydroxyl-13-(4-fluoro-3-nitro)benzoyloxy matrine;
14-hydroxyl-13-acetyloxy matrine;
14-methoxy sophocarpine;
or a pharmaceutically acceptable salt, geometric isomer, stereoisomer, solvate, ester or prodrug thereof.

The second aspect of the present invention provides a method for preparing the compound of any of the first aspect of the present invention, which comprises the following steps:
(1) in an aqueous solution of alkaline, subjecting a compound of Formula (ia): 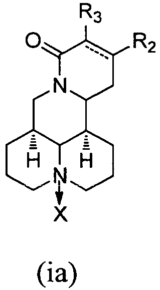 to refluxing with stirring for 2-20 h, then reacting at 10-40°C (for example, at 15-35°C, such as at room temperature) for a time (for example for 5-30 h, such as for 5-15 h), regulating pH value with an acid to 4-7 (for example 5-6), concentrating to dryness, to obtain an acid of Formula (ii): 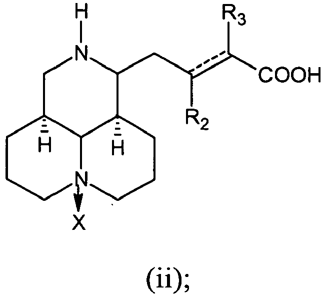
(2) in an organic solvent (e.g., petroleum ether, for example a petroleum ether having a boiling range of 60-90°C), reacting benzophenone hydrazone with yellow mercury oxide at 10-40°C(for example at 15-35°C, such as at room temperature) for a time (for example for 2-20 h, such as for 5-15 h), to obtain a solution of diphenyldiazomethane;
(3) mixing a solution of the acid of Formula (ii) in a solvent (for example alcohol, such as methanol) with the solution obtained in step (2), reacting the mixture solution at 10-40°C(for example at 15-35°C, such as at room temperature), to obtain an ester of Formula (iii): 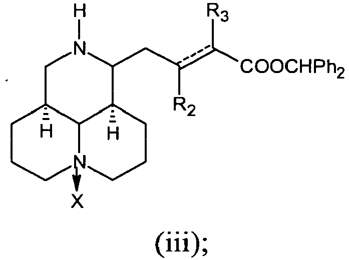
(4) in the presence of an alkaline (for example, alkali metal hydroxide or alkali metal carbonate, such as potassium hydroxide, potassium carbonate), reacting the ester of Formula (iii) with a compound represented by Formula R₁-Y at 10-40°C(for example at 15-35°C, such as at room temperature) for a time (for example for 1-50 h, such as for 2-30 h), to obtain a compound of Formula (iv): 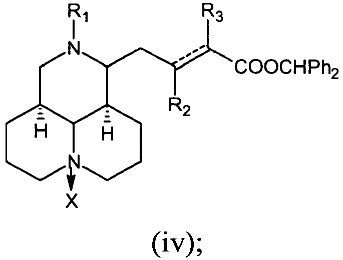
(5) in the presence of metacresol, reacting the compound of Formula (iv) at 70-90°C(for example, about 80°C) for a time (for example, for 2-20 h, such as for 5-15 h), to obtain a compound of Formula (Ia) 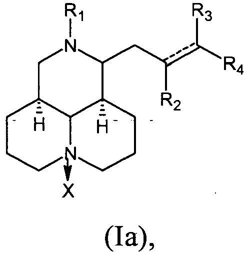 wherein Y represents halogen(for example, fluorine, chlorine, bromine or iodine), R₁, R₂, R₃, R₄, X and bond --------- have the same meanings as mentioned in any of the first aspect of the present invention.

The aforementioned is to prepare a compound of Formula (Ia) from Formula (ia) as starting material. Similarly, in the context of the present invention, a compound of Formula (ib) can be used as starting material to prepare Formula (Ib)

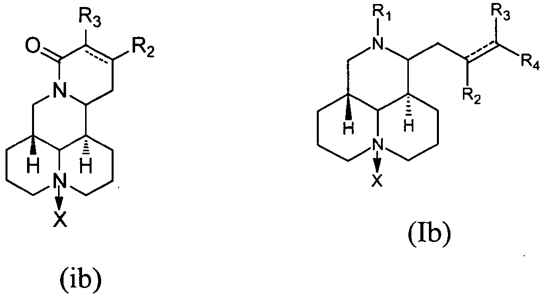

The third aspect of the present invention provides a pharmaceutical composition, comprising a therapeutically and/or prophylactically effective amount of the compound or a pharmaceutically acceptable salt, geometric isomer, stereoisomer, solvate, ester or prodrug thereof according to any of the first aspect of the present invention, and optionally one or more pharmaceutically acceptable carriers or excipients.

The fourth aspect of the present invention provides a use of the compound or a pharmaceutically acceptable salt, geometric isomer, stereoisomer, solvate, ester or prodrug thereof according to any of the first aspect of the present invention or the pharmaceutical composition according to any of the third aspect of the present invention in manufacture of a medicament for treatment and/or prophylaxis of a disease or disorder associated with viral infection. According to any of the fourth aspect of the present invention, the disease or disorder associated with viral infection is selected from inflammatory liver diseases (for example, hepatitis B, hepatitis C, hepatitis A) and AIDS.

The fifth aspect of the present invention provides a use of the compound or a pharmaceutically acceptable salt, geometric isomer, stereoisomer, solvate, ester or prodrug thereof according to any of the first aspect of the present invention or the pharmaceutical composition according to any of the third aspect of the present invention as a medicament for combating a disease or disorder associated with viral infection. According to the use of any of the fifth aspect of the present invention, the disease or disorder associated with viral infection is selected from inflammatory liver diseases (for example, hepatitis B, hepatitis C, hepatitis A) and AIDS.

The sixth aspect of the present invention provides a method for treatment and/or prophylaxis of a disease or disorder associated with viral infection in a subject in need, the method comprising administering the subject in need an therapeutically and/or prophylactically effective amount of the compound or a pharmaceutically acceptable salt, geometric isomer, stereoisomer, solvate, ester or prodrug thereof according to any of the first aspect of the present invention or the pharmaceutical composition according to any of the third aspect of the present invention. According to the method of any of the sixth aspect of the present invention, the disease or disorder associated with viral infection is selected from inflammatory liver diseases (for example, hepatitis B, hepatitis C, hepatitis A) and AIDS.

The seventh aspect of the present invention provides the compound or a pharmaceutically acceptable salt, geometric isomer, stereoisomer, solvate, ester or prodrug thereof according to any of the first aspect of the present invention for treatment and/or prophylaxis of a disease or disorder associated with viral infection. According to the compound of the seventh aspect of the present invention, the disease or disorder associated with viral infection is selected from inflammatory liver diseases (for example, hepatitis B, hepatitis C, hepatitis A) and AIDS.

The features of any one aspect or any one of any one aspect of the present invention are also applicable in any one of other aspects or any of the other aspect. In the present invention, for example, when "any of the first aspect of the present invention" is mentioned, the "any of" refers to any one of sub-aspects of the first aspect of the present invention, and similar phrases for other aspects have similar meanings.

### Detailed Description of the Invention:

The aspects and features of the present invention are further described as follows.

As for all documents as referred in the present invention, their whole contents are incorporated into the text by reference, and when the meanings in these documents are not consistent with the present invention, the expressions of the present invention are used. In addition, the terms and phrases used in the present invention have common meanings known by those skilled in the art. Nevertheless, the present invention still provides illustrations and explanations for these terms and phrases in details as much as possible, and when the meanings of the mentioned terms and phrases are not consistent with their meanings well known in the art, the meanings presented in the present invention are used.

In the structure of the compound of Formula I of the present invention, when "N-substituted" is mentioned, it represents that in the following structure formula or similar structure thereof, the N atom designated with * is substituted:

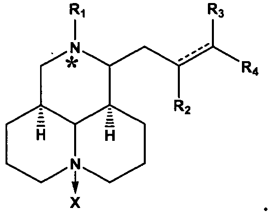

In addition, when "N-oxidized matrinic acid" is mentioned, it represents that in the following structure or similar structure thereof: 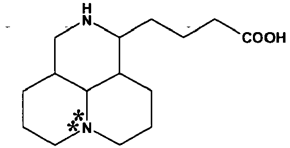 the N atom designated with ** is in oxidization state to be:

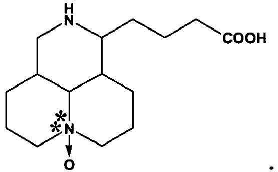

Other compounds as similarly mentioned, such as "N-oxidized kurarinol" have similar meanings. When "matrine" is mentioned in the text, it represents that in the following structure of similar structure thereof 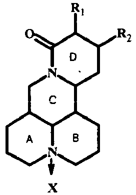 R₁ and R₂ are H, and X is not substituted.

In the text, when the term "pharmaceutically acceptable" is mentioned for example in "pharmaceutically acceptable salt", it represents that the salt is not only physiologically acceptable in a subject, but also a pharmaceutically employable synthetic substance, for example, a salt as intermediate formed in chiral resolution cannot be directly administered to a subject, but this salt can be used to obtain the final product of the present invention.

In the text, the terms "geometric isomer" and "stereoisomer" separately refer to a cis-trans-isomer originated from a double bond, and a stereoisomer originated from an asymmetric carbon atom.

In the text, the term "alkyl" comprises straight and branched saturated hydrocarbonyls with a designated number of carbon atoms. In the text,the term "C₁₋₆ alkyl" refers to an alkyl having a designated number of carbon atoms, which is a straight or branched alkyl, and can comprise its subgroups, such as C₁₋₄ alkyl, C₁₋₃ alkyl, C₁₋₂ alkyl, C₂₋₆ alkyl, C₂₋₄ alkyl, for example, methyl, ethyl, n-propyl, iso-propyl, n-butyl, iso-butyl, tert-butyl, pentyl, hexyl etc.

In the text, the term "C₂₋₆ alkenyl" is an alkenyl having a designated number of carbon atoms, which is a straight or branched alkenyl, and can comprise its subgroups, for example, C₂₋₄ alkenyl; C₂₋₃ alkenyl, C₃₋₆ alkenyl, C₃₋₄ alkenyl, such as ethenyl, propenyl, allyl, butenyl, hexenyl etc.

In the text,the term "C₂₋₁₂ alkynyl" refersto an alkynyl having a designated number of carbon atoms, which is a straight or branched alkynyl, and can comprise its subgroups, for example C₂₋₄ alkynyl, C₂₋₃ alkynyl, C₂₋₆ alkynyl, C₃₋₈ alkynyl etc, such as ethynyl, propynyl, propargyl, butynyl, hexynyl etc.

In the text,the term "C₁₋₆ alkyl-CO-"refers to alkylacyl, which links to other moiety of compound via the carbon atom of acyl, in which the term "alkyl" has the same definition as aforementioned. Other groups, such as "C₂₋₆ alkenyl-CO-", "aryl-C₁₋₆ alkyl-CO-", have similar meanings.

In the text, the term "aryl" is a monocyclic or bicyclic aromatic group in a single definition or a combined definition , (for example, phenyl or naphthyl). The examples thereof include but are not limited to phenyl, naphthyl, anthryl etc. In an embodiment, the aryl is phenyl. Similarly, the term "aryloxy-" is an aryl, which links to other moiety of compound via oxygen.

In the text, the term "C₁₋₆ alkyl-SO₂-" refers to a C₁₋₆ alkyl, which links to other moiety of compound via sulfonyl (-SO₂-).

In the text,the term "optionally substituted with 1-3 groups selected from" comprises any meanings as covered thereby, for example, phrases "optionally substituted with 1-2 groups independently selected from" and "optionally substituted with 2-3 groups independently selected from".

Linkage bond "---------" represents a single bond or double bond, for example, R₂ and R₃ separately refer to one group (for example, R₂ and R₃ separately represent a hydrogen), and this linkage bond represents a double bond; when R₂ and R₃ separately refer to two groups (for example, R₂ and R₃ represent two hydrogen atoms), and this linkage bond represents single bond. In addition, the linkage bond "---------" can be used to determine the number of substituents represented by R₂ and R₃, for example, when the linkage bond "---------" is a single bond, R₂ and R₃ separately represent two substituents (for example, separately represent two hydrogen atoms), when the linkage bond "---------" is a double bond, R₂ and R₃ separately represent one substituent (for example, separately represent one hydrogen atom).

In the text, as the group of R₁, "CH₃CH₃⁺-" represents two methyl groups which link to the ring nitrogen atom of Formula I at the same time.

In the text,the phrase "X is unsubstituted or O" separately represents the sign X in the following two structure formulas:

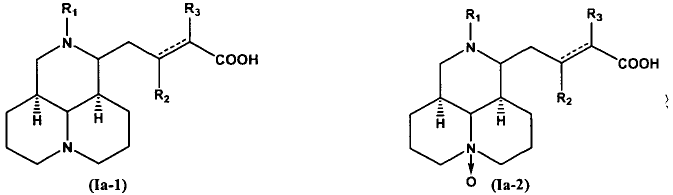

Wherein Formula (Ia-1) is a matrinic acid derivative, Formula (Ia-2) is an oxy matrinic acid derivative (i.e., X is oxygen).

In the text, especially when the compounds as prepared in the examples are named (without other specific designation), "matrinic acid" and "(5R)-matrinic acid" separately refer to a compound of formula 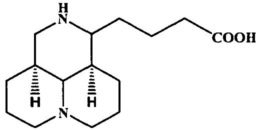 and a compound of formula 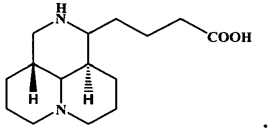 As for the N-substituted compounds of the present invention, for example, "N-acetyl matrinic acid" and "N-acetyl-(5R)-matrinic acid", they separately refer to a compound of formula 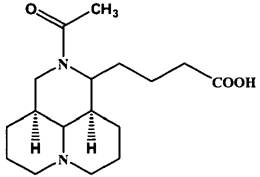 and a compound of formula 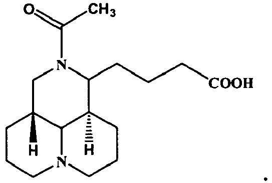 When other situations exist in names, they have similar meanings.

In the text, the term "halogen", "halogen atom", "halogenated" represent fluorine, chlorine, bromine or iodine, especially represent fluorine, chlorine or bromine.

In the text, the term "effective amount" refers to a dose that can fulfill treatment and/or prophylaxis of the disease or disorder of the present invention in a subject.

In the text, the term "pharmaceutical composition" refers to a "composition", which can fulfill treatment and/or prophylaxis of the disease or disorder of the present invention in a subject, especially a mammal.

In the text, the term "subject" can refer to a patient or an animal, especially human, dog, monkey, bovine, equine etc which is administered with the compound of Formula I of the present invention or its pharmaceutical composition to treat and/or prevent the disease or disorder of the present invention.

In the text, if not specifically stated, "%" refers to a weight/weight percentage, especially in a situation of describing solid substance. Of course, when a liquid substance is described, the "%" can refer to weight/volume percentage (for a situation in which a solid is dissolved in a liquid), or a volume/volume percentage (for a situation in which a liquid is dissolved in a liquid).

In an embodiment, the compound of Formula (I) as provided by the present invention is as follows: 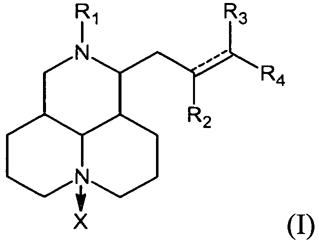
X is O or unsubstituted;
R₁ independently is -H, C₁-C₁₂ alkyl, C₅-C₁₂ aryl, C₂-C₁₂ alkenyl, C₂-C₁₂ alkynyl, C₇-C₁₂ alkenylaryl, C₇-C₁₂ alkynylaryl or C₆-C₁₂alkylaryl that is not substituted or substituted with hydroxyl, alkyloxy and halogen etc;
R₂ independently is -H, hydroxyl, C₁-C₁₂ alkyloxy, C₁-C₁₂ alkyl substituted with nitro or amino, and C₂-C₁₂ ester group;
R₃ independently is -H, hydroxyl, C₁-C₁₂ alkyloxy and C₂-C₁₂ ester group;
R₄is carboxylic acid, C₂-C₁₂ ester group, alcohol, ether, aldehyde;

According to the present invention, the preferred compounds of the present invention include but are not limited to: N-benzyl matrinic acid (DM-108), N-benzyl oxymatrinic acid (DM-1081), N-(4-methoxybenzyl) matrinic acid (DM-122), N-(4-methoxybenzyl) oxymatrinic acid (DM-1221), N-benzyl sophocarpinic acid(SC-17).

In an embodiment of the compound of Formula (I) of the present invention, when there is a double bond at side chain α, β-positions, the compound can have cis-, trans-geometric isomers. The compounds of the present invention comprise these cis- and trans-geometric isomers.

In an embodiment, the present invention refers to a method for preparing the N-substituted matrinic acid derivative of Formula (I) or a pharmaceutically acceptable salt, geometric isomer or stereoisomer thereof, which comprises the following steps:
(1), to an aqueous solution of potassium hydroxide is added matrine, heated and refluxed (5-15 h, for example about 9 h), then reacted at room temperature overnight, then used 3N hydrochloric acid to regulate pH5-6, concentrated in reduced pressure to dryness, to obtain matrinic acid;
(2), to petroleum ether with a boiling range of 60°C-90°C is added a mixture of benzophenone hydrazone and yellow mercury oxide, reacted with stirring at room temperature (3-10 h, for example about 6 h), to obtain dark violet petroleum ether solution of diphenyldiazomethane;
(3), to a methanol solution of matrinic acid was added the solution of step (2) so that the resultant mixture solution reacts at room temperature until violet disappears, filtered, concentrated to dry, soaked the residue in petroleum ether, filtered to obtain diphenylmethyl ester of matrinic acid ;
(4), diphenylmethyl ester of matrinic acid is dissolved in dichloromethane, added with anhydrous potassium carbonate, added with a solution of 4-methoxybenzyl chloride or acetyl chloride in dichloromethane under ice-water bath, reacted at room temperature until the completion of reaction, filtered, dried the filtrate by evaporation, the resultant oily substance was dissolved in meta-cresol, heated and reacted at 110°C for 5h-15h, to obtain the final product.

The compound of the present invention can be synthesized via the following exemplary reaction scheme:

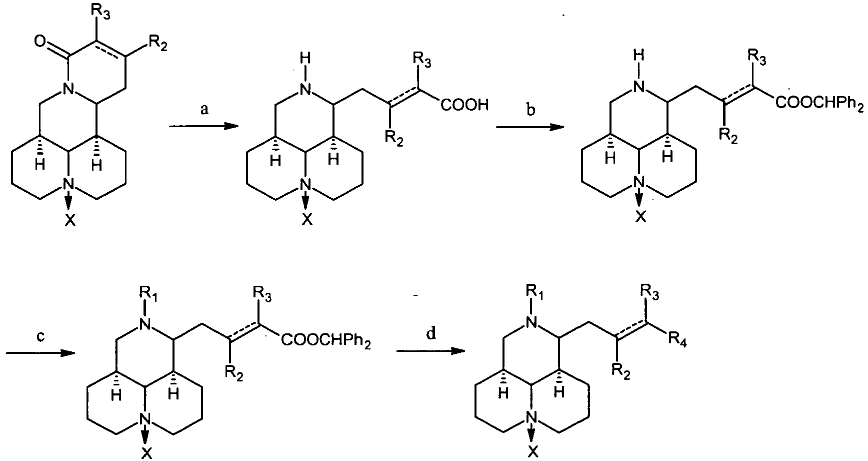

In the reaction scheme, the reaction conditions of the steps can be any one of items from a to e or a combination thereof:
a: KOH/H₂O, refluxed, 8h;
b: diphenyldiazomethane, room temperature, 12h;
c: acyl halide, sulfonyl chloride or halogenated hydrocarbon, K₂CO₃ or KOH, room temperature, 3-24h;
d: meta-cresol, 70-90°C, 8h;
e: in order to obtain the compound of Formula (I) of the present invention in which R₄ is -CH₂OH alcohol, the carboxylic acid obtained in step a can be reduced to form an alcohol, the reagents used in the reaction are for example but not limited to: LiAlH₄ or Pd/C.

In an embodiment, the present invention relates to a use of N-substituted matrinic acid derivative of Formula (I), a pharmaceutically acceptable salt, geometric isomer or stereoisomer thereof in manufacture of a medicament for prophylaxis and/or treatment of a disease or disorder associated with viral infection(for example, hepatitis, such as hepatitis B and/or hepatitis C).

In an embodiment, the present invention relates to a method for prophylaxis and/or treatment of a disease or disorder associated with viral infection (for example, hepatitis, such as hepatitis B and/orhepatitis C), in which a prophylactically and/or therapeutically effective amount of N-substituted matrinic acid derivative of Formula (I), a pharmaceutically acceptable salt, geometric isomer or stereoisomer thereof is administered to a patient in need of the prophylaxis and/or treatment.

Some compounds of the present invention can be presented in form of different isomers (for example, enantiomer and diastereoisomer).In the present invention, all of pure forms and mixture forms of these isomers, including racemic mixture are considered. Enol forms are also included therein.

The compound of the present invention can be in form of non-solvate or solvate, including hydrate, such as semi-hydrate. In general, as for the objective of the present invention, a solvate formed with a pharmaceutically acceptable solvent such as water and ethanol is equivalent to non-solvate thereof.

Some of the compounds of the present invention can also form pharmaceutically acceptable salts, such as acid addition salts. For example, nitrogen atom can form a salt with acid. Examples of suitable acids for salification are hydrochloric acid, sulfuric acid, phosphoric acid, acetic acid, citric acid, oxalic acid, malonic acid, salicylic acid, malic acid, fumaric acid, succinic acid, ascorbic acid, malic acid, methylsulfonic acid and other mineral acids and carboxylic acids well known by those skilled in the art. These salts can be prepared by conventional methods by contacting a free base with a sufficient amount of the desired acid to generate salt. When the resultant salt is treated with a suitable diluted aqueous solution of alkali, such as diluted aqueous solutions of potassium hydroxide, potassium carbonate, aqueous ammonia and sodium hydrogen carbonate, the free base can be regenerated. Various free bases may be slightly different from their salts in some physical properties (such as dissolubility in a polar solvent), but their acidic salts and free bases are equivalent between each other for the objective of the present invention. (see: for example, S. M. Berge, et al., "Pharmaceutical Salts,"J. Pharm. Sci., 66: 1-19 (1977), which is incorporated into the text by reference.

The compound of the present invention can be used in form of a pharmaceutically acceptable salt derived from an inorganic acid or organic acid. The term "pharmaceutically acceptable salt" refers to a salt suitable for contacting with tissues of human and lower animals without excessive toxicity, stimulation, allergic reaction and having a rational effect/risk ratio in a reliable medical judgment range. The pharmaceutically acceptable salts are well known in the art. For example, S. M. Berge et al described pharmaceutically acceptable salts in details in J. Pharmaceutical Sciences 1977, 66: 1 *(see below).* The salts can be prepared in site during final separation and purification procedures or prepared solely by reacting the free base functionality with a suitable organic acid. The representative acid addition salts include but are not limited to acetates, adipates, alginates, citrates, aspartates, benzoates, benzenesulfonates, bisulfates, butyrates, camphorates, camphorsulfonates, digluconates, glycerophosphates, hemisulphates, enanthates, caproates, fumarates, hydrochlorates, hydrobromates, hydriodates, 2-hydroxylesylate (isothiosulfate), lactates, maleates, mesylates, nicotinates, 2-napsylates, oxalates, palmitates, pectates, persulfates, 3-phenylpropionates, picrates, pivalates, propionates, succinates, tartrates, thiocyanates, phosphates, glutamates, bicarbonates, tosilates, and undecanoic acid slats. Similarly, alkaline nitrogen-containing group can be quatemized with the following substances: lower alkyl halides, such as chlorides bromides and iodides of methyl, ethyl, propyl and butyl; sulfuric acid dialkyl ester, such as sulfuric acid dimethyl, diethyl, dibutyl and dipentyl esters; long chain halides such as chlorides, bromides and iodides of decyl, dodecyl, tetradecyl, octadecyl; arylalkyl halides such as benzyl bromide and phenylethyl bromide and so on. Hence, a product that can be dissolved or dispersed in water or oil can be obtained. The acids for forming pharmaceutically acceptable acid addition salts include inorganic acids such as hydrochloric acid, hydrobromic acid, sulfuric acid and phosphoric acid, and organic acids such as oxalic acid, malic acid, succinic acid and citric acid.

Alkaline addition salts can be prepared in site during the final separation and purification of the compound of the present invention by reacting the carboxylic acid containing moiety of the present invention with a suitable alkali, and the alkali for example can be pharmaceutically acceptable hydroxides of metal cationic ions, carbonates and bicarbonates, or ammonia or organic primary amines, secondary amines or tertiary amines.

The pharmaceutically acceptable salts include but are not limited to salts based on alkali metals or alkaline earth metals such as lithium, sodium, potassium, calcium and aluminum etc., and nontoxic quaternary ammonium and amine cationic ions, including ammonium, tetramethylammonium, tetraethylammonium, methylammonium, dimethylammonium, trimethylammonium, triethylammonium, diethylammonium and ethylammonium etc.. Other representative organic amines for forming alkaline addition salts include ethylenediamine, ethanolamine, diethanolamine, piperidine, piperazine etc..

The actual dose levels of various active components in the pharmaceutical composition of the present invention can be modified so that the resultant amounts of active compounds can be effectively adapted to a specific patient, composition and administration manner to achieve the desired therapeutical reactions. The dose levels can be designated according to activity of specific compound, administration route, severity of disease and conditions and medical history of a patient to be treated. However, the practice in the art is that the dose of compound gradually increases from a level lower than that for achieving the desired therapeutical effects to a dose capable of achieving the desired therapeutical effects.

When adopted to the aforementioned or other treatment, a compound of the present invention in a therapeutically effective amount can be used in form of pure compound, or in form of pharmaceutically acceptable salt, ester or prodrugthere(if they exist). Alternatively, the compound can be administered via a pharmaceutical composition comprising the compound and one or more pharmaceutically acceptable excipients. The term "effective amount" or "therapeutically effective amount" of the compound of the present invention means that the compound is in an amount sufficient to achieve therapeutically reasonable ratio of effect/risk for any medical treatment. It should be understood that the total amount per day of the compound or composition of the present invention must be determined by a physician within the range of reliable medical judgement. As for any specific patients, the specific therapeutically amount must be determined based on various factors, including the diseases to be treated and severity thereof, the activity of the used specific compound, the used specific composition, the age, body weight, general health status, gender and diet of patient, the administration time and route and excretory rate of the used specific compound, the drug(s) administered in combination or simultaneously with the specific compound, and similar factors well known in the art of medicine. For example, it is a common method in the art to increase gradually the dose of compound from a level lower than that for achieving desired therapeutical effects to a level enough to achieve the desired effects.

The total daily dose of the compound of the present invention administered to human or mammal can be in range of about 0.0001 to about 1000 mg/kg/day (for example, about 0.001 to about 100 mg/kg/day, about 0.001 to about 10 mg/kg/day, about 0.01 to about 10 mg/kg/day, or about 0.1 to about 10 mg/kg/day). As for oral administration, more preferred dose can be in range of about 0.001 to about 50 mg/kg/day(for example, about 0.001 to about 40 mg/kg/day, about 0.001 to about 30 mg/kg/day, about 0.01 to about 20 mg/kg/day, or about 0.1 to about 10 mg/kg/day); as for injection administration, the dose can be determined by referring to the above oral dose, if necessary, can be appropriately adjusted. If necessary, an effective daily dose can be divided into several multiple doses to meet the requirements of administration; hence, a single dose composition can contain this amount of divided doses thereof to consist of daily dose.

The present invention also provides a pharmaceutical composition of the compound of the present invention formulated with one or more nontoxic pharmaceutically acceptable carriers.The pharmaceutical composition can be specifically formulated to form a solid or liquid form for oral administration, parenteral injection or rectal administration.

The pharmaceutical composition of the present invention can be administered orally, rectally, parenterally, endoluminally, endovaginally, intraperitoneally, topically (such as via powder, ointment or drops), buccally to a human or other mammal, or administrated as oral spray or nasal spray. The term "parenteral" in the context refers to administration manners including intravenous, intramusculary, intraperitoneal, intrathoracic, subcutaneous and intraarticular injection and transfusion.

The composition suitable for parenteral injection can comprise physiologically acceptable sterile aqueous or nonaqueous solution, dispersion dosage form, suspension, or emulsion, as well as sterile dispersion for reforming a sterile injectable solution or dispersion. The examples of suitable aqueous or nonaqueous carriers, diluents, solvents or media include water, ethanol, polyols (propylene glycol, polyethylene glycol, glycerol, etc.), vegetable oil (such olive oil), injectable organic esters such as ethyl oleate and suitable mixtures thereof.

These compositions can further comprise excipients, such as preservative, wetting agent, emulsifying agent and dispersant. The use of various antibacterial agents and antifungal agents, such as nipagins, nautisan, phenol, sorbic acid, etc. can ensure effects of combating microorganisms. It is also desired to comprise isotonizing agents such as sugars, sodium chloride, etc. The use of substances for absorption delay, such as aluminum monostearate and gelatin, can achieve the prolonged absorption of injectable dosage form.

The injectable preparation can be sterilized by filtration using a bacterial filter or by incorporating a sterilizing agent in form of sterile solid composition, and the solid composition can be dissolved or dispersed in sterile water or other sterile injectable media before clinical application.

The solid dosage forms for oral administration comprise capsules, tablets, pills, powders and granules. In such solid dosage forms, the active compound can be mixed with at least one inert pharmaceutically acceptable excipient or carrier such as sodium citrate or dicalcium phosphate and/or the following substances: a) filler or bulking agent, such as starch, lactose, sucrose, glucose, mannitol and silicic acid; b) binding agent, such as carboxymethyl cellulose, alginate, gelatin, polyvinyl pyrrolidone, sucrose, and arabic gum; c) humectant, such as glycerol; d) disintegrating agent, such as agar, calcium carbonate, potato or cassava starch, alginic acid, some silicates and sodium carbonate; e) solution blocking agent, such as paraffm wax; f) absorption accelerator, such as quaternary ammonium compounds; g) wetting agent, such as cetanol and glycerol monostearate; h) adsorbent, such as kaolin and bentonite; and i) lubricant, such as talc powder, calcium stearate, magnesium stearate, solid polyethylene glycol, sodium dodecylsulfate and their mixtures. In the cases of capsules, tablets and pills, these dosage forms may also comprise a buffering agent.

The liquid dosage form for oral administration comprises pharmaceutically acceptable emulsion, solution, suspension, syrup and elixir. Besides the active compound, the liquid dosage form may further comprise an inert diluent commonly used in the art, such as water or other solvent, solubilizer and emulsifying agent, such as ethanol, isopropanol, ethyl carbonate, ethyl acetate, benzyl alcohol, benzyl benzoate, propylene glycol, butane-1,3-diol, dimethyl formamide, oils (such as cottonseed oil, peanut oil, corn oil, embryo oil, olive oil, castor oil, and sesame oil), glycerol, tetrahydrofurfuryl alcohol, fatty acid esters of polyethylene glycol and sorbitan, and their mixtures.

The term "pharmaceutically acceptable prodrug" used in the text refers to a prodrug of the compound of the present invention, which is suitable for contacting with tissues of human and lower animals without excessive toxicity, stimulation, allergic reaction, has a rational effect/risk ratio and is effective for desired use, in a reliable medical judgment range, and in possible situations, it further represents an amphoteric ion form of the compound of the present invention. The prodrug of the present invention can be hydrolyzed in blood to convert quickly into the mother compound of the above formula in vivo. Sufficient discussions are provided by T. Higuchi and V. Stella, Pro-drugs as Novel Delivery Systems,V. 14 of the A.C.S. Symposium Series and Edward B. Roche, ed., Bioreversible Carriers in Drug Design, American Pharmaceutical Association and Pergamon Press (1987), which are incorporated herein by reference.

As for the natural deficiencies and drawbacks of "pathogen-targeting traditional antiviral drugs" such as resistance mutation and toxic and side effects, the inventors firstly propose a new theory and strategy of "cellular components-targeting antiviral studying", that is, the compounds targeting proteins that are not essential for cells but aid virus replication in cells can significantly inhibit virus replication and then exert antiviral effects; at the same time, the growth and activity of the host cells per se are not influenced; the novel antiviral drugs aiming at these targets are different from traditional antiviral drugs in: chemotherapy stress does not act on viral proteins and thus can hardly induce viral mutation against drug resistance, so that the drugs developed thereby have broad antiviral activity and good anti-resistant ability. The new theory and new strategy have been confirmed in experiments and clinical practices of using kurorinones against hepatitis B, and are effective routes for solving the problem of drug resistance of viruses.

### Brief Description of Drawings

Fig.1 shows HBVDNA content in liver of duckling upon action of DM122. In the figure, abscissa represents HBVDNA in liver, ordinate represents inhibition rate, the rod of right side 37.5mg/kg group represents inhibition rate of -5.14%.
Fig.2A shows HBVDNA content in serum of duckling upon action of DM122. In the figure, abscissa represents HBVDNA in serum, ordinate represents inhibition rate.
Fig.2B shows anti-HBV activity of Compound DM-122 in serum of duck infected with hepatitis B virus in vivo.
Fig.3 shows low toxicity of DM122 in Huh7.5 cells. In the figure, ordinate represents (standardized) survival cells.
Fig.4 shows dose-dependent inhibition effects of DM122 on intracellular Hsc70 mRNA in Huh7.5 cultured cells. In the figure, ordinate represents multiple times over the control.
Fig. 5 shows dose-dependent inhibition effects of DM122 on intracellular Hsc70 in Huh7.5 cultured cells.
Fig.6 shows inhibition effects of DM122 on intracellular virus in Huh7.5 cultured cell infected with HCV.
Fig.7A shows inhibition effects of DM122 on intracellular HCV Core and Hsc70 in Huh7.5 cultured cell infected with HCV.
Fig.7B shows changes caused by other compounds on levels of HCV core proteins and Hsc70 proteins in Huh7.5 cells.
Fig.7C shows changes caused by Compound 6b on HCV RNA in Huh7.5 cells with acute infection of HCV (cell control, virus control; INF-α: α-interferon).
Fig.8 shows effects of DM122 in reduction of Hsc70 package in virus particles in Huh7.5 cultured cell supernatant.
Fig.9 shows that DM122 does not influence bodyweight of Kunming mice on the 7^{th} day after intraperitoneal injection once. In the figure, ordinate represents bodyweight of mice, abscissa represents the number of days after ip administration of IMB-DM122 (i.e., Compound DM122).
Fig.10A shows that DM122 does not influence functions of liver and kidney of Kunming mice on the 7^{th} day after intraperitoneal injection once. In the figure, ordinate represents concentration in serum of mice.
Fig.10B shows histological examination of DM-122.In the figure, the column at left side shows tissue slices of blank control, and the column at right side shows tissue slices of liver, kidney and spleen of the 1000mg/kg group, which indicate DM-122 has good safety.
Fig.11 A shows effects of oral administration of 6b on bodyweight of mice.
Fig.11B shows effects of oral administration of 6b on functions of liver and kidney of mice. After administration of 6b, the index levels of blood are: BUN: blood urea nitrogen (mM); CRE: creatine (µM); GOP: glutamic oxalacetic transaminase (U/L); GPT: glutamate pyruvate transaminase (U/L).
Fig. 12 shows inhibition effects of Compound 6b on HBV with resistance on lamivudine.

### Embodiments of Invention

The present invention is further illustrated with the following examples, but the scope of the present invention is not limited to the following examples. Those skilled in the art would understand that the present invention can be changed and modified in various ways without departing from the spirit and scope. The present invention describes in general and/or in details the materials and experimental methods used in experiments. Although many materials and operation methods used for fulfilling the objective of the present invention are well known in the art, they are still described in the present invention in details as much as possible.

As for all of the following examples, standard operations and purification methods known in the art can be used. Unless other stated, all temperatures are represented with °C (Celsius degree). The structures of the compounds are determined by nuclear magnetic resonance(NMR) or mass spectrum (MS). m.p. is melting point expressed in °C, and temperature is not calibrated. In the following examples, matrine and oxymatrine are commercially available.

### Example 1: Synthesis of matrinic acid (DM-100):

4.96g(0.02 mol) of matrine was provided, added to an aqueous solution of 6.72g (0.12 mol, 6 eq.) of potassium hydroxide (KOH) in 100 ml water, heated and refluxed for 9h, then reacted at room temperature overnight. The reaction solution was cooled with ice-water bath, adjusted with 3N hydrochloric acid to pH5-6, concentrated in reduced pressure to dryness. The obtained solid was dissolved sufficiently in methanol, filtered, the filter cake was washed with methanol, the filtrates were combined and evaporated to obtain a crude product of matrinic acid (DM-100), this light yellow solid was recrystallized with ethanol/acetone to obtain a pure product of DM-100, total 5.23g (yield: 98.3%), melting point: 185.7-187.7°C.
MS-ESI (M/Z): 267.5 [M+H]⁺
¹H-NMR (CD₃OD, δ ppm): 3.474-3.534 (1H, m), 3.379-3.443 (1H, t, *J*=25.6), 2.905-2.946 (1H, q, *J*=16.4), 2.764-2.829 (2H, t, *J*=26), 2.243-2.314 (1H, m), 2.097-2.202 (2H, m), 1.892-2.039 (4H, m), 1.760-1.846 (1H, m), 1.398-1.706 (11H, m)
IR (KBr, cm⁻¹): 1645 (-CO, ν).

### Example 2: Synthesis of N-oxidized matrinic acid (DM-1001):

5.28g(0.02 mol) of oxymatrine was provided, added to an aqueous solution of 6.72g (0.12 mol, 6 eq.) of potassium hydroxide (KOH) in 100 ml water, heated and refluxed for 9h, then reacted at room temperature overnight. The reaction solution was cooled with ice-water bath, adjusted with 3N hydrochloric acid to PH5-6, concentrated in reduced pressure to dryness.The obtained solid was dissolved sufficiently in methanol, filtered, the filter cake was washed with methanol, the filtrates were combined and evaporated to obtain a crude product of N-oxidized matrinic acid (DM-1001), this light yellow solid was recrystallized with ethanol/acetone to obtain a pure product of DM-1001, 5.1g(yield: 90.4%), melting point: 184.6-186.9°C. MS-ESI (M/Z): 283.3 [M+H]⁺
¹H-NMR (CD₃OD, δ ppm): 4.513-4.571 (1H, m), 4.272-4.333 (1H, t, *J*=24.4), 3.363-3.383 (1H, t, *J*=8), 3.289-3.302 (1H, m), 3.006-3.033 (2H, d, *J*=10.8), 2.919-2.962 (1H, q, *J*=17.2), 2.352-2.526 (2H, m), 2.097-2.256 (4H, m), 1.962-1.998 (1H, d, *J*=14.4), 1.772-1.875 (3H, m), 1.430-1.727 (7H, m).
IR (KBr, cm⁻¹): 3529 (CO-OH, v), 3382, 3359 (N-H, v), 1707 (-CO, v).

### Example 3: Synthesis of kurarinol (DM-200):

0.95g(0.025 mol) of lithium aluminum tetrahydride was suspended in 40 ml of tetrahydrofuran, kept at 50°C. 1.33g(0.005 mol) of Compound matrinic acid (DM-100) in 10ml tetrahydrofuran suspension was added dropwise within 1h, the reaction solution was heated and refluxed for 3h. TLC showed the end of reaction, the reaction solution was added to 1.8ml of water for quenching under condition of ice water bath. After filtration, the filter cake was repeatedly refluxed with ethyl acetate, after filtration, the filtrates were combined and evaporated to dryness, the obtained solid was recrystallized with petroleum ether/ethyl acetate to obtain Compound kurarinol (DM-200) 0.45g(yield: 35.7%), melting point: 155.9-156.7°C.
MS-ESI (M/Z): 253.4 [M+H]⁺
¹H-NMR (CD₃OD, δ ppm): 3.494-3.526 (2H, t, *J*=12.8), 3.085-3.146 (1H, t, *J*=24.4), 2.999-3.019 (1H, t, *J*=8), 2.699-2.768 (2H, m), 2.516-2.546 (1H, q, *J*=12), 2.113 (1H, s), 1.871-1.965 (3H, m), 1.205-1.730 (16H, m).

### Example 4: Synthesis of N-oxidized kurarinol (DM-2001):

0.48g(0.013 mol) of lithium aluminum tetrahydride was suspended in 40 ml of tetrahydrofuran, kept at 50°C. 0.7g (0.0025 mol) of Compound oxy matrinic acid (DM-1001) in 10ml tetrahydrofuran suspension was added dropwise within 1h, the reaction solution was heated and refluxed for 3h. TLC showed the end of reaction, the reaction solution was added to 0.9ml of water for quenching under condition of ice water bath. After filtration, the filter cake was repeatedly refluxed with ethyl acetate, after filtration, the filtrates were combined and evaporated to dryness, the obtained solid was recrystallized with petroleum ether/ethyl acetate to obtain Compound N-oxidized kurarinol (DM-2001) of 0.2g(yield: 29.8%), melting point: 142.7-144.6°C.
MS-ESI (M/Z): 269.2 [M+H]⁺, 251.2 [M-18+H]⁺
¹H-NMR (CD₃OD, δ ppm): 3.495-3.527 (2H, t, *J*=12.8), 3.090-3.151 (1H, t, *J*=24.4), 3.006-3.052 (1H, t, *J*=18.4), 2.700-2.769 (2H, m), 2.522-2.562 (1H, q, *J*=16), 2.114 (1H, s), 1.872-1.966 (3H, m), 1.205-1.743 (16H, m).
IR (KBr, cm⁻¹): 3272 (N-H, ν),3091 (CH₂-OH, v).

### Synthesis of N-substituted matrinic acid:

### Example 5: Synthesis of N-acetyl matrinic acid (DM-101)

### Step 1. Synthesis of matrinic acid (DM-100):

19.84g(0.08 mol) of matrine was provided, added to an aqueous solution of 26.88g (0.48 mol, 6 eq.) of potassium hydroxide (KOH) in 500 ml water, heated and refluxed for 9h, then reacted at room temperature overnight. The reaction solution was cooled with ice-water bath, adjusted with 3N hydrochloric acid to PH5-6, concentrated in reduced pressure to dryness to obtain a crude product of matrinic acid (DM-100), this light yellow solid without purification was added to 500 ml of methanol and used in the next step.

### Step 2.Synthesis of diphenylmethyl ester of matrinic acid (DM-100P):

To a mixture of 23.52g(0.12 mol, 1.5 eq.) of benzophenone hydrazine and 26.64g(0.12 mol) of yellow mercury oxide, 300 ml of petroleum ether with boiling range of 60°C-90°C was added, stirred and reacted at room temperature for 6h, to obtain dark violet petroleum ether solution of diphenyldiazomethane. This solution was filtered to the methanol solution of matrinic acid (DM-100) obtained in the previous step, the resultant mixture solution reacted at room temperature until the disappearance of violet. After filtration, the filtrate was concentrated to dryness, the obtained residue was soaked with petroleum ether, filtered to obtain crude diphenylmethyl ester of matrinic acid (DM-100P) product, which was directly used in the next reaction without purification. Melting point: 199.6-202.0°C.

### Step 3. Synthesis of N-acetyl matrinic acid (DM-101):

2g (0.0046 mol) of diphenylmethyl ester of matrinic acid (DM-100P) was dissolved in 50 ml of dichloromethane, added with 2 g of anhydrous potassium carbonate, added dropwise with 329.2µl (0.0046 mol) of acetyl chloride in 10 ml dichloromethane solution under ice-water bath. After dropwise addition, the reaction was performed at room temperature until TLC showed the disappearance of raw material spot. After filtration, the filter cake was washed with dichloromethane, the filtrates were combined and evaporated to dryness, the obtained oily substance was dissolved in 10 ml of m-cresol, heated and reacted at 80°C for 8h-9h. The reaction solution was cooled to room temperature, added with 50 ml of methyl isobutyl ketone for dilution, sufficiently extracted with water. The water layer was evaporated to dryness, and the resultant residue was recrystallized with ethanol/acetone to obtain white solid of 92 mg. (yield: 6.5%), melting point: 196.8-198.9°C.
MS-ESI (M/Z): 309.3 [M+H]⁺
¹H-NMR (500 MHz,CD₃OD, δ ppm): 3.919 (1H, s), 3.730-3.759 (1H, m), 3.403-3.481 (3H, m), 3.257 (1H, s), 3.068-3.092 (1H, d), 2.869-2.955 (1H, m), 2.765-2.776 (2H, m), 2.318-2.375 (2H, m), 2.235-2.268 (1H, m), 1.998-2.053 (3H, d), 1.626-1.847 (11H, m).

### Example 6: Synthesis of N-p-tosyl matrinic acid (DM-102):

According to the procedure of step 3 of Example 5, the obtained corresponding ester of matrinic acid (for example, diphenylmethyl ester of matrinic acid) reacted with p-toluenesulfonyl chloride under the above reaction conditions, and the removal of protecting group was performed using 10 ml of m-cresol, heating to 70-80°C and reacting for 8h-9h. The target product was recrystallized with ethanol/acetone to obtain a white solid of 150 mg. (yield: 7.8%), melting point: 239.1-241.5°C.
MS-ESI (M/Z): 421.5 [M+H]⁺
¹H-NMR (CD₃OD, δ ppm): 7.705-7.725 (2H, d, *J*=8, Ar-H), 7.377-7.397 (2H, d, *J*=8, Ar-H), 3.636-3.684 (1H, m), 3.332-3.473 (5H, m), 2.877-2.960 (2H, m), 2.398 (3H, s), 2.311-2.350 (1H, m), 1.751-2.096 (11H, m), 1.318-1.464 (3H, m), 1.165-1.210 (1H, m).

### Example 7: Synthesis of N-(4-methoxvbenzenesulfonyl) matrinic acid (DM-103):

According to the procedure of step 3 of Example 5, the obtained corresponding ester of matrinic acid (for example, diphenylmethyl ester of matrinic acid) reacted with 4-methoxybenzenesulfonyl chloride under the above reaction conditions, and the removal of protecting group was performed using 10 ml of m-cresol, heating to 110°C and reacting for 8h-9h. The target product was separated with silica gel column chromatograph to obtain a yellow brown solid of 430 mg. (yield: 21.3%), melting point: 86.4°C, decomposition.
MS-ESI (M/Z): 437.2 [M+H]⁺
¹H-NMR (CD₃OD, δ ppm): 6.158-6.175 (2H, d, J=8.5 Hz), 5.464-5.481 (2H, d, J=8.5 Hz), 2.252 (3H, s), 1.990-2.001 (1H, m), 1.755-1.788 (2H, m), 1.504-1.559 (2H, t, J= 27.5), 1.370 (1H, s), 1.336-1.436 (7H, m).

### Example 8: Syntheis of N-chloroacetyl matrinic acid (DM-104):

According to the procedure of step 3 of Example 5, the obtained corresponding ester of matrinic acid (for example, diphenylmethyl ester of matrinic acid) reacted with chloroacetyl chloride under the above reaction conditions, and the removal of protecting group was performed using 10 ml of m-cresol, heating to 80°C and reacting for 8h-9h. The target compound was separated with silica gel column chromatograph to obtain 20 mg. (yield: 1.3%), melting point: 206.3°C, decomposition.
MS-ESI (M/Z): 343.3 [M+H]⁺, 345.3 [M+2+H]⁺, (Cl₃₅,Cl₃₇)
¹H-NMR (500 MHz, CD₃OD, δ ppm): 4.302 (1H, s), 4.176 (1H, s), 4.094 (1H, s), 3.410 (1H, s), 3.283 (2H, s), 2.891-2.940 (2H, q, *J*=24.5), 2.383 (1H, s), 2.271-2.324 (2H, m), 2.037 (1H, s), 1.150-1.890 (14H, m).

### Example 9: Synthesis of N-(2-m-methylphenoxy)acetyl matrinic acid (DM-104a):

According to the procedure of step 3 of Example 5, the obtained corresponding ester of matrinic acid (for example, diphenylmethyl ester of matrinic acid) reacted with chloroacetyl chloride (or bromoacetyl bromide), and the removal of protecting group was performed using 10 ml of m-cresol, heating to 50°C and reacting for 3h. The final product was separated with silica gel column chromatograph to obtain an off-white solid of 21 mg. Mass spectrum showed a molecular weight that chlorine (or bromine) atom was substituted with m-methylphenoxy, which indicated the substitution reaciton with m-cresol during the procedure of removing the protecting group. (yield: 1.1%), melting point: 91.7-93.9°C.
MS-ESI (M/Z): 415.4 [M+H]⁺
¹H-NMR (500 MHz, CD₃OD, δ ppm): 7.883 (1H, s), 7.503 (2H, s), 7.412 (1H, s), 5.482 (2H, s), 4.829 (1H,s), 4.123-4.165 (1H, m), 3.919-3.938 (1H, m), 3.637 (1H, m), 3.440 (1H, m), 3.065-3.170 (1H, m), 3.010 (3H, s), 2.952 (2H, s), 2.190-2.738 (16H, m).

### Example 10: Synthesis of N-(2-hydroxyl)acetylmatrinicacid (DM-105):

According to the procedure of step 3 of Example 5, the obtained corresponding ester of matrinic acid (for example, diphenylmethyl ester of matrinic acid) reacted with 2-hydroxylacetyl chloride under the above reaction conditions, the acylating reagent was 2-bromoacetyl bromide, and the removal of protecting group was performed using 10 ml of m-cresol, heating to 80°C and reacting for 8h-9h. The final product was separated with silica gel column chromatograph to obtain a light yellow solid of 35 mg. Mass spectrum showed a molecular weight that bromine atom was substituted with hydroxyl, which may be due to hydrolysis during hearting procedure. (yield: 2.3%), melting point: 217.1°C, decomposition.
MS-ESI (M/Z): 325.4 [M+H]⁺
¹H-NMR (500 MHz, CD₃OD, δ ppm): 3.526-3.568 (2H, q, *J*=21), 3.443 (1H, s), 3.319-3.385 (1H, m), 3.269-3.295 (2H, m), 3.108 (1H, m), 2.905-2.948 (2H, m), 2.380 (1H, s), 2.269-2.307 (2H, m), 2.049 (1H, s), 1.524-1.798 (11H, m), 1.104-1.133 (2H, t, *J*=14.5).

### Example 11: Synthesis of N-formylmatrinic acid (DM-106a):

According to the procedure of step 3 of Example 5, the obtained corresponding ester of matrinic acid (for example, diphenylmethyl ester of matrinic acid) reacted with 2-bromopropionyl bromide under the above reaction conditions, and the removal of protecting group was performed using 10 ml of m-cresol, heating to 110°C and reacting for 8h-9h. The final product was recrystallized with ethanol/acetone to obtain light violet solid of 85 mg. (yield: 5.4%), melting point: 213.7°C, decomposition.
MS-ESI (M/Z): 295.5 [M+H]⁺
¹H-NMR (400MHz, CD₃OD, δ ppm):¹H NMR (400 MHz, CD₃OD): δ 9.56 (s, 1H), 4.65 (s, 1H), 4.3 3-4.40 (m, 2H), 4.16-4.21 (m, 1H), 4.05 (s, 2H), 3.89-3.91 (d, *J* = 9.6 Hz, 1H), 3.69-3.74 (m, 2H), 3.06-3.16 (m, 3H), 2.82-2.85 (d, *J*= 12.0 Hz,1H), 2.30-2.67 (m, 11H).
IR: v 3506 (OH), 2923, 2735 (CH₂), 1738, 1618 (C=O), 1087 (C-O) cm⁻¹.

### Example 12: Synthesis of N-(2-m-methylphenoxy)propionylmatrinic acid (DM-106):

According to the procedure of step 3 of Example 5, the obtained corresponding ester of matrinic acid (for example, diphenylmethyl ester of matrinic acid) reacted with 2-bromopropionyl bromide under the above reaction conditions, and the removal of protecting group was performed using 10 ml of m-cresol, heating to 50°C and reacting for 3d. The final product was separated with silica gel column chromatograph to obtain a white solid of 360 mg. Mass spectrum showed the molecular weight that bromine atom was replaced with m-methylphenoxy, which indicated the substitution reaction with m-cresol during the procedure of removing protecting group. (yield: 18.2%), melting point: 95.8-97.2°C.
MS-ESI (M/Z): 429.4 [M+H]⁺
¹H-NMR (500 MHz, CD₃OD, δ ppm): 7.076-7.106 (1H, t, *J*=15), 6.730-6.774 (3H, m), 5.178-5.217 (1H, q, *J*=19.5), 3.700-3.740 (1H, m), 3.322-3.422 (3H, m), 3.125 (2H, s), 2.877-2.985 (2H, m), 2.331-2.366 (1H, d, *J*=27.5), 2.248 (3H, s), 1.564-1.864 (18H, m).
IR (KBr, cm⁻¹): 3410 (CO-OH, v), 1719 (-CO, v).

### Example 13: Synthesis of N-benzoyl matrinic acid (DM-107):

According to the procedure of step 3 of Example 5, the obtained corresponding ester of matrinic acid (for example, diphenylmethyl ester of matrinic acid) reacted with benzoyl chloride under the above reaction conditions, and the removal of protecting group was performed using 10 ml of m-cresol, heating to 110°C and reacting for 8h-9h. The target compound was separated with silica gel column chromatograph to obtain a light yellow solid of 150mg. (yield: 8.8%), melting point: 84.6-87.8°C.
MS-ESI (M/Z): 371.5 [M+H]⁺
¹H-NMR (500 MHz, CD₃OD, δ ppm): 7.396-7.410 (5H, d, *J*=7), 3.388-3.406 (1H, t, *J*=9), 2.829-2.903 (2H, q, *J*=37), 2.340 (1H, s), 2.243-2.253 (2H, d, *J*=5), 2.073-2.097 (2H, m), 1.916 (2H, s), 1.813 (1H, s), 1.354-1.715 (12H, m), 1.232 (1H, s).
IR (KBr, cm⁻¹): 3056 (CO-OH, v), 1708 (-CO, v).

### Example 14: Synthesis of N-benzylmatrinic acid (DM-108):

According to the procedure of step 3 of Example 5, the obtained corresponding ester of matrinic acid (for example, diphenylmethyl ester of matrinic acid) reacted with benzyl chloride under the above reaction conditions, and the removal of protecting group was performed using 10 ml of m-cresol, heating to 110°C and reacting for 8h-9h. The target compound was separated with silica gel column chromatograph to obtain a light yellow solid of 180mg. (yield: 10.4%), melting point: 121.3-124.1°C.
MS-ESI (M/Z): 357.5 [M+H]⁺
¹H-NMR (CD₃OD, δ ppm): 7.535-7.543 (2H, d, *J*=3.2, Ar-H), 7.451-7.467 (3H, t, *J*=6.4, Ar-H), 4.173 (2H, s), 3.830 (1H, m), 3.548 (1H, s), 3.351-3.427 (2H, t, *J*=30.4), 2.912-3.017 (3H, m), 2.439-2.471 (2H, t, *J*=12.8), 2.331 (2H, s), 2.131-2.165 (2H, d, *J*=13.6), 1.683-1.985 (10H, m), 1.551-1.585 (1H, d, *J*=113.6).
IR (KBr, cm⁻¹): 3386 (CO-OH, v), 1722 (-CO, v).

### Example 15: Synthesis of N-benzyl oxymatrinic acid (DM-1081):

According to the procedure of step 3 of Example 5, the obtained corresponding ester of oxymatrinic acid (for example, diphenylmethyl ester of oxymatrinic acid) reacted with benzoyl chloride under the above reaction conditions, and the removal of protecting group was performed using 10 ml of m-cresol, heating to 80°C and reacting for 8h-9h. The target compound was separated with silica gel column chromatograph to obtain a brown solid of 70mg. (yield: 4.2%), melting point: 121.5°C, decomposition.
MS-ESI (M/Z): 373.5 [M+H]⁺
¹H-NMR (CD₃OD, δ ppm): 7.334-7.406 (5H, m), 4.047-4.080 (1H, d, *J*=13.2), 3.983-4.016 (1H, d, *J*=13.2), 3.828-3.851 (1H, t, *J*=9.2), 3.003-3.061 (1H, m),2.822 (1H, s), 2.659 (1H, s),2.446-2.471 (1H, m), 2.152-2.287 (7H, m), 1.744-1.958 (4H, m), 1.529-1.608 (6H, m), 1.413-1.435 (2H, m).

### Example 16: Synthesis of N-benzenesulfonyl matrinic acid (DM-109):

According to the procedure of step 3 of Example 5, the obtained corresponding ester of matrinic acid (for example, diphenylmethyl ester of matrinic acid) reacted with benzenesulfonyl chloride under the above reaction conditions, and the removal of protecting group was performed using 10 ml of m-cresol, heating to 70-80°C and reacting for 8h-9h. The target product was recrystallized with ethanol/acetone to obtain a white solid of 210mg. (yield: 11.2%), melting point: 208.5-210.3°C.
MS-ESI (M/Z): 407.5 [M+H]⁺
¹H-NMR (CD₃OD, δ ppm): 7.834-7.855 (2H, d, *J*=7.2), 7.558-7.667 (3H, m), 3.671-3.720 (1H, m), 3.344-3.498 (5H, m), 2.900-2.984 (2H, m), 2.350-2.369 (1H, m), 1.734-2.123 (12H, m), 1.319-1.477 (3H, m), 1.119-1.238 (1H, m).
IR (KBr, cm⁻¹): 3062 (CO-OH, v), 1741 (-CO, v).

### Example 17: Synthesis of N-cinnamoyl matrinic acid (DM-110):

According to the procedure of step 3 of Example 5, the obtained corresponding ester of matrinic acid (for example, diphenylmethyl ester of matrinic acid) reacted with cinnamoyl chloride under the above reaction conditions, and the removal of protecting group was performed using 10 ml of m-cresol, heating to 80°C and reacting for 8h-9h. The target compound was recrystallized with ethanol/acetone to obtain an off-white solid of 78mg. (yield: 4.3%), melting point: 217.2-219.8°C.
MS-ESI (M/Z): 397.5 [M+H]⁺
¹H-NMR (500 MHz, CD₃OD, δ ppm): 7.536-7.550 (1H, d), 7.439-7.452 (2H, t), 7.252-7.324 (3H, m), 6.424-6.456 (1H, d), 2.645 (2H, t), 2.123-2.165 (2H, m), 2.032 (1H, s), 1.426-1.894 (19H, m).

### Example 18: Synthesis of N,N'-dimethyl matrinic acid (DM-111):

According to the procedure of step 3 of Example 5, the obtained corresponding ester of matrinic acid (for example, diphenylmethyl ester of matrinic acid) reacted with methyl iodide under the above reaction conditions, and the removal of protecting group was performed using 10 ml of m-cresol, heating to 110°C and reacting for 8h-9h. The final product was separated with silica gel column chromatograph to obtain a yellow brown solid of 150mg. (yield: 11%), melting point: 83.9-85.2°C.
MS-ESI (M/Z): 295.5M⁺
¹H-NMR (500 MHz, DMSO, δ ppm): 3.701-3.751 (1H, t, *J*=25), 3.482-3.494 (1H, d, *J*=6), 3.186 (3H, s), 2.953 (3H, s), 2.694-2.763 (2H, m), 2.223-2.378 (3H, m), 2.051 (2H, m), 1.802-1.948 (5H, m), 1.346-1.601 (11H, m).

### Example 19: Synthesis of N,N'-dimethyl oxymatrinic acid (DM-1111):

According to the procedure of step 3 of Example 5, the obtained corresponding ester of oxymatrinic acid (for example, diphenylmethyl ester of oxymatrinic acid) reacted with methyl iodide under the above reaction conditions, and the target product was separated with silica gel column chromatograph to obtain a brown solid of 85mg. (yield: 6.1 %), melting point: 59.7°C, decomposition.
MS-ESI (M/Z): 311.4 M⁺
¹H-NMR (500 MHz, CD₃OD, δ ppm): 3.135-3.152 (5H, m), 3.020-3.098 (2H, m), 2.950 (3H, d), 1.458-2.429 (20H, m), 1.236-1.289 (1H, m).
IR (KBr, cm⁻¹): 3398 (CO-OH, v), 1752 (-CO, v).

### Example 20: Synthesis of N-ethyl matrinic acid (DM-112):

According to the procedure of step 3 of Example 5, the obtained corresponding ester of matrinic acid (for example, diphenylmethyl ester of matrinic acid) reacted with ethyl bromide under the above reaction conditions, and the removal of protecting group was performed using 10 ml of m-cresol, heating to 110°C and reacting for 8h-9h. The final product was separated with silica gel column chromatograph to obtain a yellow brown solid of 95mg. (yield: 7%), melting point: 90.8-93.2°C.
MS-ESI (M/Z): 295.2 [M+H]⁺
¹H-NMR (CD₃OD, δ ppm): 3.525-3.568 (2H, q, *J*=21.5), 3.391-3.429 (2H, m), 3.047-3.068 (1H, d, *J*=10.5), 2.715-2.790 (1H, m), 2.661-2.702 (2H, m), 2.126-2.299 (5H, m), 1.437-2.029 (15H, m), 1.212-1.240 (1H, t, *J*=14).
IR (KBr, cm⁻¹): 3381 (CO-OH, v), 1635 (-CO, v).

### Example 21: Synthesis of N-propyl matrinic acid (DM-113):

According to the procedure of step 3 of Example 5, the obtained corresponding ester of matrinic acid (for example, diphenylmethyl ester of matrinic acid) reacted with propyl iodide under the above reaction conditions, and the removal of protecting group was performed using 10 ml of m-cresol, heating to 110°C and reacting for 8h-9h. The target product was separated with silica gel column chromatograph to obtain a yellow brown solid of 70mg. (yield: 4.9%), melting point: 82.3-84.7°C.
MS-ESI (M/Z): 309.5 [M+H]⁺
¹H-NMR (500 MHz, DMSO, δ ppm): 3.201 (1H, s), 3.058-3.103 (1H, t, *J*=22.5), 2.902 (1H, s), 2.625-2.806 (4H, m), 2.235-2.246 (2H, d, *J*=5.5), 2.047 (2H, s), 1.741-1.893 (4H, m), 1.450-1.621 (10H, m), 1.333-1.355 (3H, d, *J*=11), 0.862-0.891 (3H, t, *J*=14.5).

### Example 22: Synthesis of N-cyclopropylmethyl matrinic acid (DM-115):

According to the procedure of step 3 of Example 5, the obtained corresponding ester of matrinic acid (for example, diphenylmethyl ester of matrinic acid) reacted with cyclopropylmethyl bromide under the above reaction conditions, and the removal of protecting group was performed using 10 ml of m-cresol, heating to 110°C and reacting for 8h-9h. The target product was separated with silica gel column chromatograph to obtain a yellow brown solid of 95mg. (yield: 6.4%), melting point: 75.8-77.6°C.
MS-ESI (M/Z): 321.2 [M+H]⁺
¹H-NMR (500 MHz, DMSO, δ ppm): 3.251-3.275 (2H, d, *J*=12), 2.899-3.147 (4H, m), 1.825-2.264 (19H, m), 1.234 (1H, s), 0.825-0.840 (2H, d, *J*=7.5), 0.491 (2H, s).

### Example 23: Synthesis of N-cyclopropylmethyl oxymatrinic acid (DM-1151):

According to the procedure of step 3 of Example 5, the obtained corresponding ester of oxymatrinic acid (for example, diphenylmethyl ester of oxymatrinic acid) reacted with methyl iodide under the above reaction conditions, and the target product was separated with silica gel column chromatograph to obtain a brown solid of 75mg. (yield: 5%), melting point: 126.6-129.5°C.
MS-ESI (M/Z): 337.5 [M+H]⁺
¹H-NMR (500 MHz, CD₃OD, δ ppm): 3.821 (1H, s), 2.868-3.101 (4H, m), 2.672 (1H, s), 2.451 (4H, s), 2.084-2.164 (7H, m), 1.379-1.955 (9H, m), 1.036 (1H, s), 0.652-0.70 (2H, m), 0.219-0.298 (2H, m).
IR (KBr, cm⁻¹): 3371 (CO-OH, v).

### Example 24: Synthesis of N-hydroxylethyl matrinic acid (DM-117):

According to the procedure of step 3 of Example 5, the obtained corresponding ester of matrinic acid (for example, diphenylmethyl ester of matrinic acid) reacted with 2-bromoethanol under the above reaction conditions, and the removal of protecting group was performed using 10 ml of m-cresol, heating to 80°C and reacting for 8h-9h. The target product was recrystallized with ethanol/acetone to obtain a brown solid of 50mg. (yield: 6.9%), melting point: 118.9-120.6°C.
MS-ESI (M/Z): 311.5 [M+H]⁺
¹H-NMR (CD₃OD, δ ppm): 3.791-3.817 (2H, t, *J*=10.4), 2.795-2.857 (2H, t, *J*=24.8), 2.020-2.262 (8H, m), 1.866-1.884 (2H, d, *J*=7.2), 1.454-1.703 (14H, m).

### Example 25: Synthesis of N-(3-nitrobenzoyl) matrinic acid (DM-121):

According to the procedure of step 3 of Example 5, the obtained corresponding ester of matrinic acid (for example, diphenylmethyl ester of matrinic acid) reacted with nitrobenzoyl chloride under the above reaction conditions, and the removal of protecting group was performed using 10 ml of m-cresol, heating to 80°C and reacting for 8h-9h. The target product was separated with silica gel column chromatograph to obtain a light yellow solid of 150mg. (yield: 7.8%), melting point: 82.0-83.9°C.
MS-ESI (M/Z): 416.5 [M+H]⁺
¹H-NMR (500 MHz, CD₃OD, δ ppm): 8.345 (1H, s), 8.261-8.287 (1H, m), 7.783-7.798 (1H, d, *J*=7.5), 7.650-7.682 (1H, t, *J*=8), 3.387 (1H, m), 2.830-2.930 (1H, dd, *J*=11.5, 38.5), 2.232 (3H, s), 1.988 (3H, s), 1:376-1.843 (15H, m).
IR (KBr, cm-¹): 3070 (CO-OH, ν), 1721 (-CO, v).

### Example 26: Synthesis of N-(4-methoxybenzyl) matrinic acid (DM-122):

According to the procedure of step 3 of Example 5, the obtained corresponding ester of matrinic acid (for example, diphenylmethyl ester of matrinic acid) reacted with 4-methoxybenzyl chloride under the above reaction conditions, and the removal of protecting group was performed using 10 ml of m-cresol, heating to 110°C and reacting for 8h-9h. The target product was separated with silica gel column chromatograph to obtain a light yellow solid of 0.6g. (yield: 33.8%), melting point: 78.6-80.9°C.
MS-ESI (M/Z): 387.5 [M+H]⁺
¹H-NMR (500 MHz, CD₃OD, δ ppm): 7.387-7.404 (2H, d, *J*=8.5), 6.945-6.962 (2H, d, *J*=8.5), 4.660-4.685 (1H, d, *J*=12.5), 3.914-3.939 (1H, d, *J*=12.5), 3.740 (3H, s), 3.364-3.409 (1H, m), 3.115-3.186 (2H, m), 2.993-3.019 (1H, m), 2.820-2.855 (1H, m), 2.605 (2H, s), 2.363-2.446 (2H, m), 2.160-2.216 (2H, t, *J*=28), 2.030-2.056 (2H, d, *J*=13), 1.570-1.908 (10H, m), 1.487-1.513 (1H, d, *J*=13).

### Example 27: Synthesis of N-(4-methoxybenzyl) oxymatrinic acid (DM-1221):

According to the procedure of step 3 of Example 5, the obtained corresponding ester of oxy matrinic acid (for example, diphenylmethyl ester of oxymatrinic acid) reacted with 4-methoxybenzyl chloride under the above reaction conditions. The target product was separated with silica gel column chromatograph to obtain a brown solid of 90mg. (yield: 5%), melting point: 122.4-124.1°C.
MS-ESI (M/Z): 403.5 [M+H]⁺
¹H-NMR (500 MHz, CD₃OD, δ ppm): 6.744-6.759 (2H, d, *J*=7.5), 6.401-6.417 (2H, d, *J*=8), 3.497-3.523 (1H, d, *J*=113), 3.442-3.416 (1H, d, *J*=113), 3.296 (1H, s), 3.250 (3H, s), 2.509 (1H, s), 2.297 (1H, s), 2.144 (1H, s), 1.941 (1H, s), 1.662-1.768 (7H, m), 1.262-1.463 (4H, m), 1.054-1.085 (6H, m), 0.898 (2H, s).
IR (KBr, cm⁻¹): 3394 (CO-OH, v), 1652 (-CO, v).

### Example 28: Synthesis of N-(4-nitrobenzyl)matrinicacid (DM-123):

According to the procedure of step 3 of Example 5. The obtained corresponding ester of matrinic acid (for example, diphenylmethyl ester of matrinic acid) reacted with 4-nitrobenzyl bromide under the above reaction conditions, and the removal of protecting group was performed using 10 ml of m-cresol, heating to 110°C and reacting for 8h-9h. The target product was separated with silica gel column chromatograph to obtain a brown solid of 60mg. (yield:3.2%), melting point: 198.6°C, decomposition.
MS-ESI (M/Z): 402.5 [M+H]⁺
¹H-NMR (CD₃OD, δ ppm): 8.139-8.161 (2H, d), 7.582-7.603 (2H, d), 4.209-4.244 (1H, d), 3.433-3.469 (1H, d), 2.973 (1H, s), 2.862-2.890 (1H, m), 2.554-2.659 (2H, t), 2.413-2.456 (2H, m), 1.982-2.203 (7H, m), 1.401-1.777 (11H, m).

### Example 29: Synthesis of N-(2-chlorobenzyl) matrinic acid (DM-124):

According to the procedure of step 3 of Example 5. The obtained corresponding ester of matrinic acid (for example, diphenylmethyl ester of matrinic acid) reacted with 2-chlorobenzyl chloride under the above reaction conditions, and the removal of protecting group was performed using 10 ml of m-cresol, heating to 110°C and reacting for 8h-9h. The target product was separated with silica gel column chromatograph to obtain a light yellow brown solid of 85mg. (yield:4.7%), melting point: 140.2-143.1°C.
MS-ESI (M/Z): 391.2 [M+H]⁺, 393.2 [(M+2)+H]⁺, (Cl₃₅,Cl₃₇)
¹H-NMR (CD3OD, δ ppm): 7.673-7.688 (1H, d), 7.528-7.547 (1H, d), 7.402-7.480 (2H, m), 4.442 (1H, s), 3.555 (1H, s), 3.359-3.432 (2H, t), 2.923-3.027 (4H, m), 2.419-2.450 (2H, t), 2.349 (2H, s), 1.698-2.158 (13H, m), 1.581-1.615 (1H, d).

### Example 30: Synthesis of N-(3-chlorobenzyl) matrinic acid (DM-125):

According to the procedure of step 3 of Example 5, the obtained corresponding ester of matrinic acid (for example, diphenylmethyl ester of matrinic acid) reacted with 3-chlorobenzyl chloride under the above reaction conditions, and the removal of protecting group was performed using 10 ml of m-cresol, heating to 110°C and reacting for 8h-9h. The target product was separated with silica gel column chromatograph to obtain a light brown solid of 50mg. (yield: 2.8%), melting point: 78.0-80.1°C.
MS-ESI (M/Z): 391.4 [M+H]⁺, 393.4 [(M+2)+H]⁺,(Cl₃₅,Cl₃₇)
¹H-NMR (CD₃OD, δ ppm): 7.575 (1H, s), 7.412-7.450 (3H, m), 4.680 (1H, s), 3.847 (1H, s), 3.373-3.456 (2H, m), 2.819-3.037 (4H, m), 2.401-2.434 (2H, t), 2.264-2.374 (2H, m), 1.550-1.919 (14H, m).
IR (KBr, cm⁻¹): 3386 (CO-OH, v), 1719 (-CO, v).

### Example 31: Synthesis of N-(4-chlorobenzyl) matrinic acid (DM-126):

According to the procedure of step 3 of Example 5,the obtained corresponding ester of matrinic acid (for example, diphenylmethyl ester of matrinic acid) reacted with 4-chlorobenzyl chloride under the above reaction conditions, and the removal of protecting group was performed using 10 ml of m-cresol, heating to 110°C and reacting for 8h-9h. The target product was separated with silica gel column chromatograph to obtain a light yellow brown solid of 50mg. (yield: 2.8%), melting point: 105.3-107.9°C.
MS-ESI (M/Z): 391.2 [M+H]⁺, 393.2 [(M+2)+H]⁺,(Cl₃₅,Cl₃₇)
¹H-NMR (CD₃OD, δ ppm): 7.416-7.437 (2H, d), 7.362-7.383 (2H, d), 4.535 (1H, s), 3.718 (1H, s), 3.307-3.422 (2H, m), 2.725-3.150 (4H, m), 2.335-2.367 (2H, t), 2.147-2.227 (2H, m), 1.502-2.071 (14H, m).
IR (KBr, cm⁻¹): 3395 (CO-OH, v), 1721 (-CO, v).

### Example 32: Synthesis of N-(3,4-dichlorobenzyl) matrinic acid (DM-127):

According to the procedure of step 3 of Example 5, the obtained corresponding ester of matrinic acid (for example, diphenylmethyl ester of matrinic acid) reacted with 3,4-dichlorobenzyl chloride under the above reaction conditions, and the removal of protecting group was performed using 10 ml of m-cresol, heating to 110°C and reacting for 8h-9h. The target product was separated with silica gel column chromatograph to obtain a yellow brown solid of 75mg. (yield:3.8%), melting point: 92.5-94.7°C.
MS-ESI (M/Z): 425.4 [M+H]⁺, 427.4 [(M+2)+H]⁺, 429.4 [(M+4)+H]⁺,(Cl₃₅,Cl₃₇)
¹H-NMR (500 MHz, DMSO, δ ppm): 7.977 (1H, s), 7.708-7.724 (1H, d), 7.566-7.581 (1H, d), 4.184 (1H, s), 3.589 (1H, s), 3.245-3.298 (2H, t), 2.861-2.903 (4H, m), 2.359 (4H, m), 1.503-2.031 (14H, m).
IR (KBr, cm⁻¹): 3386 (CO-OH, v), 1722 (-CO, v).

### Example 33: Synthesis of N-(4-bromobenzyl) matrinic acid (DM-128):

According to the procedure of step 3 of Example 5, the obtained corresponding ester of matrinic acid (for example, diphenylmethyl ester of matrinic acid) reacted with 4-bromobenzyl bromide under the above reaction conditions, and the removal of protecting group was performed using 10 ml of m-cresol, heating to 110°C and reacting for 8h-9h. The target product was separated with silica gel column chromatograph to obtain a yellow brown solid of 20mg. (yield:l%), melting point: 133.5-135.0°C.
MS-ESI (M/Z): 435.2 [M+H]⁺, 437.2 [(M+2)+H]⁺,(Br₇₉, Br₈₁)
¹H-NMR (CD₃OD, δ ppm): 7.481-7.502 (2H, d), 7.308-7.329 (2H, d), 4.221-4.250 (1H, d), 3.584-3.616 (1H, d), 3.023-3.103 (3H, m), 2.826 (2H, m), 2.513-2.607 (3H, m), 2.213-2.339 (2H, m), 2.108-2.133 (1H, d), 1.956-2.031 (2H, t), 1.427-838 (11H, m).

### Example 34: Synthesis of N-(2,3-dichlorobenzyl) matrinic acid (DM-129):

According to the procedure of step 3 of Example 5, the obtained corresponding ester of matrinic acid (for example, diphenylmethyl ester of matrinic acid) reacted with 2,3-dichlorobenzyl chloride under the above reaction conditions, and the removal of protecting group was performed using 10 ml of m-cresol, heating to 110°C and reacting for 8h-9h. The final product was separated with silica gel column chromatograph to obtain a yellow brown solid of 120mg. (yield:6.1 %), melting point: 112.0-114.2°C.
MS-ESI (M/Z): 425.4 [M+H]⁺, 427.2 [(M+2)+H]⁺, 429.3 [(M+4)+H]⁺,(Cl₃₅,Cl₃₇)
¹H-NMR (CD₃OD, δ ppm): 7.665-7.686 (1H, d), 7.558-7.562 (1H, d), 7.390-7.415 (1H, m), 4.196 (1H, s), 3.555 (1H, s), 3.327-3.402 (2H, t), 2.860-3.033 (4H, m), 2.328-2.392 (4H, m), 1.561-2.113 (14H, m).

### Example 35: Synthesis of N-(2-methyl-5-nitrobenzoyl) matrinic acid (DM-131):

According to the procedure of step 3 of Example 5, the obtained corresponding ester of matrinic acid (for example, diphenylmethyl ester of matrinic acid) reacted with 2-methyl-5-nitrobenzoyl chloride under the above reaction conditions, and the removal of protecting group was performed using 10 ml of m-cresol, heating to 80°C and reacting for 8h-9h. The target product was separated with silica gel column chromatograph to obtain a light yellow solid of 180mg. (yield: 9%), melting point: 86.8-89.0°C.
MS-ESI (M/Z): 430.6 [M+H]+
1H-NMR (CD30D, δ ppm): 8.559-8.565 (1H, d, J=2.4), 8.161-8.167 (1H, d, J=2.4), 7.438-7.460 (1H, d, J=8.8), 4.485-4.532 (3H, m), 2.023-2.395 (12H, m), 1.465-1.856 (12H, m).
IR (KBr, cm-1): 3040 (CO-OH, v), 1719 (-CO, v).

### Example 36: Synthesis of N-(4-fluorobenzyl) matrinic acid (DM-132)

According to the procedure of step 3 of Example 5, the obtained corresponding ester of matrinic acid (for example, diphenylmethyl ester of matrinic acid) reacted with 4-fluorobenzyl chloride under the above reaction conditions, and the removal of protecting group was performed using 10 ml of m-cresol, heating to 110°C and reacting for 8h-9h. The target product was separated with silica gel column chromatograph to obtain an off-white solid of 95mg. (yield:5.5%), melting point: 84.9-87.3°C.
MS-ESI (M/Z): 375.5 [M+H]⁺
¹H-NMR (CD₃OD, δ ppm): 7.409-7.443 (2H, m), 7.035-7.078 (2H, t), 4.314-4.347 (1H, d), 3.596-3.629 (1H, d), 3.101-3.250 (2H, m), 3.000 (1H, s), 2.880-2.944 (1H, t), 2.554-2.666 (3H, m), 2.270-2.319 (2H, m), 1.973-2.172 (3H, m), 1.533-1.868 (11H, m), 1.414-1.447 (1H, d).

### Example 37: Synthesis of N-(3-fluorobenzyl) matrinic acid (DM-133):

According to the procedure of step 3 of Example 5, the obtained corresponding ester of matrinic acid (for example, diphenylmethyl ester of matrinic acid) reacted with 3-fluorobenzyl chloride under the above reaction conditions, and the removal of protecting group was performed using 10 ml of m-cresol, heating to 110°C and reacting for 8h-9h. The target product was separated with silica gel column chromatograph to obtain a light yellow brown solid of 75mg. (yield:4.3%), melting point: 81.5-83.1°C.
MS-ESI (M/Z): 375.5 [M+H]⁺
¹H-NMR (CD₃OD, δ ppm): 7.424-7.479 (1H, m), 7.345-7.375 (2H, t), 7.142-7.189 (1H, m), 4.056-4.089 (1H, d), 3.656-3.689 (1H, d), 3.547 (1H, s), 3.326-3.402 (2H, t), 2.880-3.028 (3H, m), 2.372-2.439 (4H, m), 1.665-2.139 (13H, m), 1.542-1.577 (1H, d).

### Example 38: Synthesis of N-(4-cyanobenzyl) matrinic acid (DM-134):

According to the procedure of step 3 of Example 5, the obtained corresponding ester of matrinic acid (for example, diphenylmethyl ester of matrinic acid) reacted with 4-cyanobenzyl bromide under the above reaction conditions, and the removal of protecting group was performed using 10 ml of m-cresol, heating to 110°C and reacting for 8h-9h. The final product was separated with silica gel column chromatograph to obtain a light yellow brown solid of 70mg. (yield:4%), melting point: 88.0-90.9°C.
MS-ESI (M/Z): 382.5 [M+H]⁺
¹H-NMR (CD₃OD, δ ppm): 7.583-7.603 (2H, d), 7.488-7.508 (2H, d), 4.147-4.182 (1H, d), 3.383-3.418 (1H, d), 3.128-3.185 (2H, t), 3.055 (1H, s), 2.875-2.902 (1H, t), 2.598-2.695 (3H, m), 2.372-2.415 (1H, m), 2.073-2.146 (3H, m), 1.952-1.982 (2H, d), 1.508-1.770 (10H, m), 1.356-1.389 (1H, d).

### Example 39: Synthesis of N-(2-fluorobenzyl)matrinicacid (DM-135):

According to the procedure of step 3 of Example 5, the obtained corresponding ester of matrinic acid (for example, diphenylmethyl ester of matrinic acid) reacted with 2-fluorobenzyl chloride under the above reaction conditions, and the removal of protecting group was performed using 10 ml of m-cresol, heating to 110°C and reacting for 8h-9h. The target product was separated with silica gel column chromatograph to obtain a light yellow solid of 50mg. (yield:2.9%), melting point: 90.3-92.7°C.
MS-ESI (M/Z): 375.4[M+H]⁺
¹H-NMR (CD₃OD, δ ppm): 7.570-7.599 (1H, t), 7.427-7.467 (1H, m), 7.222-7.252 (1H, t), 7.166-7.203 (1H, t), 4.718-4.744 (1H, d), 4.066-4.092 (1H, d), 3.844-3.861 (1H, d), 3.521-3.570 (1H, t), 3.474 (1H, s), 3.298-3.367 (2H, m), 2.888-2.977 (3H, m), 2.389-2.414 (3H, t), 2.296-2.318 (1H, d), 1.666-2.109 (11H, m), 1.549-1.577 (1H, d).
IR (KBr, cm⁻¹): 3424 (CO-OH, v), 1721 (-CO, v).

### Example 40: Synthesis of N-(4-ethenyl benzyl) matrinic acid (DM-136):

According to the procedure of step 3 of Example 5, the obtained corresponding ester of matrinic acid (for example, diphenylmethyl ester of matrinic acid) reacted with 4-ethenyl benzyl chloride under the above reaction conditions, and the removal of protecting group was performed using 10 ml of m-cresol and reacting at room temperature until TLC showed the substantial completion of reaction. The final product was separated with silica gel column chromatograph to obtain a white solid of 110mg. (yield:6.2%), melting point: 190.2°C, decomposition.
MS-ESI (M/Z): 397.5(382.5+15) [M+H]⁺
¹H-NMR (500 MHz, CD₃OD, δ ppm): 7.523 (4H, s), 6.708-6.765 (1H, m), 5.819-5.855 (1H, d), 5.279-5.301 (1H, d), 4.123 (1H, s), 3.558-3.571 (1H, s), 3.357-3.477 (2H, m), 2.974-3.024 (3H, m), 2.866-2.894 (1H, t), 2.476-2.487 (2H, d), 2.245-2.335 (2H, m), 2.032-2.064 (2H, m), 1.682-2.032 (11H, m), 1.560-1.589 (1H, d).

### Example 41: Synthesis of N-(3 -nitrobenzyl) matrinic acid (DM-137):

According to the procedure of step 3 of Example 5, the obtained corresponding ester of matrinic acid (for example, diphenylmethyl ester of matrinic acid) reacted with 3-nitrobenzyl bromide under the above reaction conditions, and the removal of protecting group was performed using 10 ml of m-cresol, heating to 110°C and reacting for 8h-9h. The target product was separated with silica gel column chromatograph to obtain a brown solid of 60mg. (yield:3.2%), melting point: 92.5-94.7°C.
MS-ESI (M/Z): 402.5 [M+H]⁺
¹H-NMR (500 MHz, CD₃OD, δ ppm): 8.230 (1H, s), 8.087-8.103 (1H, d), 7.741-7.756 (1H, d), 7.523-7.554 (1H, t), 4.254-4.282 (1H, d), 3.425-3.453 (1H, d), 3.315-3.524 (2H, d), 2.849-2.952 (3H, m), 2.513-2.590 (2H, m), 2.322-2.349 (1H, t), 2.190-2.275 (4H, m), 1.591-1.810 (11H, m), 1.462-1.490 (1H, d).

### Example 42: Synthesis of N-(2-methylbenzyl) matrinic acid (DM-138):

According to the procedure of step 3 of Example 5, the obtained corresponding ester of matrinic acid (for example, diphenylmethyl ester of matrinic acid) reacted with 2-methylbenzyl bromide under the above reaction conditions, and the removal of protecting group was performed using 10 ml of m-cresol, heating to 110°C and reacting for 8h-9h. The target product was separated with silica gel column chromatograph to obtain a brown solid of 85mg. (yield:5%), melting point: 110.9-113.6°C.
MS-ESI (M/Z): 371.4 [M+H]⁺
¹H-NMR (500 MHz, CD₃OD, δ ppm): 7.354-7.367 (1H, d), 7.162-7.204 (3H, m), 4.352-4.418 (1H, d), 3.672-3.713 (1H, d), 3.094-3.115 (1H, d), 3.034-3.056 (1H, d), 2.839 (2H, s), 2.499-2.592 (3H, m), 2.375 (3H, s), 2.227-2.323 (2H, m), 2.049-2.098(2H, t), 1.987-2.013 (1H, d), 1.863-1.885 (1H, m), 1.566-1.792 (9H, m), 1.517-1.534 (1H, d), 1.389-1.411 (1H, d).

### Example 43: Synthesis of N-(3-methylbenzyl) matrinic acid (DM-139):

According to the procedure of step 3 of Example 5, the obtained corresponding ester of matrinic acid (for example, diphenylmethyl ester of matrinic acid) reacted with 3-methylbenzyl chloride under the above reaction conditions, and the removal of protecting group was performed using 10 ml of m-cresol, heating to 110°C and reacting for 8h-9h. The target product was separated with silica gel column chromatograph to obtain a light yellow solid of 65mg. (yield:3.8%), melting point: 103.3-105.2°C.
MS-ESI (M/Z): 371.5 [M+H]⁺
¹H-NMR (CD₃OD, δ ppm): 7.386 (1H, s), 7.327-7.338 (2H, d), 7.273-7.312 (1H, d), 4.194-4.216 (1H, d), 4.123 (1H, d), 3.808-3.862 (1H, t), 3.592 (1H, s), 3.347-3.418 (3H, m), 2.934-3.040 (3H, m), 2.409-2.451 (4H, t), 2.349 (3H, s), 1.709-2.154 (11H, m), 1.542-1.569 (1H, d).

### Example 44: Synthesis of N-(4-methylbenzyl) matrinic acid (DM-140):

According to the procedure of step 3 of Example 5, the obtained corresponding ester of matrinic acid (for example, diphenylmethyl ester of matrinic acid) reacted with 4-methylbenzyl bromide under the above reaction conditions, and the removal of protecting group was performed using 10 ml of m-cresol, heating to 110°C and reacting for 8h-9h. The target product was separated with silica gel column chromatograph to obtain a light yellow solid of 70mg. (yield:4.1%), melting point: 106.0-107.8°C.
MS-ESI (M/Z): 371.2 [M+H]⁺
¹H-NMR (500 MHz, CD₃OD, δ ppm): 7.421-7.437 (2H, d), 7.261-7.276 (2H, d), 4.191 (1H, s), 4.114 (1H, s), 3.802-3.856 (1H, t), 3.591 (1H, s), 3.348-3.418.(2H, m), 2.930-3.038 (3H, m), 2.393-2.453 (4H, m), 2.331 (3H, s), 2.125-2.153 (2H, d), 1.935-2.007 (3H, m), 1.652-1.864 (7H, m), 1.538-1.566 (1H, d).

### Example 45: Synthesis of N-(3-methoxy)benzyl matrinic acid (DM-142):

Referring to the method for synthesis of N-(4-methoxybenzyl) matrinic acid in Example 26, matrinic acid reacted with 3-methoxybenzyl chloride under the above conditions, and the removal of protecting group was performed using 10 ml of m-cresol, heating to 110°C and reacting for 8h-9h. The target product was separated with silica gel column chromatograph to obtain a light brown solid of 45mg. (yield: 2.5 %), melting point: 97.6-100:1°C.
MS-ESI (M/Z): 387.2 [M+H]⁺
¹H-NMR (500 MHz, CD₃OD, δ ppm): 7.331-7.363 (1H, t, *J*=16), 7.180 (1H, s), 7.101-7.116 (1H, d, *J*=7.5), 6.983-7.003 (1H, q, *J*=10), 4.200 (1H, s), 4.108 (1H, s), 3.793 (3H, s), 3.820-3.874 (1H, t, *J*=27), 3.616 (1H, s), 3.344-3.416 (2H, q, *J*=36), 2.926-3.056 (3H, m), 2.402-2.480 (4H, m), 1.675-2.152 (12H, m), 1.533-1.560 (1H, d, *J*=13.5).

### Example 46: Synthesis of N-(2-pyridyl)benzylmatrinic acid (DM-143):

According to the procedure of step 3 of Example 5, the obtained corresponding ester of matrinic acid (for example, diphenylmethyl ester of matrinic acid) reacted with 2-pyridylbenzyl chloride hydrochloride under the above reaction conditions, and the removal of protecting group was performed using 10 ml of m-cresol, heating to 50°C and reacting for 8h-9h(or reacting at room temperature until TLC showed the substantial completion of the reaction). The target product was separated with silica gel column chromatograph to obtain a light brown solid of 20mg. (yield:1.2%), melting point: 89.9-91.4°C.
MS-ESI (M/Z): 358.5[M+H]+
1H-NMR (400 MHz, CD30D, δ ppm): 8.714 (1H, d, J=4.8), 8.06 (1H, t, J=7.6), 7.76 (1H, d, J=8), 7.57-7.60 (1H, m), 4.99 (1H, d, J=14.8), 4.47 (1H, d, J=14.8), 4.23 (1H, d, J=12.4), 3.89 (1H, t, J=13.6), 3.63 (1H, s), 3.34-3.42 (2H, m), 3.18-3.23 (1H, m), 2.96-3.07 (2H, m), 2.35-2.61 (4H, m),1.60-2.15 (12H, m).

### Example 47: Synthesis of N-(3-pyridyl) benzyl matrinic acid (DM-144)

According to the procedure of step 3 of Example 5, the obtained corresponding ester of matrinic acid (for example, diphenylmethyl ester of matrinic acid) reacted with 3-pyridylbenzyl chloride hydrochloride under the above reaction conditions, and the removal of protecting group was performed using 3N KOH aqueous solution and reacting at room temperature until TLC showed the substantial completion of the reaction). The target product was separated with silica gel column chromatograph to obtain a brown solid (yield:3%).
MS-ESI (M/Z): 358.5[M+H]+
1H-NMR (400 MHz, CD30D, δ ppm): 8.98 (1H,s), 8.49 (1H,d, J=8), 8.25 (1H, d, J=8), 7.73-7.76 (1H, m), (1H, s), 5.09 (1H, s), 4.30(1H, s), 4.26 (1H, s), 4.14 (1H, s), 3.79 (1H, s), 3.59-3.62 (2H, m), 2.94-3.05 (3H, m), 2.37-2.56 (4H, m),1.59-2.15 (12H, m).

### Example 48: Synthesis of N-(4-pyridyl)benzyl matrinic acid (DM-145):

According to the procedure of step 3 of Example 5, the obtained corresponding ester of matrinic acid (for example, diphenylmethyl ester of matrinic acid) reacted with 4-pyridylbenzyl chloride hydrochloride under the above reaction conditions, and the removal of protecting group was performed using 3N KOH aqueous solution and reacting at room temperature until TLC showed the substantial completion of the reaction. The target product was separated with silica gel column chromatograph to obtain a brown solid (yield: 3.7%).
MS-ESI (M/Z): 358.5[M+H]+
1H-NMR (400 MHz, CD30D, δ ppm): 8.95 (2H,d,J=6), 8.43(2H,d, J=6), 5.23 (1H, s), 4.51(1H, s), 4.21 (1H, s), 3.84 (1H, s), 3.64 (1H, s), 3.42-3.47 (2H, m), 2.98-3.17 (3H, m), 2.42-2.70 (4H, m),1.60-2.15 (12H, m).

### Example 49: Synthesis of N-(4-thiazolyl)benzyl matrinic acid (DM-146):

According to the procedure of step 3 of Example 5, the obtained corresponding ester of matrinic acid (for example, diphenylmethyl ester of matrinic acid) reacted with 4-chloromethylthiazole hydrochloride under the above reaction conditions, the removal of protecting group was performed using 10 ml of m-cresol and reacting at room temperature until TLC showed the substantial completion of the reaction. The target product was sufficiently soaked with ethyl ether, filtered to obtain a light brown solid of 35mg. (yield:2.1%), melting point: 79.5-81.0°C.
MS-ESI (M/Z): 364.2 [M+H]⁺
¹H-NMR (500 MHz, CD₃OD, δ ppm): 8.896 (1H, s), 7.502 (1H, s), 4.094-4.122 (1H, d), 3.692-3.720 (1H, d), 2.112-2.181 (5H, m), 1.903-2.007 (5H, m), 1.551-1.672 (7H, m), 1.336-1.436 (7H, m).

### Example 50: Synthesis of N-(1-naphthyl) benzyl matrinic acid (DM-148):

According to the procedure of step 3 of Example 5, the obtained corresponding ester of matrinic acid (for example, diphenylmethyl ester of matrinic acid) reacted with 1-chloromethylnaphthalene hydrochloride under the above reaction conditions, and the removal of protecting group was performed using 10 ml of m-cresol, heating to 110°C and reacting for 8h-9h. The target product was separated with silica gel column chromatograph to obtain a light brown solid (yield: 5.8%).
MS-ESI (M/Z): 407.5[M+H]+
1H-NMR (400 MHz, CD3OD, δ ppm): 8.40 (1H, d, J=7.6), 8.01(1H, d, J=8), 7.95 (1H, d, J=8), 7.80 (1H, d, J=6.8), 7.67 (1H,t, J=7.6), 7.56 (2H,t, J=7.8),5.43 (1H, s), 4.40(1H, s), 4.06 (1H, s), 3.54 (1H, s), 3.34-3.45 (2H, m), 2.89-3.03 (2H, m), 2.65-2.70 (1H, m), 1.60-2.55 (16H, m), 1.34 (1H, d, J=14).

### Example 51: Synthesis of N-(4-methoxybenzoyl) matrinic acid (DM-151):

According to the procedure of step 3 of Example 5, the obtained corresponding ester of matrinic acid (for example, diphenylmethyl ester of matrinic acid) reacted with 4-methoxybenzoyl chloride under the above reaction conditions, and the removal of protecting group was performed using 10 ml of m-cresol, heating to 110°C and reacting for 8h-9h. The target product was separated with silica gel column chromatograph to obtain a light yellow solid of 390 mg. (yield: 21.2%), melting point: 68.2-69.1°C.
MS-ESI (M/Z): 401.2 [M+H]+
1H-NMR (400 MHz, CD30D, δ ppm): 7.37 (d, J = 8.4 Hz, 2H), 6.92 (d, J = 8.4 Hz, 2H), 4.01 (s, 1H), 3.78 (s, 3H), 3.57-3.60 (m, 1H), 3.43 (dd, J = 8, 13.6 Hz, 1H), 2.83-2.91 (m, 2H), 2.38 (s, 1H), 2.24 (t, J = 6.2 Hz, 2H), 2.11 (dd, J = 11.8, 23.8 Hz, 2H), 1.86-1.99 (m, 3H), 1.67-1.85 (m, 2H), 1.35-1.62 (m, 9H).

### Example 52: Synthesis of N-(4-cyanobenzoyl) matrinic acid (DM-152):

According to the procedure of step 3 of Example 5, the obtained corresponding ester of matrinic acid (for example, diphenylmethyl ester of matrinic acid) reacted with 4-cyanobenzoyl chloride under the above reaction conditions, and the removal of protecting group was performed using 10 ml of m-cresol, heating to 110°C and reacting for 8h-9h. The target product was separated with silica gel column chromatograph to obtain a light brown solid of 310 mg. (yield: 17.03%), melting point: 83.5°C, decomposition.
MS-ESI (M/Z): 396.3 [M+H]+
1H-NMR (400 MHz, CD30D, δ ppm): 7.764-7.784 (d, J=8 Hz, 2H), 7.552-7.572 (d, J=8 Hz, 2H), 3.289-3.351 (m, 1H), 2.775-2.857 (m, 2H), 2.191-2.245 (m, 3H), 1.954-1.988 (m, 4H), 1.378-1.726 (m, 14H).

### Example 53: Synthesis of N-(4-methylbenzoyl) matrinic acid (DM-153):

According to the procedure of step 3 of Example 5, the obtained corresponding ester of matrinic acid (for example, diphenylmethyl ester of matrinic acid) reacted with 4-methoxybenzoyl chloride under the above reaction conditions, and the removal of protecting group was performed using 10 ml of m-cresol, heating to 110°C and reacting for 8h-9h. The target product was separated with silica gel column chromatograph to obtain a light yellow solid of 460 mg. (yield: 25.9%), melting point: 72.8-74.8°C.
MS-ESI (M/Z): 385.2 [M+H]+
1H-NMR (400 MHz, CD30D, δ ppm): 7.30 (d, J = 8 Hz, 2H), 7.20 (d, J = 8 Hz, 2H), 4.01 (s, 1H), 3.37-3.42 (m, 1H), 2.87 (dd, J = 11.6, 20.4 Hz, 2H), 2.36 (s, 1H), 2.33 (s, 3H), 2.22-2.77 (m, 2H), 2.10 (dd, J = 11.8, 24.2 Hz, 2H), 1.70-1.91 (m, 5H), 1.36-1.63 (m, 10H).

### Example 54: Synthesis of N-(4-fluorobenzoyl) matrinic acid (DM-154):

According to the procedure of step 3 of Example 5, the obtained corresponding ester of matrinic acid (for example, diphenylmethyl ester of matrinic acid) reacted with 4-fluorobenzoyl chloride under the above reaction conditions, and the removal of protecting group was performed using 10 ml of m-cresol, heating to 110°C and reacting for 8h-9h. The target product was separated with silica gel column chromatograph to obtain a yellow brown solid of 170mg. (yield: 9.5%), melting point: 67.9-69.3°C.
MS-ESI (M/Z): 389.1 [M+H]+
1H-NMR (400 MHz, CD30D, δ ppm): 7.434-7.469 (dd, J=5.2, 8.8 Hz, 2H), 7.105-7.149 (t, J=8.8 Hz, 2H), 4.027 (s, 1H), 3.520-3.573 (m, 1H), 3.357-3.412 (m, 1H), 2.806-2.889 (m, 2H), 2.245-2.305 (m, 3H), 2.014-2.102 (m, 2H), 1.361-1.914 (m, 14H).

### Example 55: Synthesis of N-(4-trifluoromethylbenzoyl) matrinic acid DM-155):

According to the procedure of step 3 of Example 5, the obtained corresponding ester of matrinic acid (for example, diphenylmethyl ester of matrinic acid) reacted with 4-trifluoromethylbenzoyl chloride under the above reaction conditions, and the removal of protecting group was performed using 10 ml of m-cresol, heating to 110°C and reacting for 8h-9h. The target product was separated with silica gel column chromatograph to obtain a yellow brown solid of 120mg. (yield: 6%), melting point: 93.2-95.0°C.
MS-ESI (M/Z): 439.3 [M+H]+
1H-NMR (400 MHz, CD30D, δ ppm): 7.700-7.720 (d, J=8 Hz, 2H), 7.569-7.589 (d, J=8 Hz, 2H), 3.358-3.377 (m, 1H), 2.780-2.860 (m, 2H), 2.190-2.255 (m, 3H), 1.935-2.014 (m, 4H), 1.375-1.733 (m, 14H).

### Example 56: Synthesis of N-(4-trifluoromethoxybenzyl) matrinic acid (DM-162):

According to the procedure of step 3 of Example 5, the obtained corresponding ester of matrinic acid (for example, diphenylmethyl ester of matrinic acid) reacted with 4-trifluoromethoxybenzyl chloride under the above reaction conditions, and the removal of protecting group was performed using 10 ml of m-cresol, heating to 110°C and reacting for 8h-9h. The target product was separated with silica gel column chromatograph to obtain a light yellow solid of 210 mg. (yield: 10.3%), melting point: 80.5°C, decomposition.
MS-ESI (M/Z): 441.5 [M+H]+
1H-NMR (CD30D, δ ppm): 7.484-7.505 (2H, d, J=8.8 Hz), 7.225-7.246 (2H, d, J=8.4 Hz), 4.259-4.292 (1H, d, J=13.2 Hz), 3.612-3.646 (1H, d, J=13.2 Hz), 3.036-3.118 (3H, m), 2.777-2.854 (2H, m), 2.499-2.594 (3H, m), 2.120-2.306 (3H, m), 1.965-2.039 (2H, m), 1.809-1.842 (1H, m), 1.522-1.734 (9H, m), 1.423-1.452 (1H, m).

### Example 57: Synthesis of sophocarpinic acid (SC-1):

12.3g(0.05 mol)of sophocarpine was provided, added to an aqueous solution of 33.6g (0.6 mol) of potassium hydroxide (KOH) in 300 ml water, heated and refluxed for 9h, then reacted at room temperature overnight. The reaction solution was cooled with ice-water bath, adjusted with 3N hydrochloric acid to PH6-7, concentrated in reduced pressure to dryness. The obtained solid was dissolved sufficiently in ethanol, filtered, the filter cake was washed with ethanol, the filtrates were combined and evaporated to obtain a crude product of sophocarpinic acid(SC-1), this light yellow solid was recrystallized with ethanol/acetone twice to obtain a pure product of sophocarpinic acid (SC-1, also called as "Huai Guo Suan"), total 4.9g(yield: 37.1%).
MS-ESI (M/Z): 265.4 [M+H]⁺
¹H-NMR (CD₃OD, δ ppm): 6.50-6.58(1H, m), 6.01-6.05(1H, d, J=15.6 Hz), 3.51-3.55(1H, m), 3.01-3.34(6H, m), 2.76-2.80(1H, m), 2.44-2.58 (3H, m), 2.01-2.28(3H, m), 1.58-1.80(8H, m).

### Synthesis of N-substituted sophocarpinic acid:

### Example 58: Synthesis of N-benzoyl sophocarpinic acid (SC-15):

### Step 1. Preparation of sophocarpinic acid (SC-1):

12.3g (0.05 mol) of sophocarpine was provided, added to an aqueous solution of 33.6g (0.6 mol) of potassium hydroxide (KOH) in 300 ml water, heated and refluxed for 9h, then reacted at room temperature overnight. The reaction solution was cooled with ice-water bath and adjusted with 3N hydrochloric acid to PH 6-7, concentrated in reduced pressure to dryness. The obtained solid was added to methanol and sufficiently dissolved, filtered, the filter cake was washed with methanol, and the filtrates were combined to obtain a solution of SC-1 in methanol and directly used in the next step of reaction:

### Step 2. Preparation of diphenyldiazomethane:

9.8g(0.05mol) of benzophenone hydrazine was provided, added to 120ml of petroleum ether (30-60°C), added with 13.05g(0.15mol) of electrolytic manganese dioxide and refluxed for 1h, suction filtered, the filter cake was washed with petroleum ether, the filtrates were combined and directly used in the next step of reaction.

### Step 3. Preparation of diphenylmethyl ester of sophocarpinic acid:

A solution of diphenyldiazomethane in petroleum ether was directly added to the solution of SC-1 in methanol, stirred at room temperature, until purple faded completely, about 12 h, concentrated to dryness, and dichloromethane and water were added for layer seperationg. The dichloromethane layer was dried over anhydrous Na₂SO₄, to obtain a solution of diphenylmethyl ester of sophocarpinic acid in dichloromethane, which solution was directly used in the following reaction for synthesis of N-substituted sophocarpinic acid.

### Step 4.Synthesis of N-benzoyl sophocarpinic acid (SC-15):

To the solution of diphenylmethyl ester of sophocarpinic acid in dichloromethane obtained in step 3, was added 3.45g(0.025mol) of K₂CO₃, added dropwise with 2.15mL(18.5mmol) of benzoyl chloride, stirred at room temperature, until TLC detection showed the disappearance of raw material spot, filtered to remove inorganic salt, concentrated, subjected to flash column chromatography to obtain 1g of white solid of A, and 1 g of white solid of B. A and B were separately added to 15mL of m-cresol, reacted at 80-90°C for 8 h, 50mL of methyl butyl ketone was added, extracted with water for 3 times, the water layers were combined, concentrated to obtain a white solid of A: 0.26g, melting point: 152-154°C; B: 0.27g, melting point: 108-110°C.
SC-15A:
MS-ESI (M/Z): 369.0 [M+H]+
1H-NMR (D20, δ ppm): 7.65-7.76(2H, m), 7.57-7.63(3H, m), 7.30(1H, t, J=7.6 Hz), 6.28(1H, d, J=15.6Hz), 4.52(1H, s), 4.10(1H, s), 3.68-3.70(1H, m), 3.50-3.52(1H, m), 3.39-3.41(2H, m), 2.94-2.97(1H, m), 2.75(1H, s), 2.51(1H, s), 2.33-2.41(2H,m), 1.69-2.31(9H, m).
SC-15B:
MS-ESI (M/Z): 369.0 [M+H]+
1H-NMR (D20, δ ppm): 7.50(2H, d, J=6.8Hz), 7.36-7.47(3H, m), 6.04-6.16(2H, m), 4.53(1H, s), 3.82(1H, s), 3.49-3.59(2H, m), 3.31(1H, d, J=10.8Hz), 3.17(1H, d, J=15.6Hz), 2.95-3.01(1H, m), 2.72(1H, s), 2.29(1H, d, J=9.2Hz), 2.14(2H, s), 1.95(2H, d, J=12.8Hz), 1.82(2H, s), 1.35-1.59(5H, m).

### Example 59: Synthesis of N-benzyl sophocarpinic acid (SC-17):

To the solution of diphenylmethyl ester of sophocarpinic acid in dichloromethane obtained in step 3 of example 58 was added 3.45g(0.025mol) of K₂CO₃, added dropwise with 2.2mL(18.5mmol) of benzyl bromide, stirred at room temperature, until TLC detection showed the disappearance of raw material spot, filtered to remove inorganic salt, concentrated, subjected to flash column chromatography to obtain 1g of white solid of A, 1.1g of white solid of B, which were separately added with 15ml of m-cresol, 80-90°C, reacted for 8 h, added with methyl butyl ketone 50mL, extracted with water for 3 times, the water layers were combined, concentrated to obtain a white solid of A: 0.51g, melting point:.B: 0.53g, melting point: 103-105°C.
SC-17A:
MS-ESI (M/Z): 355.4[M+H]+: 1H-NMR (D2O, δ ppm): 7.43-7.62 (5H, m), 6.09-6.13 (1H, m), 5.40-5.46 (1H, m), 4.40 (1H, s), 2.95-3.48 (4H, m), 1.93-2.31 (10H,m), 1.56-1.73 (8H, m).
SC-17B:
MS-ESI (M/Z): 355.4[M+H]+
1H-NMR (D20, δ ppm): 7.36-7.49 (5H, m), 6.03-6.07(1H, m), 5.37 (1H, dd, J=9.2, 14.8 Hz), 4.24-4.39 (1H, m), 3.00-3.50 (5H, m), 2.74-2.97(5H, m), 1.94-2.13 (3H, m), 1.50-1.97 (8H, m).

### Example 60: Synthesis of N-acetyloxy sophocarpinic acid (SC-19):

To the solution of diphenylmethyl ester of sophocarpinic acid in dichloromethane obtained in step 3 of example 58 was added 3.45g(0.025mol) of K₂CO₃, added dropwise with 1.76mL(18.5mmol) of ethyl chloroformate, stirred at room temperature, until TLC detection showed the disappearance of raw material spot, filtered to remove inorganic salt, concentrated, subjected to flash column chromatography to obtain 1g of white solid, added with 15 ml of m-cresol, reacted at 80-90°C for 8 h, added with methyl butyl ketone 50mL, extracted with water for 3 times, the water layers were combined, concentrated to obtain a white solid: 0.30g.
MS-ESI (M/Z): 337.5[M+H]⁺: ¹H-NMR (D₂O, δ ppm):6.54-6.62(1H, m), 5.88(1H, d, J=15.2Hz), 4.11-4.62(2H, m), 3.30-3.64(5H, m), 2.91-3.00(3H, m), 2.39-2.55(2H, m), 2.20(1H, s), 2.09(1H, s), 1.77-1.85(8H, m), 1.28(3H, t, J=6.8 Hz).

### Example 61: Synthesis of N-propyl sophocarpinic acid (SC-21):

To the solution of diphenylmethyl ester of sophocarpinic acid in dichloromethane obtained in step 3 of example 58 was added 3.45g(0.025mol) of K₂CO₃, added dropwise with 1.8mL(18.5mmol) of propyl iodide, stirred at room temperature, until TLC detection showed the disappearance of raw material spot, filtered to remove inorganic salt, concentrated, subjected to flash column chromatography to obtain 1g of white solid of A, 1g of white solid of B, separately added with 15 ml of m-cresol, reacted at 80-90°C for 8 h, added with methyl butyl ketone 50mL, extracted with water for 3 times, the water layers were combined, concenrated to obtain white solid of A: 0.26g, B: 0.18g, melting point: 206-208°C. A and B were two geometric isomers.
SC-21A:
MS-ESI (M/Z): 307.5[M+H]⁺: ¹H-NMR (D₂O, δ ppm): 6.08-6.15(1H, m), 5.29-5.36(1H, m), 3.67(1H,s), 2.83-3.23(9H, m), 2.18-2.36(3H, m), 1.57-2.00(11H,m), 0.89-0.99(3H,m).
SC-21B:
MS-ESI (M/Z): 307.5[M+H]⁺: ¹H-NMR (D₂O, δ ppm): 6.08-6.14(1H, m), 5.30-5.36(1H, m), 3.68(1H, s), 2.84-3.24(9H, m), 2.19-2.36(3H, m), 1.57-2.00(11H, m), 0.89-0.99(3H, m).

### Example 62: Synthesis of N-(2-fluoro-4-bromobenzyl) sophocarpinic acid (SC-22)

To the solution of diphenylmethyl ester of sophocarpinic acid in dichloromethane obtained in step 3 of example 58 was added 3.45g(0.025mol) of K₂CO₃, added dropwise with 4.96g(18.5mmol) of 2-fluoro-4-bromobenzyl bromide, stirred at room temperature, until TLC detection showed the disappearance of raw material spot, filtered to remove inorganic salt, concentrated, subjected to flash column chromatography to obtain 1.1g of white solid, added with 15 ml of m-cresol, reacted at 80-90°C for 8 h, added with methyl butyl ketone 50mL, extracted with water for 3 times, the water layers were combined, concenrated to obtain a white solid of 0.25g, melting point: 103-105°C.
MS-ESI (M/Z): 451.3 M+543.4[M+2]+: 1H-NMR (D20, δ ppm): 7.40-7.46 (2H, m), 7.29-7.35 (1H,m), 6.69-6.75 (1H, m), 6.05 (1H, d, J=15.6Hz), 4.19-4.27 (1H, m),3.48-3.51 (1H, d, J=12.8 Hz), 3.25-3.32 (3H, m), 3.07-3.09 (1H, d, J=7.2Hz), 2.86-2.90 (3H, m), 2.53-2.78 (3H, m), 1.93-2.19(3H, m), 1.58-1.74(8H,m).

### Example 63: Synthesis of N-pivaloyl sophocarpinic acid SC-23)

To the solution of diphenylmethyl ester of sophocarpinic acid in dichloromethane obtained in step 3 of example 58 was added 3.45g(0.025mol) of K₂CO₃, added dropwise with 2.29mL (18.5mmol) of pivalyl chloride, stirred at room temperature, until TLC detection showed the disappearance of raw material spot, filtered to remove inorganic salt, concentrated, subjected to flash column chromatography to obtain 0.8g of white solid, added with 15 ml of m-cresol, reacted at 80-90°C for 8 h, added with methyl butyl ketone 50mL, extracted with water for 3 times, the water layers were combined, concenrated to obtain a white solid of 0.25g.
MS-ESI (M/Z): 349.5[M+H]⁺: ¹H-NMR (D₂O, δ ppm):5.75-5.96(2H, m), 4.01-4.05 (1H, m), 3.55-3.67 (2H, m), 3.37-3.40(2H, m), 2.95-2.02(3H, m), 2.55-2.64(1H, m), 2.25-2.38(2H, m), 1.70-2.02(9H, m), 1.21-1.29(9H, m).

### Example 64: Synthesis ofN-pentyl sophocarpinic acid (SC-32):

To the solution of diphenylmethyl ester of sophocarpinic acid in dichloromethane obtained in step 3 of example 58 was added 3.45g(0.025mol) of K₂CO₃, added dropwise with 2.4mL(18.5mmol) pentyl iodide, stirred at room temperature, until TLC detection showed the disappearance of raw material spot, filtered to remove inorganic salt, concentrated, subjected to flash column chromatography to obtain 1g of white solid of A, 1g of white solid of B, which were separately added with 15ml of m-cresol, 80-90°C, reacted for 8 h, added with methyl butyl ketone 50mL, extracted with water for 3 times, the water layers were combined, concentrated to obtain white solid of A: 0.18g,melting point:.B: 0.26g, melting point: 66-67°C.A and B were two geometric isomers.
SC-32A:
MS-ESI (M/Z): 335.5[M+H]⁺: ¹H-NMR (D₂O, δ ppm): 6.11-6.16(1H, m), 5.31-5.37(1H, m), 3.72(1H, s), 2.84-3.30(9H, m), 2.23-2.44(3H, m), 2.03-2.06(1H, d, J=11.6 Hz), 1.60-1.93(11H, m), 1.28-1.33(4H, m), 0.88-0.93(3H, m).
SC-32B:
MS-ESI (M/Z): 335.5[M+H]⁺: ¹H-NMR (D₂O, δ ppm): 6.10-6.17(1H, m), 5.30-5.36(1H, m), 3.71(1H, s), 2.71-3.25(9H, m), 2.20-2.36(3H, m), 2.02-2.00(1H, d, J=11.2 Hz), 1.58-1.93(11H, m), 1.20-1.37(4H, m), 0.88-0.93(3H, m).

### Example 65: Synthesis of N-(4-cyanobenzyl) sophocarpinic acid (SC-36):

To the solution of diphenylmethyl ester of sophocarpinic acid in dichloromethane obtained in step 3 of example 58 was added 3.45g(0.025mol) of K₂CO₃, added dropwise with 3.63g(18.5mmol) of 4-cyanobenzyl bromide, stirred at room temperature, until TLC detection showed the disappearance of raw material spot, filtered to remove inorganic salt, concentrated, subjected to flash column chromatography to obtain 1.1.g of white solid of A, 1g of white solid of B, which were separately added with 15ml of m-cresol, 80-90°C, reacted for 8 h, added with methyl butyl ketone 50mL, extracted with water for 3 times, the water layers were combined, concenrated to obtain a white solid of A: 0.23g, melting point: 150-152°C, B: 0.17g, melting point: 128-130°C. A and B were two geometric isomers.
SC-36A:
MS-ESI (M/Z): 380.4[M+H]+: 1H-NMR (D20, δ ppm): 7.92(2H, d, J=8 Hz), 7.69(2H, d, J=8 Hz), 6.35-6.42(1H, m), 5.66(1H, dd, J=9.2, 15.6 Hz), 4.91-4.94(1H, d, J=13.6 Hz), 4.20-4.23(1H, d, J=13.6 Hz), 4.01-4.06(1H, m), 3.75(1H, s), 3.46-3.53(2H, m), 3.23-3.37(4H, m), 3.08-3.18(2H, m), 2.49-2.52(2H, d, J=10.8 Hz), 2.25(1H, s), 1.67-2.06(8H, m).
SC-36B:
MS-ESI (M/Z): 380.6[M+H]⁺: ¹H-NMR (D₂O, δ ppm): 7.90-7.94(2H, m), 7.69(2H, d, J=8 Hz), 6.93-7.00(1H, m), 6.27-6.31(1H, d, J=15.6Hz), 4.92 (1H, d, J=13.2 Hz), 4.36(1H, d, J=13.2 Hz), 4.03-4.06(1H, m), 3.85(1H, s), 3.46-3.54(2H, m), 3.04-3.36(6H, m), 2.49-2.63(2H, m), 2.23(1H, s), 1.63-2.06(8H, m).

### Example 66: Synthesis of N-4-nitrobenzyl sophocarpinic acid (SC-61A):

To the solution of diphenylmethyl ester of sophocarpinic acid in dichloromethane obtained in step 3 of example 58 was added 3.45g(0.025mol) of K₂CO₃, added dropwise with 4-nitrobenzyl bromide4g(18.5mmol), stirred at room temperature, until TLC detection showed the disappearance of raw material spot, filtered to remove inorganic salt, concentrated, subjected to flash column chromatography to obtain 1.5g of white solid, added with 15ml of m-cresol, reacted at 80-90°C for 8 h, added with methyl butyl ketone 50mL, extracted with water for 3 times, the water layers were combined, concenrated to obtain a white solid of 0.4g, melting point: 139-141°C.
MS-ESI (M/Z): 400.2[M+H]+
1H-NMR (CD3OD,δppm): 8.12-8.15(2H, m), 7.51-7.57(2H, m), 6.97-7.04(1H, m), 5.85(1H, d, J=15.6 Hz), 4.23(1H, d, J=14.4 Hz), 3.27-3.47(4H, m), 2.90-3.01(3H, m), 2.79-2.85(1H, m), 2.47-2.78(3H, m), 2.19-2.22(1H, m), 1.93-2.08(2H, m), 1.65-1.88(7H, m).

### Example 67: Synthesis of N-2-methylbenzyl sophocarpinic acid (SC-62A):

To the solution of diphenylmethyl ester of sophocarpinic acid in dichloromethane obtained in step 3 of example 58 was added 3.45g(0.025mo1) of K₂CO₃, added dropwise with 2.47mL(18.5mmol) of 2-methylbenzyl bromide, stirred at room temperature, until TLC detection showed the disappearance of raw material spot, filtered to remove inorganic salt, concentrated, subjected to flash column chromatography to obtain 1.1g of white solid, added with 15ml of m-cresol, reacted at 80-90°C for 8 h, added with methyl butyl ketone 50mL, extracted with water for 3 times, the water layers were combined, concenrated to obtain a white solid of 0.3g, melting point: 98-100°C. MS-ESI (M/Z): 369.2[M+H]+
1H-NMR (CD3OD,δppm):7.13-7.31 (4H, m), 5.95-6.06 (1H, m), 5.30 (1H, dd, J=9.2, 15.2 Hz),4.46 (1H, m),3.03-3.10(3H, m), 2.99(2H, d, J=7.2 Hz), 2.82-2.93(2H, m), 2.42-2.61 (3H, m), 2.31-2.33 (3H, m), 2.19 (1H, s), 1.87-2.03(3H, m), 1.41-1.79 (7H, m).

### Example 68: Synthesis of N-4-bromobenzyl sophocarpinic acid (SC-64A):

To the solution of diphenylmethyl ester of sophocarpinic acid in dichloromethane obtained in step 3 of example 58 was added 3.45g(0.025mol) of K₂CO₃, added dropwise with 4.63g(18.5mmol)4-bromobenzyl bromide, stirred at room temperature, until TLC detection showed the disappearance of raw material spot, filtered to remove inorganic salt, concentrated, subjected to flash column chromatography to obtain 1.45g of white solid, added with 15ml of m-cresol, reacted at 80-90°C for 8 h, added with methyl butyl ketone 50mL, extracted with water for 3 times, the water layers were combined, concenrated to obtain a white solid of 0.51 g, melting point: 98-100°C.
MS-ESI (M/Z): 433.4M+
¹H-NMR (CD₃OD,δppm):7.42-7.47 (2H, m), 7.24-7.26 (2H, d, J=8 Hz), 5.94-6.01(1H, m), 5.25(1H, dd, J=9.2, 15.2Hz,), 4.13-4.29(1H, m), 2.89-3.20(5H, m), 2.42-2.63(4H, m), 1.71-2.48(5H, m), 1.41-1.68(7H, m).

### Example 69: Synthesis of N-4-trifluoromethylbenzyl sophocarpinic acid (SC-69A):

To the solution of diphenylmethyl ester of sophocarpinic acid in dichloromethane obtained in step 3 of example 58 was added 3.45g(0.025mol) of K₂CO₃, added dropwise with 4.42g(18.5mmol)4-trifluoromethylbenzyl bromide, stirred at room temperature, until TLC detection showed the disappearance of raw material spot, filtered to remove inorganic salt, concentrated, subjected to flash column chromatography to obtain 1.1g of white solid, added with 15ml of m-cresol, reacted at 80-90°C for 8 h, added with methyl butyl ketone, extracted with water for 3 times, the water layers were combined, concenrated to obtain a white solid of 0.51g, melting point: 102-103°C.
MS-ESI (M/Z): 439.2[M+H]+
1H-NMR (CD3OD,δppm): 7.37(2H, d, J=8 Hz), 7.25(2H, d, J=8 Hz), 5.64-5.71(1H, m), 5.22(1H, dd, J=8.8, 15.6 Hz), 4.09-4.15(1H, m), 3.35-3.44(2H, m), 3.11-3.17(1H, m), 2.97-3.01(2H, m), 2.64-2.73(3H, m), 2.16-2.23(1H, m), 2.04(1H, s), 1.21-1.99(11H, m).

### Example 70: Synthesis of N-p-trifluoromethylbenzene sulfonyl sophocarpinic acid (SC-84A):

To 1/2 portion of the solution of diphenylmethyl ester of sophocarpinic acid in dichloromethane obtained in the step 3 of Example 58 was added 3.45g(0.025mol) of K₂CO₃, added dropwise with 3.05g (12.5mmol) of trifluoromethylbenzenesulfonyl chloride, stirred at room temperature, until TLC detection showed the disappearance of raw material spot, filtered to remove inorganic salt, concentrated, subjected to flash column chromatography to obtain 3.3g of white solid, added with 15 ml of m-cresol, reacted at 80-90°C for 8 h, added with methyl butyl ketone 50mL, extracted with water for 3 times, the water layers were combined, concenrated to obtain a white solid of 1.5g. Flash column chromatography was performed to obtain 0.5g of SC-84A.
HRMS-ESI (M/Z): C₂₂H₂₈N₂O₄F₃S 473.1715(M+1);
474.1768 (M+2);1H-NMR (CD3OD, δ ppm): 7.95(2H, d, J=8.4Hz), 7.85(2H, d, J=8.4Hz), 5.47-5.49(2H, m), 3.79(1H, dd, J=4.4, 12Hz), 3.40-3.50(2H, m), 3.05(1H, t, J=12Hz), 2.94(3H, s), 2.70(1H, s), 2.20-2.39(2H, m), 2.08-2.11(1H, m), 1.81-1.86(2H, m), 1.61-1.70(3H, m), 1.33-1.54(4H, m).

### Example 71: Synthesis of N-m-cyanobenzenesulfonyl sophocarpinic acid (SC-89B:)

To 1/2 portion of the solution of diphenylmethyl ester of sophocarpinic acid in dichloromethane obtained in the step 3 of Example 58 was added 3.45g(0.025mol) of K₂CO₃, added dropwise with m-cyanobenzenesulfonyl chloride 2.52g (12.5mmol), stirred at room temperature, until TLC detection showed the disappearance of raw material spot, filtered to remove inorganic salt, concentrated, subjected to flash column chromatography to obtain 3.8g of white solid, added with 15 ml of m-cresol, reacted at 80-90°C for 8 h, added with methyl butyl ketone 50mL, extracted with water for 3 times, the water layers were combined, concentrated to obtain a white solid,subjected to flash column chromatography,SC-89B 0.8g.
HRMS-ESI (M/Z): C22H28N3O4S 430.1782(M+1);
1H-NMR (CDC13, δ ppm): 8.01-8.07(2H, m), 7.78(1H, d, J=8Hz), 7.61(1H, J=8Hz), 5.40-5.56(2H, m), 4.11-4.16(1H, m), 3.50-3.91(2H, m), 3.42-3.49(1H, m), 3.18-3.42(1H, m), 2.58-2.91(3H, m), 2.19-2.39(4H, m), 1.42-2.06(8H, m).

### Example 72: Synthesis of 13-hydroxyl matrine (SC-27-C)

12.3g(0.05 mol) of sophocarpine was provided, added to an aqueous solution of 33.6g (0.6 mol) of potassium hydroxide (KOH) in 300 ml water, heated and refluxed for 9h, then reacted at room temperature overnight. The reaction solution was cooled with ice-water bath and adjusted with 3N hydrochloric acid to PH7-7.5, concentrated in reduced pressure to dryness. Methanol was used for soaking to remove inorganic salt, the methanol was concentrated to dryness, and the column chromatography (using dichloromethane and methanol as eluting solution) to obtain SC-27-C 6.1g (46.2 %).
1H NMR (CD3OD, 400 Hz): δ 4.51 (dd, J = 4.4, 13.8 Hz, 1H), 4.14-4.18 (m, 1H), 3.86-3.92 (m, 1H), 3.47 (s, 1H), 3.36 (m, 2H), 3.24-3.26 (m, 1H), 2.89-3.04 (m, 3H), 2.52 (dd, J = 3.6, 17.6 Hz, 1H), 2.37 (dt, J = 17.6, 3.2 Hz, 1H), 2.17-2.24 (m, 1H), 1.95-2.04 (m, 2H), 1.81-1.94 (m, 3H), 1.69-1.79 (m, 4H), 1.59-1.68 (m, 1H);
HRMS: calcd for C15H25N2O2 (M+H)+: 265.1916; found: 265.1921.

### Example 73: Synthesis of N-benzyl-13-hydroxyl matrinic acid (SC-17C):

### Step 1. Synthesis of 13-hydroxyl, matrinic acid

SC-27-C(6.6g, 25mmol) was added to an aqueous solution of 16.8g(0.3 mol) of potassium hydroxide (KOH) in 150 ml water, heated and refluxed for 9h, then reacted at room temperature overnight. The reaction solution was cooled with ice-water bath and adjusted with 3N hydrochloric acid to PH7-7.5, concentrated in reduced pressure to dryness. Methanol was used for soaking to remove inorganic salt, the methanol solution was concentrated to reach half of the original volume, which was used for the next step of reaction.

### Step 2. Preparation of diphenyldiazomethane:

4.9g(0.025mol) of benzophenone hydrazine was provided, added to 60mL petroleum ether (30-60°C), added with 6.5g(74.8mmol) of electrolytic manganese dioxide and refluxed for 1h, suction filtered, the filter cake was washed with petroleum ether, the filtrates were combined and directly used in the next step of reaction.

### Step 3.Preparation of diphenylmethyl ester of 13-hydroxyl matrinic acid :

The solution of diphenyldiazomethane in petroleum ether was directly added to a solution of 13-hydroxyl matrinic acid in methanol, stirred at room temperature, until purple faded completely, about 12 h, concentrated to dryness, added with dichloromethane, and directly used in the next step of reaction.

The above solution of diphenylmethyl ester of 13-hydroxyl matrinic acid in dichloromethane was added to 1.725g(12.5mmol) of K₂CO₃, added dropwise with 1.1mL(9.25mmol) of benzyl bromide, stirred at room temperature, until TLC detection showed the disappearance of raw material spot, filtered to remove inorganic salt, concentrated, subjected to flash column chromatography to obtain 0.9g of white solid, added with 15ml of m-cresol, reacted at 80-90°C for 8 h, added with methyl butyl ketone 50mL, extracted with water for 3 times, the water layers were combined, concentrated to obtain a white solid of 0.3 1 g, melting point: 115-117°C.
MS-ESI (M/Z): 373.4[M+H]+
1H-NMR (CD30D, δ ppm): 7.26-7.44(5H, m), 4.38-4.47(1H, m), 4.08-4.13(1H, m), 3.72-3.74(1H, m), 3.15-3.25(2H, m), 2.76-3.18(3H, m), 2.59-2.70(1H, m), 2.32-2.49(3H, m), 1.85-2.25(6H, m), 1.39-1.72(7H, m).

### Example 74: Synthesis of N-4-cyanobenzyl-13-hydroxyl matrinic acid (SC-36C)

To the solution of diphenylmethyl ester of 13-hydroxyl matrinic acid in dichloromethane obtained in the step 3 of Example 73 was added 1.725g(12.5mmol) of K₂CO₃, added dropwise with 1.81g(9.25mmol) of 4-cyanobenzyl bromide, stirred at room temperature, until TLC detection showed the disappearance of raw material spot, filtered to remove inorganic salt, concentrated, subjected to flash column chromatography to obtain 1.0g of white solid, added with 15ml of m-cresol, reacted at 80-90°C for 8 h, added with methyl butyl ketone 50mL, extracted with water for 3 times, the water layers were combined, concentrated to obtain a white solid of 0.53g, melting point: 119-121°C.
MS-ESI (M/Z): 398.3[M+H]+
1H-NMR (CD30D, δ ppm):7.65(2H, d, J=8 Hz), 7.54(2H, d, J=8 Hz), 4.20(1H, d, J=14.4 Hz), 4.00-4.03(1H, m), 3.64(1H, d, J=14.4 Hz), 3.05-3.25(2H, m), 2.97(1H, s), 2.66-2.82(1H, m), 2.56-2.66(1H, m), 2.29-2.50(4H, m), 2.19(1H, s), 1.90-2.10(4H, m), 1.43-1.82(8H, m).

### Example 75: Synthesis of N-2-methylbenzyl-13-hydroxyl matrinic acid (SC-62B):

To the solution of diphenylmethyl ester of 13-hydroxyl matrinic acid in dichloromethane obtained in the step 3 of Example 73 was added 1.725g(12.5mmol) of K₂CO₃, added dropwise with 1.8mL(13.75mmol) of 2-methylbenzyl bromide, stirred at room temperature, until TLC detection showed the disappearance of raw material spot, filtered to remove inorganic salt, concentrated, subjected to flash column chromatography to obtain 1.0g of white solid, added with 15ml of m-cresol, reacted at 80-90°C for 8 h, added with methyl butyl ketone 50mL, extracted with water for 3 times, the water layers were combined, concentrated to obtain a white solid of 0.5g, melting point: 105-107°C.
MS-ESI (M/Z): 387.4 [M+H]+
1H-NMR (D2O,δppm):7.26-7.37(4H, m), 4.28(1H, s), 4.04-4.06(1H, m), 3.54(1H, dd, J=6.8, 14.4Hz), 3.08-3.32(4H, m), 2.53-2.71(3H, m), 2.37-2.51(m, 6H),2.26(1H, s), 1.93-2.26(5H, m), 1.40-1.65(6H, m).

### Example 76: Synthesis of N-4-bromobenzyl-13-hydroxyl matrinic acid (SC-64B):

To the solution of diphenylmethyl ester of 13-hydroxyl matrinic acid in dichloromethane obtained in the step 3 of Example 73 was added 1.725g(12.5mmol) of K₂CO₃, added dropwise with 3.44g(13.75mmol) of 4-bromobenzyl bromide, stirred at room temperature, until TLC detection showed the disappearance of raw material spot, filtered to remove inorganic salt, concentrated, subjected to flash column chromatography to obtain 0.8g of white solid, added with 15ml of m-cresol, reacted at 80-90°C for 8 h, added with methyl butyl ketone 50mL, extracted with water for 3 times, the water layers were combined, concentrated to obtain a white solid of 0.32g, melting point: 106-108°C.
MS-ESI (M/Z): 451.3 [M]+, 453.3[M+2]+
1H-NMR(CD3OD,δppm):7.32-7.36(2H, m), 7.49-7.53(2H, m), 4.22-4.25(1H, m), 4.03-4.05(1H, m), 3.46(1H, m), 2.90-3.07(4H, m), 2.58-2.66(1H, m), 2.31-2.50(3H, m), 2.16-2.27(2H, m), 1.74-2.19(5H, m), 1.41-1.70(7H, m).

### Example 77: Synthesis of N-3, 5-dimethylbenzyl-13-hydroxyl matrinic acid (SC-65B):

To the solution of diphenylmethyl ester of 13-hydroxyl matrinic acid in dichloromethane obtained in the step 3 of Example 73 was added 1.725g(12.5mmol) of K₂CO₃, added dropwise with 2.74g(13.75mmol) of 3,5-dimethylbenzyl bromide, stirred at room temperature, until TLC detection showed the disappearance of raw material spot, filtered to remove inorganic salt, concentrated, subjected to flash column chromatography to obtain 0.6g of white solid, added with 10ml of m-cresol, 80-90°C, reacted for 8 h, added with methyl butyl ketone 50mL, extracted with water for 3 times, the water layers were combined, concentrated to obtain a white solid of 0.22g, melting point: 135-137°C.
MS-ESI (M/Z): 401.6[M+1]+
1H-NMR(CD3OD,δppm):7.00-7.04(3H, m), 4.35-4.38(1H, m), 4.07-4.12(2H, m), 3.79(1H, br), 3.24-3.30(2H, m), 2.82-2.91(2H, m), 2.64(1H, dd, J=4, 12.8Hz), 2.32-2.49(3H, m), 2.27(6H, s), 2.06-2.25(4H, m), 1.87-1.99(3H, m), 1.36-1.65(7H, m).

### Example 78: Synthesis of N-4-trifluoromethylbenzyl-13-hydroxyl matrinic acid (SC-69B):

The dichloromethane solution of diphenylmethyl ester of 13-hydroxyl matrinic acid obtained in the step 3 of Example 73 was added to 1.725g(12.5mmol) of K₂CO₃, added dropwise with 3.29g(13.75mmol) of 4-trifluoromethylbenzyl bromide, stirred at room temperature, until TLC detection showed the disappearance of raw material spot, filtered to remove inorganic salt, concentrated, subjected to flash column chromatography to obtain 0.8g of white solid, added with 10ml of m-cresol, 80-90°C, reacted for 8 h, added with methyl butyl ketone 50mL, extracted with water for 3 times, the water layers were combined, concentrated to obtain a white solid of 0.4g, melting point: 114-116°C.
MS-ESI (M/Z): 457.3[M+1]+
1H-NMR(CD3OD,δppm):7.42 (2H, d, J=8.8 Hz), 7.26(2H, d, J=7.6Hz), 3.97-4.10(2H, m), 3.05-3.27(2H, m), 2.64-2.79(3H, m), 2.11-2.41(4H, m), 1.98-2.00(1H, m), 1.60-1.83(8H, m), 1.18-1.36(5H, m).

### Example 79: Synthesis of N-acetyl-(5R)-matrinic acid

Step 1. Synthesis (5R)-matrinic acid: 12.4g(0.05 mol) of sophoridine was provided, added to an aqueous solution of 33.6g(0.6mol, 12 eq.) of potassium hydroxide in 300 ml water, heated and refluxed for 9h, then reacted at room temperature overnight. The reaction solution was cooled with ice-water bath and adjusted with 3N hydrochloric acid to PH5-6, concentrated in reduced pressure to dryness. The obtained solid was added to methanol and sufficiently dissolved, filtered, the filter cake was washed with methanol, the filtrates were combined and evaporated to dryness to obtained a crude product (5R)-matrinic acid. This yellow solid was recrystallized with ethanol/acetone to obtain a pure product (5R)-matrinic acid, total 12.1 g(yield: 91.0%).
MS-ESI (M/Z): 267.1 [M+H]⁺
¹H NMR (CD₃OD, 400 Hz, ppm): δ 3.54-3.50 (m, 1H), 3.44-3.37 (m, 1H), 3.30-3.23 (m, 3H), 3.21-3.15 (m, 1H), 3.06-3.02 (m, 1H), 2.95-2.92 (m, 1H), 2.84-2.78 (m, 1H), 2.47-2.39 (m, 1H), 2.27-2.23 (m, 2H), 2.17-2.14 (m, 1H), 2.01-1.55 (m, 10H), 1.36-1.25 (m, 1H).

Step 2. The mixture of 14.7g(0.075 mol) of benzophenone hydrazone and 18.75g(0.22 mol) of manganese dioxide was added to 300 ml of petroleum ether with a boiling range of 30°C-60°C, refluxed at 50°C for 1.5 h, to obtain a dark violet solution of diphenyldiazomethane in petroleum ether. This solution was filtered to the solution (5R)-matrinic acid in methanol as obtained in the previous step, and the obtained mixture reacted at room temperature until violet disappeared. After filtration, the filtrate was concentrated to obtain an oily product, which was added to 80ml of acetone for sufficient soaking, the obtained solid was filtered, the filter cake was washed with petroleum ether, to obtain diphenylmethyl ester of (5R)-matrinic acid crude product 10.0g, which was directly used in the next step of reaction without purification (yield: 46.3%).

Step 3. Synthesis of N-acetyl-(5R)-matrinic acid: 2.0 g(0.0046 mol) diphenylmethyl ester of (5R)-matrinic acid was dissolved in 100 ml of dichloromethane, added with 1.5g of anhydrous potassium carbonate, added dropwise with a solution of 0.49 ml (0.0069 mol) acetyl chloride in 10 ml dichloromethane under cooling with ice-water bath. After dropwise addition, the reaction was performed at room temperature until TLC showed the disappearance of raw material spot. After filtration, the filter cake was washed with dichloromethane, the filtrates were combined and evaporated to dryness, the obtained oily product was dissolved in 30 ml of m-cresol, and reacted at 80°C for 4h-5h. The reaction solution was cooled to to room temperature, added with 50 ml methyl isobutyl ketone for dilution, sufficiently extracted with water. The water layers were combined and evaporated to dryness, and the obtained residue was recrystallized with ethanol/acetone to obtain a yellow solid of 0.16 g(yield: 11.0%), melting point: 127.4-129.6°C.
MS-ESI (M/Z): 309.2 [M+H]⁺
¹H NMR (CD₃OD, 400 Hz, ppm): δ 4.61-4.58 (m, 1H), 4.41 (dd, *J* = 13.6, 4.4 Hz, 1H), 3.84 (t, *J* = 7.6 Hz, 1H), 3.66 (dd, *J* = 14, 4 Hz, 1H), 3.53-3.47 (m, 2H), 3.43-3.36 (m, 2H), 3.15-3.12 (m, 2H), 2.94-2.88 (m, 3H), 2.44-2.38 (m, 1H), 2.35-2.30 (m, 1H), 2.22-1.41 (m, 10H), 1.32-1.22 (m, 2H).

### Examples 80: Synthesis of N-tert-butyryl-(5R)-matrinic acid:

According to the step 3 of Example 79, the acylating reagent was tert-butyryl chloride. The target product was recrystallized with ethanol/acetone to obtain a white solid of 0.19 g(yield: 12.1%), melting point: 52.2-54.6°C.
MS-ESI (M/Z): 351.6 [M+H]⁺
¹H NMR (CD₃OD, 400 Hz, ppm): δ 4.06-4.03 (m, 1H), 3.62-3.50 (m, 2H), 3.33-3.20 (m, 5H), 3.00-2.87 (m, 2H), 2.36-2.26 (m, 1H), 2.23-2.19 (m, 2H), 2.09-1.91 (m, 3H), 1.80-1.64 (m, 4H), 1.55-1.41 (m, 4H), 1.36-1.20 (m, 9H).

### Example 81: Synthesis of N-bromoacetyl-(5R)-matrinic acid:

According to the step 3 of Example 79, the acylating reagent was bromoacetyl bromide. The target product was recrystallized with, ethanol/acetone to obtain a light yellow solid of 0.18 g(yield: 10.0%), melting point: 127.4-129.6°C.
MS-ESI (M/Z): 387.4 [M+H]⁺
¹H NMR (DMSO-*d₆*, 400 Hz, ppm): δ 9.62 (s, 1H), 4.45-4.41 (m, 1H), 4.24-4.12 (m, 2H), 4.08-4.00 (m, 1H), 3.73-3.63 (m, 2H), 3.51-3.39 (m, 1H), 3.21 (s, 2H), 3.01 (s, 1H), 2.87-2.80 (m, 1H), 2.49-2.32 (m, 1H), 2.27-2.18 (m, 2H), 2.01-1.98 (m, 1H), 1.90-1.67 (m, 6H), 1.55-1.27 (m, 5H).

### Example 82: Synthesis of N-formylethyl ester group-(5R)-matrinic acid:

According to the step 3 of Example 79, the acylating reagent was ethyl chloroformate. The target product was recrystallized with ethanol/acetone to obtain a white solid of 0.14 g(yield: 9.0%), melting point: 48.2-50.7°C. MS-ESI (M/Z): 339.3 [M+H]⁺
¹H NMR (CD₃OD, 400 Hz, ppm): δ 4.17-3.94 (m, 4H), 3.56-3.42 (m, 2H), 3.32-3.24 (m, 1H), 3.17-3.14 (m, 1H), 2.94 (d, *J* = 12 Hz, 1H), 2.69-2.56 (m, 1H), 2.29-2.15 (m, 3H), 1.98-1.47 (m, 12H), 1.28-1.18 (m, 4H).

### Example 83: Synthesis of N-formylmethyl ester group-(5R)-matrinic acid:

According to the step 3 of Example 79, the acylating reagent was methyl chloroformate. The target product was recrystallized with ethanol/acetone to obtain a light yellow solid of 0.13 g(yield: 9.0 %), decomposition point: 170.2-172.2°C.
MS-ESI (M/Z): 325.5 [M+H]⁺
¹H NMR (CD₃OD, 400 Hz, ppm): δ 4.15-3.90 (m, 2H), 3.62 (d, *J =* 5.2 Hz, 3H), 3.47-3.38 (m, 2H), 3.20-3.13 (m, 2H), 2.89 (d, *J* = 12.0 Hz, 1H), 2.67-2.55 (m, 1H), 2.28-2.15 (m, 3H), 1.96-1.87 (m, 3H), 1.81-1.45 (m, 9H), 1.29-1.20 (m, 1H).

### Example 84: Synthesis of N-formylbenzyl ester group-(5R)-matrinic acid:

According to the step 3 of Example 79, the acylating reagent was benzyl chloroformate. The target product was recrystallized with ethanol/acetone to obtain a white solid of 0.11 g(yield: 6.0 %), decomposition point: 233.2-236.5°C.
MS-ESI (M/Z): 401.3 [M+H]⁺
¹H NMR (CD₃OD, 400 Hz, ppm): δ 7.31-7.24 (m, 5H), 4.17-4.11 (m, 1H), 4.03-3.97 (m, 1H), 3.48-3.39 (m, 2H), 3.25-3.16 (m, 2H), 2.91 (d, *J* = 12 Hz, 1H), 2.71-2.57 (m, 1H), 2.27-2.13 (m, 3H), 1.97-1.66 (m, 8H), 1.57-1.45 (m, 6H), 1.29-1.21 (m, 1H).

### Example 85: Synthesis of N-benzenesulfonyl-(5R)-matrinic acid:

According to the step 3 of Example 79, the sulfonating reagent was benzenesulfonyl chloride. The target product was recrystallized with ethanol/acetone to obtain a yellow solid of 0.30 g(yield: 16.0 %), melting point: 66.3-68.7°C.
MS-ESI (M/Z): 407.5 [M+H]⁺
¹H NMR (CD₃OD, 400 Hz, ppm): δ 7.80-7.78 (m, 2H), 7.61-7.50 (m, 3H), 3.92-3.89 (m, 1H), 3.74-3.70 (m, 1H), 3.57-3.46 (m, 2H), 3.01 (d, *J* = 12.8 Hz, 1H), 2.71-2.65 (m, 1H), 2.35-2.32 (m, 1H), 2.20-2.15 (m, 2H), 2.00-1.91 (m, 3H), 1.84-1.73 (m, 4H), 1.68-1.62 (m, 1H), 1.61-1.35 (m, 6H), 1.29-1.21 (m, 1H).

### Example 86: Syntheses of N-p-tosyl-(5R)-matrinic acid:

According to the step 3 of Example 79, the sulfonating reagent was p-toluenesulfonyl chloride.The target product was recrystallized with ethanol/acetone to obtain a yellow solid of 0.29 g(yield: 15.2 %), melting point: 64.2-66.9°C.
MS-ESI (M/Z): 421.5 [M+H]⁺
¹H NMR (CD₃OD, 400 Hz, ppm): δ 7.67-7.65 (m, 2H), 7.34-7.32 (m, 2H), 3.89 (t, *J* = 6.4 Hz, 1H), 3.68 (dd, *J* =13.6, 4.4 Hz, 1H), 3.56-3.46 (m, 2H), 3.01 (d, *J* = 12.8 Hz, 1H), 2.69-2.62 (m, 1H), 2.37-2.31 (m, 5H), 2.21-2.16 (m, 2H), 2.10-1.89 (m, 3H), 1.83-1.66 (m, 4H), 1.58-1.36 (m, 6H), 1.27-1.21 (m, 1H).

### Example 87: Synthesis of N-benzoyl (5R)-matrinic acid:

According to the step 3 of Example 79, the acylating reagent was benzoyl chloride. The target product was recrystallized with ethanol/acetone to obtain a light yellow solid of 0.17g (yield: 10.3 %), melting point: 191.2-194.0°C.
MS-ESI (M/Z): 371.2 [M+H]⁺
¹H NMR (CD₃OD, 400 Hz, ppm): δ 7.43-7.31 (m, 5H), 3.50-3.36 (m, 2H), 3.24-3.07 (m, 3H), 2.95-2.84 (m, 2H), 2.37-2.19 (m, 2H), 2.08-2.01 (m, 2H), 1. 96-1.24 (m, 12H), 1.19-1.10 (m, 1H).

### Example 88: Syntheses of N-(3-nitrobenzoyl)-(5R)-matrinic acid:

2.0 g(0.0120mol) of nitrobenzoic acid was provided, placed in a 100ml round bottom flask, added with 10 ml of SOCl₂, refluxed for 1.5h, concentrated in reduced pressure to be oily, the oily product was used as acylating agent.

2.0 g(0.0046 mol) (5R)-diphenylmethyl ester of matrinic acid was dissolved in 100 ml of dichloromethane, added with 1.5g of anhydrous potassium carbonate, added dropwise with the above oily product in this system, stirred at room temperature for 10h, until TLC showed the disappearance of raw material spot, and the generation of new compound. The new compound was separated with silica gel column chromatography, and concentrated to be oily. This oily product was added to 30 ml of m-cresol, reacted at 80°C for 4h-5h. The reaction solution was cooled to room temperature, added with 50 ml of methyl isobutyl ketone, sufficiently extracted with water. The water layers were combined and evaporated to dryness to obtain a white solid of 0.21 g(yield: 11.1 %), melting point: 74.2-75.8°C.
MS-ESI (M/Z): 416.1 [M+H]⁺
¹H NMR (CD₃OD, 400 Hz, ppm): δ 8.32-8.20 (m, 2H), 7.82-7.68 (m, 2H), 3.68-3.58 (m, 3H), 3.08-3.02 (m, 2H), 2.49-2.33 (m, 3H), 2.19-2.17 (m, 1H), 2.02-1.97 (m, 3H), 1.87-1.64 (m, 9H), 1.42 (s, 2H), 1.24-1.19 (m, 1H).

### Example 89: Synthesis of N-(p-methylbenzoyl)-(5R)-matrinic acid:

According to the step 3 of Example 79, the acylating agent was p-methylbenzoyl chloride, to obtain white solid of 0.19 g(yield: 11.0 %), decomposition point: 215.0-217.0°C.
MS-ESI (M/Z): 385.4 [M+H]⁺
¹H NMR (CD₃OD, 400 Hz, ppm): δ 7.29-7.22 (m, 4H), 3.64-3.43 (m, 3H), 3.19-3.15 (m, 2H), 2.97-2.91 (m, 2H), 2.39-2.32 (m, 4H), 2.29-2.19 (m, 1H), 2.11-2.04 (m, 1H), 1.93-1.61 (m, 11H), 1.52-1.15 (m, 3H).

### Example 90: Synthesis of N-(p-methoxybenzoyl)-(5R)-matrinic acid:

According to the step 3 of Example 79, the acylating agent was p-methoxybenzoyl chloride, to obtain white solid of 0.18 g(yield: 10.3%), melting point: 74.0-76.0°C.
MS-ESI (M/Z): 401.5 [M+H]⁺
¹H NMR (CD₃OD, 400 Hz, ppm): δ 7.38-7.28 (m, 2H), 6.97-6.94 (m, 2H), 3.78 (d, *J* = 3.6 Hz, 3H), 3.74-3.54 (m, 3H), 3.05-2.96 (m, 2H), 2.44-2.29 (m, 3H), 2.21 (t, *J* = 7.2 Hz, 1H), 2.10-1.54 (m, 13H), 1.48-1.35 (m, 1H), 1.23-1.19 (m, 1H).

### Example 91: Synthesis of N-(p-fluorobenzoyl)-(5R)-matrinic acid:

According to the step 3 of Example 79, the acylating agent was p-fluorobenzoyl chloride, to obtain light yellow solid of 0.09 g(yield: 5.1 %), melting point: 47.0-49.0°C.
MS-ESI (M/Z): 389.5 [M+H]⁺
¹H NMR (CDCl₃, 400 Hz, ppm): δ 11.70 (s, br, 1H), 7.41-7.10 (m, 4H), 3.88 (s, 1H), 3.57 (s, 2H), 3.24-3.13 (m, 3H), 2.65-2.09 (m, 6H), 1.89-1.25 (m, 12H).

### Example 92: Synthesis of N-(2-methyl-5-nitrobenzoyl) -(5R)-matrinic acid:

According to the step 3 of Example 79, the acylating agent was 2-methyl-5-nitrobenzoyl chloride, to obtain light yellow solid of 0.12 g(yield: 6.1 %), decomposition point: 232.2-234.0°C.
MS-ESI (M/Z): 430.2 [M+H]⁺
¹H NMR (DMSO-d*₆*, 400 Hz, ppm): δ 8.15 (d, *J* = 8.4 Hz, 1H), 7.96-7.82 (m, 1H), 7.61-7.54 (m, 1H), 2.95-2.62 (m, 6H), 2.40-2.20 (m, 6H), 2.03-1.34 (m, 13H), 1.22-1.11 (m, 2H).

### Example 93: Synthesis of N-(3-nitro-4-fluorobenzoyl) -(5R)-matrinic acid:

According to the step 3 of Example 79, the acylating agent was 3-nitro-4-fluorobenzoyl chloride, to obtain white solid of 0.22 g(yield: 11.3%), melting point: 168.0-170.0°C.
MS-ESI (M/Z): 434.1 [M+H]⁺
¹H NMR (CD₃OD, 400 Hz, ppm): δ 8.12-8.08 (m, 1H), 7.80-7.72 (m, 1H), 7.52-7.48 (m, 1H), 3.57-3.55 (m, 1H), 3.51-3.40 (m, 2H), 3.20-3.16 (m, 1H), 3.06-2.93 (m, 2H), 2.40-1.55 (m, 16H), 1.41-1.34 (m, 2H).

### Example 94: Synthesis of N-(3-nitro-4-methoxybenzoyl)-(5R)-matrinic acid:

According to the step 3 of Example 79, the acylating agent was 3-nitro-4-methoxybenzoyl chloride, to obtain yellow solid of 0.07 g(yield: 3.4%), melting point: 201.4-203.0°C.
MS-ESI (M/Z): 446.5 [M+H]⁺
¹H NMR (DMSO-d*₆*, 400 Hz, ppm): δ 7.84-7.79 (m, 1H), 7.67-7.59 (m, 1H), 7.44-7.39 (m, 1H), 4.19-4.17 (m, 2H), 3.61-3.16 (m, 6H), 3.11-2.97 (m, 2H), 2.85 (s, 2H), 2.18-2.04 (m, 3H), 1.87-1.03 (m, 12H).

### Synthesis scheme of Examples 95-114

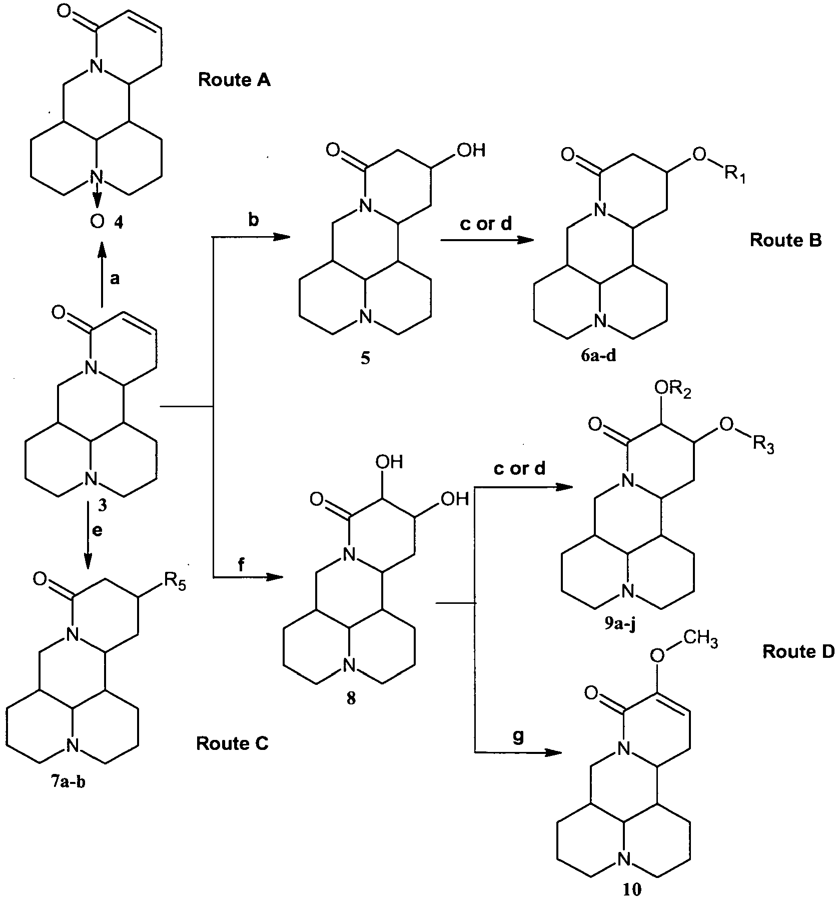

### Example 95: Synthesis of oxidized sophocarpine (4):

Sophocarpine(1.0 g, 4.07 mmol) was added to 30%H₂O₂ (2 mL, 19.6 mmol) at room temperature with stirring, reacted at 50°Cfor 16 h, extracted with CH₂Cl₂ to remove unreacted sophocarpine, the residue was concentrated, mixed with silica gel, and flash column chromatography was performed using CH₂Cl₂ and MeOH as mobile phases to obtain oxidized sophocarpine, white solid (0.7 g, 66%). Mp 207-208°C. 1H NMR (CD30D, 400 MHz): δ 6.61-6.66 (m, 1H), 5.80 (dd, J = 9.6, 2.4 Hz, 1H), 4.48-4.55 (m, 1H), 3.87-3.92 (m, 1H), 3.74 (t, J = 12.8 Hz, 1H), 3.32-3.41 (m, 3H), 3.16-3.25 (m, 1H), 2.67-2.74 (m, 1H), 2.31-2.51 (m, 3H), 1.94-2.10 (m, 3H), 1.67-1.87(m, 4H), 1.56-1.64 (m, 2H); HRMS: calcd for C15H23N2O2 (M+H)+, 263.1760; found, 263.1758.

### Example 96: Synthesis of 13-hydroxyl matrine(5):

At room temperature, sophocarpine(12.3 g, 0.05 mol) was added to an aqueous solution of KOH (33.6 g, 0.6 mol) in water (300 mL) , refluxed for 8h, cooled to 0°C, neutralized with 3N hydrochloric acid to PH=7, concentrated to dryness, added with 150mL of methanol, suction filtered to remove inorganic salt, the residue was concentrated, and flash column chromatography was performed using CH₂Cl₂ and MeOH as mobile phases to obtain 13-hydroxyl matrine, white solid (6.2 g, 47%). Mp 211-213 °C. 1H NMR (CD30D, 400 MHz): δ 4.51 (dd, J = 4.4, 13.8 Hz, 1H), 4.14-4.18 (m, 1H), 3.86-3.92 (m, 1H), 3.47 (s, 1H), 3.36 (m, 2H), 3.24-3.26 (m, 1H), 2.89-3.04 (m, 3H), 2.52 (dd, J = 3.6, 17.6 Hz, 1H), 2.37 (dt, J = 17.6, 3.2 Hz, 1H), 2.17-2.24 (m, 1H), 1.95-2.04 (m, 2H), 1.81-1.94 (m, 3H), 1.69-1.79 (m, 4H), 1.59-1.68 (m, 1H); HRMS: calcd for C15H25N2O2 (M+H)+, 265.1916; found, 265.1921.

### Example 97: Synthesis of 13-methoxy matrine(6a):

The Compound SC-27-C(0.8. g, 3 mmol) in 50% KOH aqueous solution(2 mL) was added dropwise to iodomethane (3.3 mmol), stirred at room temperature for 8h, after the end of reaction, neutralized with 3N hydrochloric acid, extracted with CH₂Cl₂, the organic phases were combined, dried over Na₂SO₄. After filtration and concentration, flash column chromatography was performed using cyclohexane and EA as mobile phases to obtain 13-methoxy matrine, white solid (0.18g, 22%). Mp 62-64 °C. 1H NMR (CDC13, 400 MHz): δ 4.30 (dd, J = 4.4, 12.4 Hz, 1H), 3.91-3.97 (m, 1H), 3.63 (d, J = 4 Hz, 1H), 3.28 (s, 3H), 3.06 (t, J = 12.8 Hz, 1H), 2.73-2.81 (m, 2H), 2.40-2.56 (m, 2H), 2.15-2.20 (m, 1H), 2.06 (s, 1H), 1.84-1.96 (m, 3H), 1.61-1.74 (m, 4H), 1.33-1.57 (m, 6H); HRMS: calcd for C16H27N2O2 (M+H)+, 279.2073; found, 279.2085.

### Example 98: Synthesis of 13-benzyloxy matrine(6c):

Compound SC-27-C(0.8 g, 3 mmol) in 50%KOH aqueous solution (2 mL) was added dropwise to benzyl bromide(3.3 mmol), stirred at room temperature for 8h, after the end of reaction, neutralized with 3N hydrochloric acid, extracted with CH₂Cl₂, the organic layers were combined, dried withNa₂SO₄. After filteration, concentration, flash column chromatography was performed using cyclohexane and EA as mobile phases to obtain 13-benzyloxy matrine, white solid (0.6g, 57%). Mp 121-123 °C. 1H NMR (CDCl3, 400 MHz): δ 7.24-7.34 (m, 5H), 4.47-4.56 (m, 2H), 4.35 (dd, J = 4.0, 12.8 Hz, 1H), 3.99-4.05 (m, 1H), 3.84 (d, J = 4.0 Hz, 1H), 3.10 (t, J = 12.8 Hz, 1H), 2.76-2.84 (m, 2H), 2.45-2.65(m, 2H), 2.16-2.23 (m, 1H), 2.09 (s, 1H),1.82-1.98 (m, 3H), 1.33-1.77 (m, 10H); HRMS: calcd for C22H31N2O2 (M+H)+, 355.2386; found, 355.2371.

### Example 99: Synthesis of 13-ethoxy matrine(6b):

Compound SC-27-C(0.8 g, 3 mmol) in 50% KOH aqueous solution (2 mL) was added dropwise with ethyl iodide (3.3 mmol), stirred at room temperature for 8h, after the end of reaction, neutralized with 3N hydrochloric acid, extracted with CH₂Cl₂, the organic layers were combined, dried over Na₂SO₄. After filteration and concentraiton, flash column chromatography was performed using cyclohexane and EA as mobile phases to obtain 13-ethoxy matrine, white solid (0.17g, 20%). Mp 71-73 °C. 1H NMR (CDC13, 400 MHz): δ 4.54-4.61 (m, 1H), 3.47-3.90 (m, 7H), 3.17 (s, 1H), 2.51-2.74 (m, 3H), 2.30-2.48 (m, 3H), 1.61-2.10 (m, 10H), 1.22 (t, J = 6.8 Hz, 3H); HRMS: calcd for C17H29N2O2 (M+H)+, 293.2229; found, 293.2230.

### Example 100: Synthesis of 13-benzoyloxy matrine(6d):

SC-27-C(1.0 g, 3.79 mmol) in anhydrous CH₂Cl₂ (10.0 mL) solution was added dropwise to benzoyl chloride(0.48 mL, 4.17 mmol), after dropwise addition, added with KOH powder (0.42 g, 7.5 mmol) in batches, the mixture was stirred at room temperature for 4h, filtered to remove inorganic salt, the organic layer was concentrated, and flash column chromatography was performed using CH₂Cl₂ and MeOH as mobile phase to obtain 13-hydroxyl matrine, white solid (0.51 g, 37%). Mp 108-110 °C, 1H NMR (CDCl3, 400 MHz): δ 7.96 (d, J = 7.2 Hz, 2H), 7.56 (t, J = 7.6 Hz, 1H), 7.41-7.45 (m, 2H), 5.38-5.39 (m, 1H), 4.32 (dd, J = 4.4, 12.8 Hz, 1H), 4.05 (m, 1H), 3.10 (t, J = 12.8 Hz, 1H), 2.47-2.81 (m, 4H), 2.40-2.46 (m, 1H), 2.17 (s, 1H), 1.91-2.03 (m, 3H), 1.39-1.82 (m, 10H); HRMS: calcd for C22H29N2O3 (M+H)+, 369.2178; found, 369.2188.

### Example 101: Synthesis of 13-nitromethyl matrine(7a):

Sophocarpine(4.92 g, 20 mmol) in acetonitrile (10 mL) solution was added to nitromethane (1.08 mL, 20 mmol), stirred at room temperature, added with DBU (3.04 g, 20 mmol), stirred at room temperature overnight, the reaction solution was poured in 50ml of water, adjusted with diluted hydrochloric acid to PH=2,then extracted with CH₂Cl₂, dried over Na₂SO_{4.} suction filtered, concentrated.Using EA and MeOH as mobile phases, flash column chromatography was performed to obtain 13-nitromethyl matrine, white solid (2.90 g, 47%). Mp 80-82 °C. 1H NMR (CDCl3, 400 MHz): δ 4.28-4.39 (m, 3H), 4.04-4.09 (m, 1H), 3.12 (t, J = 12.4 Hz, 1H), 2.78-2.85 (m, 3H), 2.56 (dd, J = 5.2, 17.2 Hz, 1H), 2.16-2.24 (m, 2H), 1.91-2.01 (m, 5H), 1.82-1.90 (m, 3H), 1.51-1.78 (m, 3H), 1.38-1.48 (m, 3H); HRMS: calcd for C16H26N3O3 (M+H)+, 308.1974; found, 308.1973.

### Example 102: Synthesis of 13-methylaminomatrine(7b):

At 0°C, metal sodium (0.46 g, 20 mmol) in batches was added to a solution of methylamine in ethanol (60 mL), after the end of reaction, added with sophocarpine(2.46 g, 10 mmol), heated to room temperature, stirred for 24h, concentrated to dryness, added with ethyl ether, filtered to remove the precipitated solid, the filtrate was concentrated, and flash column chromatography was performed using CH₂Cl₂ and MeOH as mobile phases to obtain 13-methylaminomatrine, white solid (1.1 g, 40%). Mp 80-81 °C. 1H NMR (CDC13, 400 MHz): δ 4.34 (dd, J = 4.4, 12.8 Hz, 1H), 4.00 (br, 1H), 3.10 (t, J = 12.8 Hz, 1H), 2.94 (m, 1H), 2.83 (m, 2H), 2.56 (dd, J = 4.4, 16.8 Hz, 1H), 2.46 (s, 3H), 2.32 (dd, J = 5.6, 16.8 Hz, 1H), 2.12 (s, 1H), 1.89-1.97 (m, 4H), 1.63-1.77 (m, 5H), 1.37-1.54 (m, 6H); HRMS: calcd for C16H28N30 (M+H)+, 278.2232; found, 278.2243.

### Example 103: Synthesis of 13,14-dihydroxyl matrine(8):

At 0°C, to sophocarpine(6.15 g, 25 mmol) in mixture solution of water (20 mL) and acetone (20 mL) was added KMnO₄ (5.93 g, 37.5 mmol) in batches, the mixture reacted at 0°C for about 1h, TLC detection showed the completation of reaction. Methanol (50 mL) was added, the mixture was suction filtered, the filter cake was washed with methanol, the filtrate was concentrated to remove methanol and acetone, the residue was added to 10% NaOH aqueous solution to regulate PH as 10-11, extracted with CH₂Cl₂ for 3 times, the organic layers were combined, dried ove anhydrous Na₂SO₄. After concentration, ethyl ether was added to precipitate solid, suction filtered to obtain white solid (3.3 g, 47%). Mp 156-158 °C. 1H NMR (CD3Cl, 400 MHz): δ 4.27 (s, 1H), 4.09-4.18 (m, 3H), 3.96 (s, 1H), 3.21 (t, J = 12.4 Hz, 1H), 2.80 (m, 2H), 2.60 (s, 1H), 2.38 (d, J = 13.2 Hz, 1H), 2.07 (m, 1H), 1.85-2.04 (m, 3H), 1.41-1.71 (m, 10H); HRMS: calcd for C15H25N2O3 (M+H)+, 281.1865; found, 281.1882.

### Example 104: Synthesis of 13,14-dimethoxy matrine(9a):

At room temperature and with stirring, Compound SC-1(0.84g, 3 mmol) in 50%KOH(2 mL) solution was added dropwise to methyl iodide(0.4mL, 6.5 mmol), stirred at room temperature for 8h, after reaction, neutralized with 3N diluted hydrochloric acid, extracted with CH₂Cl₂, the organic layers were combined, dried over anhydrous Na₂SO₄. After concentration; using CH₂Cl₂ as mobile phase, flash column chromatography was performed to obtain 13, 14-dimethoxy matrine, white solid (0.32g, 35%). Mp 77-79 °C. 1H NMR (CD30D, 400 MHz): δ 4.16 (dd, J = 4.4, 12.8 Hz, 1H), 3.78-3.84 (m, 1H), 3.60-3.63 (m, 1H), 3.56-3.57 (m, 1H), 3.44 (s, 3H), 3.35 (s, 3H), 3.02 (t, J = 12.8 Hz, 1H), 2.74-2.81 (m, 2H), 2.14-2.20 (m, 2H), 1.91-2.02 (m, 3H), 1.21-1.76 (m, 10H); HRMS: calcd for C17H29N2O3 (M+H)+, 309.2178; found, 309.2191.

### Example 105: Synthesis of 13,14-dibenzyloxy matrine(9b):

At room temperature and with stirring, Compound SC-1(0.84g, 3 mmol) in 50%KOH(2 mL) solution was added dropwise to benzyl bromide(0.77mL, 6.5 mmol), stirred at room temperature for 8h, after the end of reaction, neutralized with 3N diluted hydrochloric acid, extracted with CH₂Cl₂, the organic layers were combined, dried over anhydrous Na₂SO₄. After concentration, using CH₂Cl₂ as mobile phase, flash column chromatography was performed to obtain 13,14-dimethoxy matrine, white solid (1.12g, 81%). Mp 105-108 °C. 1H NMR (CD30D, 400 MHz): δ 7.21-7.43 (m, 10H), 4.99 (d, J = 12.4 Hz, 1H), 4.66 (m, 3H), 4.45 (m, 2H), 3.30-3.90 (m, 6H), 2.69 (m, 2H), 2.07-2.27 (m, 3H), 1.59-1.89 (m, 8H), 1.39 (m, 1H); HRMS: calcd for C29H37N2O3 (M+H)+, 461.2804; found, 461.2820.

### Example 106: Synthesis of 13,14-diacetyloxy matrine(9c):

To a solution of SC-1(0.84g, 3 mmol) in anhydrous CH₂Cl₂ (5.0 mL) was added dropwise chloroacetyl chloride (0.68mL, 9 mmol), after dropwise addition, added with KOH(0.34g, 6mmol) powder, stirred at room temperature for 8h, filtered to remove inorganic salt, concentrated, flash column chromatography was performed using CH₂Cl₂ as mobile phase to obtain 13,14-diacetyloxy matrine, white solid (0.38g, 30%), Mp 64-66 °C. 1H NMR (CD30D, 400 MHz): δ 5.31 (d, J = 2.8 Hz, 1H), 5.23 (d, J = 2.8 Hz, 1H), 4.10-4.20 (m, 4H), 3.86-3.93 (m, 1H), 3.09 (t, J = 12.8 Hz, 1H), 2.73-2.80 (m, 2H), 2.46 (dt, J = 15.2, 5.6 Hz, 1H), 2.14 (s, 1H), 1.93-2.01 (m, 2H), 1.72-1.88 (m, 2H), 1.39-1.71 (m, 10H), 1.24 (m, 6H); HRMS: calcd for C21H33N2O7 (M+H)+, 425.2282; found, 425.2325.

### Example 107: Synthesis of 13,14-di(4-fluoro-3-nitro) benzoyloxy matrine(9d):

To a solution of SC-1(0.84g, 3 mmol), in anhydrous CH₂Cl₂ (5.0 mL) was added dropwise 4-fluoro-3-nitrobenzoyl chloride(1.83g, 9 mmol), after dropwise addition, stirred at room temperature for 24h, concentrated, and flash column chromatography was performed using CH₂Cl₂ and methanol as mobile phases to obtain 13,14-di(4-fluoro-3-nitro) benzoyloxy matrine, white solid (0.57g, 31%). Mp 115-117 °C. 1H NMR (CD30D, 400 MHz): δ 8.62 (dd, J = 7.2, 2.4 Hz, 1H), 8.48 (dd, J = 7.2, 2.0 Hz, 1H), 8.34-8.38 (m, 1H), 8.22-8.26 (m, 1H), 7.54-7.59 (m, 1H), 7.45-7.50 (m, 1H), 5.83-5.87 (m, 2H), 4.28 (dd, J = 13.2, 4.4 Hz, 1H), 4.09-4.16 (m, 1H), 3.23 (t, J = 12.8 Hz, 1H), 2.80-2.84 (m, 2H), 2.61-2.67 (m, 1H), 2.22 (s, 1H), 1.41-2.12 (m, 13H); HRMS: calcd for C29H29N4O9F2 (M+H)+, 615.1903; found, 615.1885.

### Example 108: Synthesis of 14-hydroxyl-13-acetyloxy matrine(9e):

To a solution of SC-1(0.84g, 3 mmol) in anhydrous CH₂Cl₂ (5.0 mL) was added dropwise chloroacetyl chloride (0.64mL, 9 mmol), after dropwise addition, stirred at room temperature for 8h, concentrated, and flash column chromatography was performed using CH₂Cl₂ and as mobile phases to obtain 14-hydroxyl-13-acetyloxy matrine, white solid (0.2g, 21%). Mp 194-196 °C. 1H NMR (CDC13, 400 MHz): δ 12.22 (s, 1H), 5.19 (s, 1H), 5.00 (s, 1H), 4.63 (m, 1H), 4.56 (dd, J = 4, 14.4 Hz, 1H), 4.33-4.40 (m, 2H), 3.74 (t, J = 13.6 Hz, 1H), 3.51-3.59 (m, 2H), 3.13 (d, J = 9.2 Hz, 1H), 2.52-2.66 (m, 3H), 2.33-2.43 (m, 2H), 2.02-2.13 (m, 3H), 1.87-1.90 (m, 1H), 1.63-1.75 (m, 6H); HRMS: calcd for C17H27N2O4 (M+H)+, 323.1971; found, 323.1966.

### Example 109: Synthesis of 14-hydroxyl-13-chloroacetyloxy matrine(9f):

To a solution of SC-1(0.84g, 3 mmol) in anhydrous CH₂Cl₂ (5.0 mL) was added dropwise chloroacetyl chloride (0.68mL, 9 mmol), after dropwise addition, stirred at room temperature for 8h, concentrated, and flash column chromatography was performed using CH₂Cl₂ and ethanol as mobile phases to obtain 14-hydroxyl-13-chloroacetyloxy matrine, white solid (0.27g, 25%). Mp 226-227 °C. 1H NMR (CD30D, 400 MHz): δ 5.43 (s, 1H), 4.41-4.42 (m, 1H), 4.32 (d, J = 15.2 Hz, 1H), 4.19 (d, J = 15.2 Hz, 2H), 3.95 (m, 1H), 3.36-3.57 (m, 4H), 2.97-3.05 (m, 3H), 2.46-2.50 (m, 1H), 1.69-2.07 (m, 10H); HRMS: calcd for C17H26ClN2O4 (M+H)+, 357.1581; found, 357.1582.

### Example 110: Synthesis of 14-hydroxyl-13-2-chloropropionyloxy matrine(9g):

To a solution of SC-1(0.84g, 3 mmol) in anhydrous CH₂Cl₂ (5.0 mL) was added dropwise 2-chloropropionyl chloride (0.92mL, 9 mmol), after dropwise addition, stirred at room temperature for 5h, concentrated to obtain a light yellow crude product, which was recrystallized with ethanol for twice to obtain white solid (0.57g, 51%). Mp 239-240 °C. 1H NMR (CD30D, 400 MHz): δ 5.32-5.34 (m, 1H), 4.58-4.63 (m, 1H), 4.37 (dt, J = 4.4, 14 Hz, 1H), 4.26 (d, J = 2.8 Hz, 1H), 3.77-3.84 (m, 1H), 3.50 (s, 1H), 3.37 (t, J = 13.2 Hz, 2H), 3.02 (m, 2H), 2.87 (t, J = 13.6 Hz, 1H), 2.36 (dt, J = 5.2, 14.4 Hz, 1H), 1.96-2.10 (m, 3H), 1.71-1.86 (m, 8H), 1.67-1.69 (m, 3H); HRMS: calcd for C18H28ClN2O4 (M+H)+, 371.1738; found, 371.1753.

### Example 111: Synthesis of 14-hydroxyl-13-benzoyloxy matrine(9h):

To a solution of SC-1(0.84g, 3 mmol) in anhydrous CH₂Cl₂ (5.0 mL) was added dropwise benzoyl chloride(1.0mL, 9 mmol), after dropwise addition, stirred at room temperature for 3h, concentrated, flash column chromatography was performed using CH₂Cl₂ and MeOH as mobile phase to obtain 14-hydroxyl-13-benzoyloxy matrine, white solid (0.36g, 31%). Mp 225-226 °C.1H NMR (CD30D, 400 MHz): δ 8.10 (d, J = 7.6 Hz, 2H), 7.58 (t, J = 7.6 Hz, 1H), 7.44 (t, J = 7.6 Hz, 2H), 5.55 (s, 1H), 4.37-4.44 (m, 2H), 3.79-3.86 (m, 1H), 3.51 (s, 1H), 3.01-3.06 (m, 2H), 2.86 (t, J = 13.6 Hz, 1H), 2.38-2.44 (m, 1H), 1.79-2.13 (m, 12H), 1.12 (t, J = 6.8 Hz, 1H); HRMS: calcd for C22H29N2O4 (M+H)+, 385.2127; found, 385.2131.

### Example 112: Synthesis of 14-hydroxyl-13-(4-fluoro-3-nitro)benzoyloxy matrine(9i):

To a solution of SC-1(0.84g, 3 mmol) in anhydrous CH₂Cl₂ (5.0 mL) was added dropwise 4-fluoro-3-nitrobenzoyl chloride(1.83g, 9 mmol), after dropwise addition, stirred at room temperature for 1h, concentrated, and flash column chromatography was performed using CH₂Cl₂ and MeOH as mobile phase to obtain 14-hydroxyl-13-(4-fluoro-3-nitro) benzoyloxy matrine, white solid (0.42g, 31%). Mp 103-105 °C. 1H NMR (CD3OD, 400 MHz): δ 8.60 (dd, J = 2.0, 7.2 Hz, 1H), 8.26-8.30 (m, 1H), 7.49-7.53 (m, 1H), 5.60 (t, J = 2.8 Hz, 1H), 4.24-4.30 (m, 2H), 3.88-3.95 (m, 1H), 3.14 (t, J = 12.8 Hz, 1H), 2.79 (d, J = 11.2 Hz, 2H),2.53 (dt, J = 5.6, 14.8 Hz, 1H), 2.24 (s, 1H), 2.01-2.06 (t, J = 10.8 Hz, 2H), 1.38-1.89 (m, 11H); HRMS: calcd for C22H27N3O6F (M+H)+, 448.1883; found, 448.1881.

### Example 113: Synthesis of 14-hydroxyl-13-acetyloxy matrine(9j):

To a solution of SC-1(0.84g, 3 mmol) in anhydrous CH₂Cl₂ (5.0 mL) was added dropwise to ethyl chloroformate(0.29mL, 9 mmol), after dropwise addition, stirred at room temperature for 12h, concentrated, and flash column chromatography was performed using CH₂Cl₂ and ethanol as mobile phase to obtain 14-hydroxyl-13-acetyloxy matrine, white solid (0.30g, 28%). Mp 50-52 °C. 1H NMR (CDC13, 400 MHz): δ 4.99-5.03 (m, 1H), 4.17-4.29 (m, 4H), 3.96-4.00 (m, 1H), 3.13 (t, J = 12.8 Hz, 1H), 2.76-2.83 (m, 2H), 2.17-2.22 (m, 1H), 2.09-2.13 (m, 2H), 1.91-1.99 (m, 2H), 1.34-1.84 (m, 10H), 1.28-1.32 (m, 4H); HRMS: calcd for C18H29N2O5 (M+H)+, 353.2077; found, 353.2076.

### Example 114: Synthesis of 14-methoxy sophocarpine(10):

To SC-1(0.84 g, 3 mmol) in 50% KOH aqueous solution (2 mL) was added dropwise methyl iodide (0.37 mL, 6 mmol), after dropwise addition, added with 1ml of acetone, the mixture was stirred at room temperature for 8h, after the end of reaction, neutralized with 3N diluted hydrochloric acid, extracted with CH₂Cl₂, the organic layers were combined, dried over anhydrous Na₂SO₄. After filtration and concentration, flash column chromatography was performed using CH₂Cl₂ as mobile phase to obtain 14-methoxy sophocarpine, white solid (0.30 g, 36%). Mp 110-111 °C. 1H NMR (CD30D, 400 MHz): δ 5.43 (t, J = 4.0 Hz, 1H), 3.99 (dd, J = 4.4, 13.0 Hz, 1H), 3.80-3.86 (m, 1H), 3.53 (s, 3H), 3.12 (t, J = 12.8 Hz, 1H), 2.77 (t, J = 10.8 Hz, 2H), 2.65 (dt, J = 6, 17.6 Hz, 1H), 2.14-2.24 (m, 2H), 1.84-2.00 (m, 3H), 1.54-1.76 (m, 6H), 1.40-1.49 (m, 3H); HRMS: calcd for C16H25N2O2(M+H)+, 277.1916; found, 277.1908.

### Test Example 1: Detection of HBV Hsc70 gene expression level upon action of compound on cells

2.2.15 cells (purchased from Vertex Pharmaceutical Co., USA) in a number of 1×10⁶ were inocubated to 6-well plate, cultured in a culture medium containing 10% fetal calf serum for 24hr, the original culture medium was discarded, replaced with a culutre medium containing 400µg/ml OMTR, which was used as control. Compounds were firstly dissolved in MEM to form 20mg/ml mother liquid, which was diluted with culture medium when used and then applied to cells. The compounds of Examples 1-114 were separately used for treatment for 12, 24, 36hr, then cells were harvested for extraction of RNA and DNA, then the changes of Hsc70 mRNA and HBV DNA were detected by real-time fluorescent quantified PCR.

Table 1 shows the structures of the compounds in Examples 1-94 of the present invention and their detection results in down-regulation of liver cell Hsc70 gene expression activity.

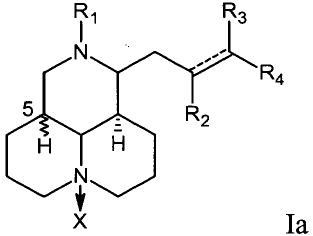

**Table 1**

| Example | Code | X | R₁ | R₂* | R₃* | R₄ | 5-C | Hsc70 inhibition rate ** |
|---|---|---|---|---|---|---|---|---|
| | OMT | | | | | | S | 1 |
| | MT | | | | | | S | -16 |
| 1 | DM-100 | - | H(matrinic acid) | H₂ | H₂ | COOH | S | 1.02 |
| 2 | DM-1001 | O | H(oxymatrinic acid) | H₂ | H₂ | COOH | S | 0.92 |
| 3 | DM-200 | - | H(kurarinol) | H₂ | H₂ | CH₂OH | S | 0.81 |
| 4 | DM-2001 | O | H(oxidized kurarinol) | H₂ | H₂ | CH₂OH | S | -0.42 |
| 5 | DM-101 | - | CH₃CO- | H₂ | H₂ | COOH | S | 0.73 |
| 6 | DM-102 | - | *p*-CH₃C₆H₄SO₂- | H₂ | H₂ | COOH | S | 1.1 |
| 7 | DM-104 | - | ClCH₂CO- | H₂ | H₂ | COOH | S | -7 |
| 8 | DM-104a | - | (*m*-CH₃C₆H₄O)CH₂CO- | H₂ | H₂ | COOH | S | -2.98 |
| 9 | DM-105 | - | HOCH₂CO- | H₂ | H₂ | COOH | S | < 10 |
| 10 | DM-106a | - | CH₃CHOHCO- | H₂ | H₂ | COOH | S | -54 |
| 11 | DM-106 | - | CH₃(*m*-CH₃C₆H₄O)CHCO- | H₂ | H₂ | COOH | S | 1.67 |
| 12 | DM-107 | - | C₆H₅CO- | H₂ | H₂ | COOH | S | 1.04 |
| 13 | DM-121 | - | *m*-NO₂C₆H₄CO- | H₂ | H₂ | COOH | S | 0.23 |
| 14 | DM-131 | - | 2-CH₃-5-NO₂C₆H₃CO- | H₂ | H₂ | COOH | S | -0.86 |
| 15 | DM-108 | - | C₆H₅CH₂- | H₂ | H₂ | COOH | S | 1.25 |
| 16 | DM-1081 | O | C₆H₅CH₂- | H₂ | H₂ | COOH | S | 1.26 |
| 17 | DM-109 | - | C₆H₅SO₂- | H₂ | H₂ | COOH | S | <10 |
| 18 | DM-110 | - | C₆H₅CH=CHCO- | H₂ | H₂ | COOH | S | <10 |
| 19 | DM-111 | - | CH₃CH₃⁺- | H₂ | H₂ | COOH | S | 0.25 |
| 20 | DM-1111 | O | CH₃CH₃⁺- | H₂ | H₂ | COOH | S | -0.25 |
| 21 | DM-112 | - | CH₃CH₂- | H₂ | H₂ | COOH | S | <10 |
| 22 | DM-113 | - | CH₃CH₂CH₂- | H₂ | H₂ | COOH | S | <10 |
| 23 | DM-115 | - | (CH₂CH₂CH)CH₂- | H₂ | H₂ | COOH | S | -33 |
| 24 | DM-1151 | O | (CH₂CH₂CH)CH₂- | H₂ | H₂ | COOH | S | 0.52 |
| 25 | DM-117 | - | HOCH₂CH₂- | H₂ | H₂ | COOH | S | 0.84 |
| 26 | DM-122 | - | *p*-CH₃OC₆H₄CH₂- | H₂ | H₂ | COOH | S | 1.56 |
| 27 | DM-1221 | O | *p*-CH₃OC₆H₄CH₂- | H₂ | H₂ | COOH | S | 1.34 |
| 28 | DM-123 | - | *p-*NO₂C₆H₄CH₂- | H₂ | H₂ | COOH | S | -1.6 |
| 29 | DM-124 | - | *o*-ClC₆H₄CH₂- | H₂ | H₂ | COOH | S | 0.32 |
| 30 | DM-125 | - | *m*-ClC₆H₄CH₂- | H₂ | H₂ | COOH | S | <10 |
| 31 | DM-126 | - | *p*-ClC₆H₄CH₂- | H₂ | H₂ | COOH | S | 0.62 |
| 32 | DM-127 | - | 3-Cl-4-ClC₆H₃CH₂- | H₂ | H₂ | COOH | S | -2.92 |
| 33 | DM-128 | - | *p*-BrC₆H₄CH₂- | H₂ | H₂ | COOH | S | -5.78 |
| 34 | DM-129 | - | 2-Cl-4-ClC₆H₃CH₂- | H₂ | H₂ | COOH | S | 0.27 |
| 35 | DM-132 | - | *p*-FC₆H₄CH₂- | H₂ | H₂ | COOH | S | 0.99 |
| 36 | DM-133 | - | *m*-FC₆H₄CH₂- | H₂ | H₂ | COOH | S | 0.81 |
| 37 | DM-134 | - | *p*-CNC₆H₄CH₂- | H₂ | H₂ | COOH | S | 0.57 |
| 38 | DM-135 | - | *o-*FC₆H₄CH₂- | H₂ | H₂ | COOH | S | 0.41 |
| 39 | DM-136 | - | (*p*-CH₂=CH)C₆H₄CH₂- | H₂ | H₂ | COOH | S | 0.76 |
| 40 | DM-137 | - | *m*-NO₂C₆H₄CH₂- | H₂ | H₂ | COOH | S | 1.31 |
| 41 | DM-138 | - | *o*-CH₃C₆H₄CH₂- | H₂ | H₂ | COOH | S | 1.35 |
| 42 | DM-139 | - | *m*-CH₃C₆H₄CH₂- | H₂ | H₂ | COOH | S | 1.39 |
| 43 | DM-140 | - | *p*-CH₃C₆H₄CH₂- | H₂ | H₂ | COOH | S | 0.77 |
| 44 | DM-142 | - | *m*-CH₃OC₆H₄CH₂- | H₂ | H₂ | COOH | S | 1.07 |
| 45 | DM-143 | - | 2-C₅H₄NCH₂- | H₂ | H₂ | COOH | S | 1.1 |
| 46 | DM-144 | - | 3-C₅H₄NCH₂- | H₂ | H₂ | COOH | S | |
| 47 | DM-145 | - | 4-C₅H₄NCH₂- | H₂ | H₂ | COOH | S | |
| 48 | DM-148 | - | 1-naphthylbenzyl- | H₂ | H₂ | COOH | S | |
| 49 | DM-151 | - | 4-CH₃OC₆H₄CO- | H₂ | H₂ | COOH | S | |
| 50 | DM-152 | - | 4-CNC₆H₄CO- | H₂ | H₂ | COOH | S | |
| 51 | DM-153 | - | 4-CH₃C₆H₄CO- | H₂ | H₂ | COOH | S | |
| 52 | DM-154 | - | 4-FC₆H₄CO- | H₂ | H₂ | COOH | S | |
| 53 | DM-155 | - | 4-CF₃C₆H₄CO- | H₂ | H₂ | COOH | S | |
| 54 | DM-103 | - | 4-CH₃OC₆H₄SO₂- | H₂ | H₂ | COOH | S | |
| 55 | DM-162 | - | 4-CF₃OC₆H₄CH₂- | H₂ | H₂ | COOH | S | |
| 56 | DM-146 | - | 4-C₃H₂SNCH₂- | H₂ | H₂ | COOH | S | 0.92 |
| 57 | SC-1 | - | H | H | H | COOH | S | |
| 58 | SC-15 | - | C₆H₅CO- | H | H | COOH | S | |
| 59 | SC-17 | - | C₆H₅CH₂- | H | H | COOH | S | |
| 60 | SC-19 | - | C₂H₅OCO- | H | H | COOH | S | |
| 61 | SC-21 | - | CH₃CH₂CH₂- | H | H | COOH | S | |
| 62 | SC-22 | - | 2-F-4-BrC₆H₃CH₂- | H | H | COOH | S | |
| 63 | SC-23 | - | (CH₃)₃CO- | H | H | COOH | S | |
| 64 | SC-32 | - | CH₃(CH₂)₄- | H | H | COOH | S | |
| 65 | SC-36 | - | 4-CNC₆H₄CH₂- | H | H | COOH | S | |
| 66 | SC-61A | - | 4-NO₂C₆H₄CH₂- | H | H | COOH | S | |
| 67 | SC-62A | - | 2-CH₃C₆H₄CH₂- | H | H | COOH | S | |
| 68 | SC-64A | - | 4-BrC₆H₄CH₂- | H | H | COOH | S | |
| 69 | SC-69A | - | 4-CF₃C₆H₄CH₂- | H | H | COOH | S | |
| 70 | SC-84A | - | 4-CF₃C₆H₄SO₂- | H | H | COOH | S | |
| 71 | SC-89B | - | 3-CNC₆H₄SO₂- | H | H | COOH | S | |
| 73 | SC-17C | - | C₆H₅CH₂- | OH | H₂ | COOH | S | |
| 74 | SC-36C | - | 4-CNC₆H₄CH₂- | OH | H₂ | COOH | S | |
| 75 | SC-62B | - | 2-CH₃C₆H₄CH₂- | OH | H₂ | COOH | S | |
| 76 | SC-64B | - | 4-BrC₆H₄CH₂- | OH | H₂ | COOH | S | |
| 77 | SC-65B | - | 3,5-(CH₃)₂C₆H₃CH₂- | OH | H₂ | COOH | S | |
| 78 | SC-69B | - | 4-CF₃C₆H₄CH₂- | OH | H₂ | COOH | S | |
| 79 | SP-3 | - | CH₃CO- | H₂ | H₂ | COOH | R | |
| 80 | SP-6 | - | (CH₃)₃CCO- | H₂ | H₂ | COOH | R | |
| 81 | L1 | - | BrCH₂CO- | H₂ | H₂ | COOH | R | |
| 82 | SP-10 | - | CH₃CH₂OCO- | H₂ | H₂ | COOH | R | |
| 83 | L4 | - | CH₃OCO- | H₂ | H₂ | COOH | R | |
| 84 | L5 | - | C₆H₅CH₂OCO- | H₂ | H₂ | COOH | R | |
| 85 | SP-8 | - | C₆H₅SO₂- | H₂ | H₂ | COOH | R | |
| 86 | SP-7 | - | 4-CH₃C₆H₄SO₂- | H₂ | H₂ | COOH | R | |
| 87 | SP-4 | - | C₆H₅CO- | H₂ | H₂ | COOH | R | |
| 88 | L9 | - | 3-NO₂C₆H₄CO- | H₂ | H₂ | COOH | R | |
| 89 | L7 | - | 4-CH₃C₆H₄CO- | H₂ | H₂ | COOH | R | |
| 90 | L11 | - | 4-OCH₃C₆H₄CO- | H₂ | H₂ | COOH | R | |
| 91 | L10 | - | 4-FC₆H₄CO- | H₂ | H₂ | COOH | R | |
| 92 | L2 | - | 2-CH₃-5-NO₂C₆H₃CO- | H₂ | H₂ | COOH | R | |
| 93 | L8 | - | 3-NO₂-4-FC₆H₃CO- | H₂ | H₂ | COOH | R | |
| 94 | L12 | - | 3-NO₂-4-OCH₃C₆H₃CO- | H₂ | H₂ | COOH | R | |
| Notation: * in the column, "H₂" represents that the R₂ or R₃ group separately is 2 hydrogen atoms, so that --------- forms a single bond; similarly, in the column, "H" represents that the R₂ or R₃ group separately is 1 hydrogen atom, so that --------- forms a double bond. ** represents down-regulation of Hsc70 expression activity, expressed in relative OMT ratio. | | | | | | | | |

Table 2 shows the structures of the compounds of Formula (II) in Examples 95-114 of the present invention, and their detection results of down-regulation of liver cell Hsc70 gene.

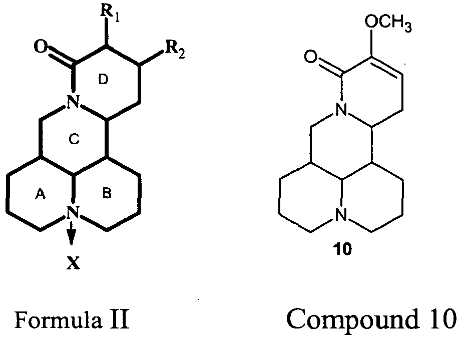

**Table 2**

| Compound No. | R1 | R2 | X | Hsc70 mRNA |
|---|---|---|---|---|
| 1 | H | H | O | 55.0 ± 0.10 |
| 2 | H | H | | 52.4 ± 0.17 |
| 3 | CH= | CH= | | 21.6 ± 0.13 |
| 4 | CH= | CH= | O | 15.3 ± 0.21 |

| In the following compounds 5-10, N is not substituted: | | | | |
|---|---|---|---|---|
| Compound No. | R1 | R2 | | Hsc70 mRNA |
| 5 | H | OH | | 50.4 ± 0.19 |
| 6a | H | OCH3 | | 48.6 ± 0.16 |
| 6b | H | OC2H5 | | 74.1 ± 0.11 |
| 6c | H | OCH2C6H5 | | 24.7 ± 0.10 |
| 6d | H | OCOC6H5 | | 51.0 ± 0.11 |
| 7a | H | CH2NO2 | | 35.8 ± 0.09 |
| 7b | H | NHCH3 | | 40.1 ± 0.09 |
| 8 | OH | OH | | 38.9 ± 0.13 |
| 9a | OCH3 | OCH3 | | 39.3 ± 0.20 |
| 9b | OCH2C6H5 | OCH2C6H5 | | 45.4 ± 0.11 |
| 9c | OCO2Et | OCO2Et | | <10 |
| 9d | OCOC6H5F-p-NO2-m | OCOC6H5F-p-NO2-m | | 30.8 ± 0.06 |
| 9e | OH | OCOCH3 | | 37.1 ± 0.16 |
| 9f | OH | OCOCH2Cl | | 88.2 ± 0.21 |
| 9g | OH | OCOCHClCH3 | | 48.6 ± 0.11 |
| 9h | OH | OCOC6H5 | | 34.5 ± 0.10 |
| 9i | OH | OCOC6H5F-p-NO2-m | | 43.4 ± 0.08 |
| 9j | OH | OCO2Et | | 54.8 ± 0.21 |
| 10 | | | | 39.7 ± 0.16 |

In sum, the above detections show that the compounds of the present invention have activity in down-regulation of Hsc70 expression, it has been known that the compounds targeting to liver cell Hsc70 are featured with broad antiviral spectrum (including hepatitis B virus, hepatitis C virus and ADIS virus), not easy to generate drug resistance, high safety. Hence, the compounds of the present invention can be used for a broad spectrum of anti-viruses.

### Test Example 2: In vitro effects of the compounds on HBV DNA replication in 2.2.15 cells

### Drug TC₅₀ by MTT detection

2.2.15 cells (all cells were purchased from Vertex Pharmaceutical Co., USA) in exponential growth phase were inoculated on 96-well culture plate, 2×10⁵ cells/well, added with culture media containing PFA diluted in different concentration rates, 3 wells for each diluted concentration, placed in 37°C CO₂-containing incubator and cultivated for 48 h; the supernatant was discarded, 100 µl of MTT (0.5 mg/ml) in culture medium was added, cultivation was continuously performed at 37°C for 4 h;each well was added to 100 µl of 50% DMF-20% SDS destaining solution, stood at 37°C overnight; ELIASA was used to measure optical density at wavelength of 570 mn(OD₅₇₀).

In each experiment, 3 cell control wells and 3 blank controls well were set, the results were used in formula, (cell control OD₅₇₀- drug-medicated cell OD₅₇₀)/cell control OD₅₇₀, to calculate cell death rate (%), and Reed-Muench method was used to calculate half toxic concentration TC₅₀. The results are shown in Table 3.

### Test Example 3: DHBV animal test

Infection and administration: Peking ducks (purchased from Institute of Animals, Chinese Academy of Medical Sciences) were infected via foot vein with 0.2ml DHBV positive serum. Blood samples were collected on the 7^{th} day after infection, each duck was marked and recorded using anklet, and administered with drug after hemostasis. 42 Peking ducks were randomly divided into 7 groups, orally administered daily with Compound DM-122 in doses of 150 mg/kg, 75 mg/kg, 37.5 mg/kg, twice per day, for consecutive 15 days. Bodyweight was expressed as 100g/duck, each duck was administered with 1ml: the corresponding doses were: 15 mg/ml, 7.5 mg/ml, 3.75 mg/ml. The control group was administered with physiological saline (1ml); positive control was administered with 3TC (lamivudine), in dose of 50mg/kg(5mg/ml,1ml).

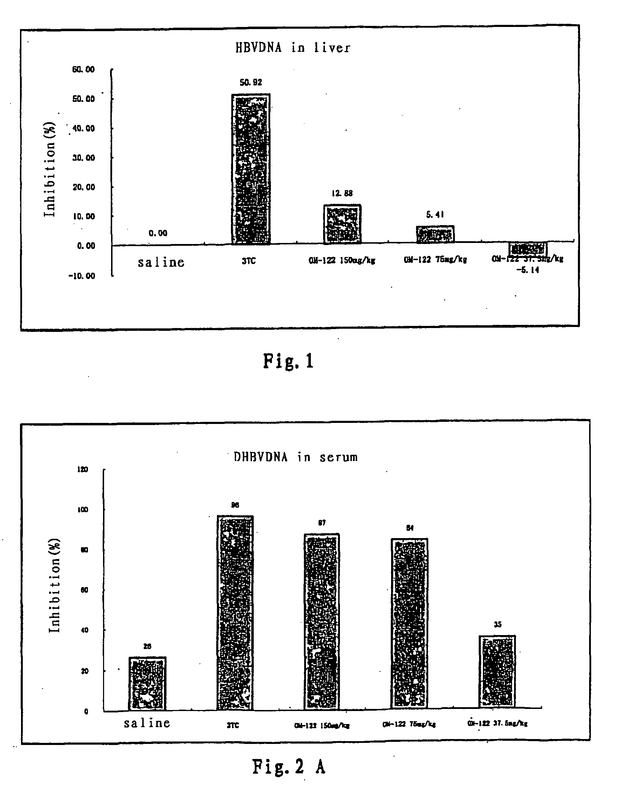

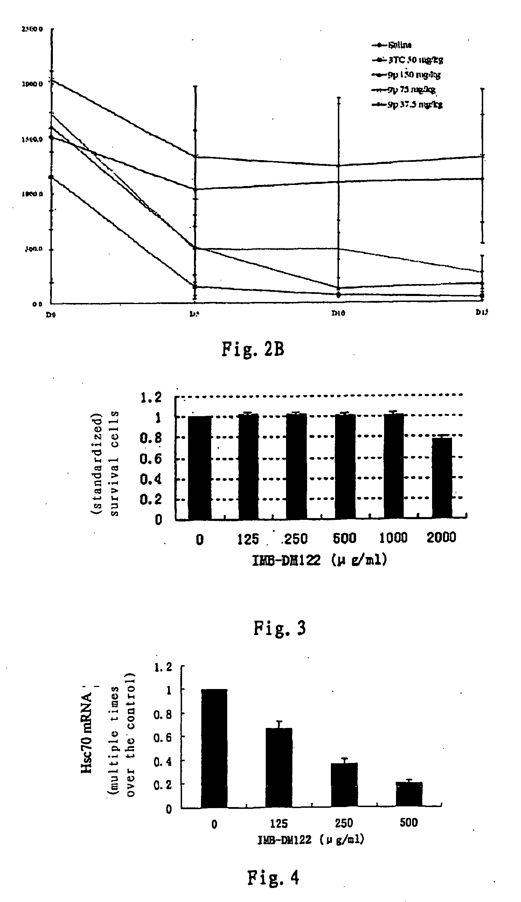

Before administration (T0), and on the 5^{th} day (T5), the 10^{th} day (T10), the 15^{th} day (T15) after administration, blood sample not less than 500µl was separately collected from foot veins, and serum was separated and stored at -70°C; on the 15^{th} day, the animal was killed, laparotomized to take liver, and the liver was washed with pre-cooled physiological saline, cut into pieces, sub-packaged, and stored at -70°. The results are separately shown in Fig.1 and Fig.2(A, B), which separately showed the results of HBV DNA content in liver of duckling upon action of DM122 and the results of HBV DNA content in serum of duckling upon action of DM122.

It can be seen from the results that Compound DM122 as example indicates that the compounds of the present invention have good activity against hepatitis B virus and high safety. Using similar assay method, other compounds of the present invention were assayed as well, and results similar to those of DM122 were obtained.

### Test Example 4: Toxicity of compounds on Huh7.5 cells

Huh7.5 cells (purchased from Vertex Pharmaceutical Co., USA) were digested with pancreatin containing EDTA, then formulated into 1×10⁵ cells/ml and inoculated in an amount of 0.ml to 96-well culture plate, placed in an incubator with 37°C, 5% CO₂ content, and saturation humidity, and cultured for 6h, added with different drug solutions (DM122) formulated with culture medium, and cell control was set. Cultivation was continuously performed for 96 h in the incubator with 37°C, 5%CO₂ content, and saturation humidity. MTT staining method was used to determine the toxicity of compound to cells. The survival rate (%) of cells under different drug concentrations were calculated in comparison with the normal cell without drug medication. Tthe results showed the compounds had less toxicity to cells (see: Fig.3). Similar methods were used to assay other compounds and the results similar to those of DM122 were obtained.

### Test Example 5: Inhibition effects of compound on Hsc70 in Huh7.5 cultured cell

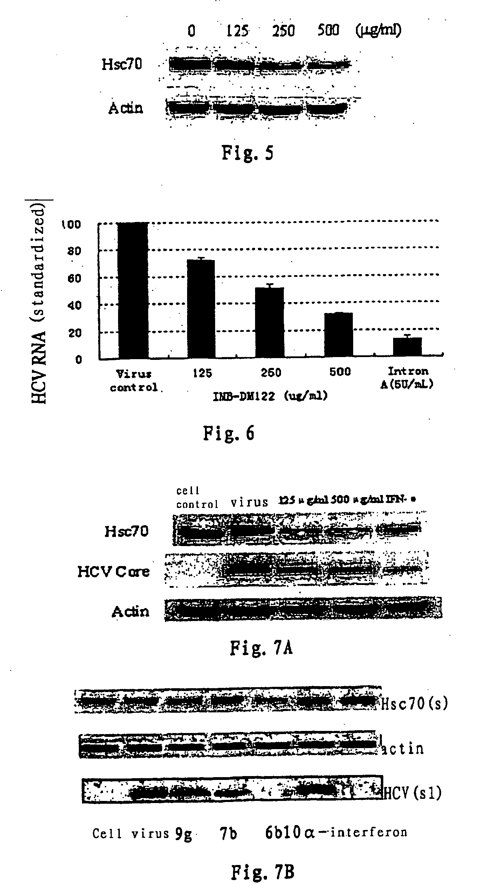

2×10⁵/ml Huh7.5 cells (purchased from Vertex Pharmaceutical Co., USA) were inoculated in an amount of 3ml on 6-well culture plate, placed in an incubator with 37°C, 5%CO₂ content, saturation humidity, and cultured for 24h, added with different drug solutions (DM122) formulated with culture medium, and cell control was set. Cultivation was continuously performed in the incubator with 37°C, 5%CO₂ content, saturation humidity for 24h, a kit for RNA extraction was used to extract intracellular RNA from the cells, and one-step qRT-PCR was used to determine intracellular contents of Hsc70 and GAPDHRNA. After the cultivation of cells was performed for 48h, the cells were digested and harvested, then the cells were split with CytoBuster™ Protein Extraction Buffer that contained many kinds of protease inhibitors, total intracellular proteins were extracted, and Western blotting was used to analyze the contents of Hsc70 and GAPDH proteins. In comparison with the cell control, the inhibition effects on Hsc70 upon action of different drugs were analyzed. The results show dose-dependent inhibition effects of compound on intracellular Hsc70 mRNA in Huh7.5 cultured cell upon the action of compound (the results are shown in Fig.4), and dose-dependent inhibition effects on intracellular Hsc70 protein as well (the results are shown in Fig.5). This suggests that the target of compound is Hsc70.

By using similar method, other compounds of the present invention were assayed, and the results similar to those of DM122 were obtained as well.

### Test Example 6: Inhibition effects of compounds on intracellular HCV RNA in Huh7.5 cultured cell after HCV infection

1×10⁵/ml Huh7.5 cells (purchased from Vertex Pharmaceutical Co., USA) were inoculated in an amount of 0.1ml on 96-well culture plate, placed in an incubator with 37°C, 5%CO₂ content and saturation humidity, and cultured for 24h, HCV virus in 45IU/cell was used to infect Huh7.5, different drug solutions (DM122) formulated with culture medium were added, and cell control and HCV infection control were set. Cultivation was continuously performed in the incubator with 37°C, 5%CO₂ content and saturation humidity for 72h, kit for RNA extraction was used to extract intracellular RNA, one-step qRT-PCR was used to determine intracellular contents of HCV and GAPDH RNA, and the inhibition effects of drug on HCV infection was analyzed. The results show dose-dependent effects on intracellular HCV RNA in Huh7.5 cultured cell after HCV infection (the results are shown in Fig.6).

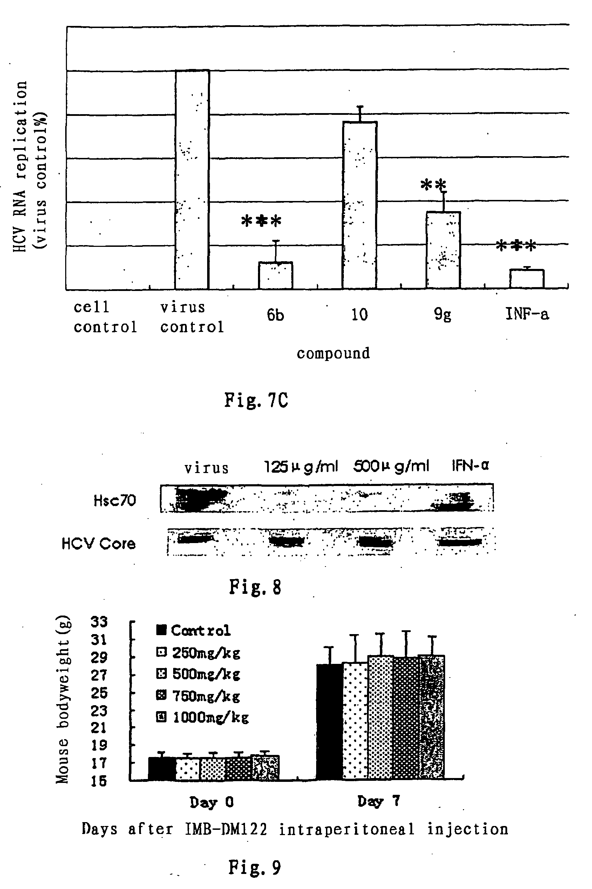

1×10⁵/ml Huh7.5 cells were inoculated in an amount of 3ml on 6-well culture plate, placed in an incubator with 37°C, 5%CO₂ content and saturation humidity, and cultured for 24h, HCV virus solution in 45IU/cell was used to infect Huh7.5, then different drug solutions (DM122) formulated with culture medium were added, and cell control and HCV infection control were set. Cultivation was continuously performed in the incubator with 37°C, 5%CO₂ content and saturation humidity for 72h, the cells were harvested, then the cells were split with CytoBuster™ Protein Extraction Buffer that contained many kinds of protease inhibitors, total intracellular proteins were extracted, and Western blotting was used to analyze the contents of HCV Core, Hsc70 and GAPDH proteins. In comparison with the virus control group, the inhibition effects on HCV infection upon action of different drugs were analyzed. The results showed dose-dependent inhibition effects on intracellular HCV Core and Hsc70 proteins in Huh7.5 cultured cell after HCV infection, and the reduction of HCV Core protein was consistent with the reduction of Hsc70 protein (the results are shown in Fig.7A).

The above results confirm at nucleic acid level and protein level, Compound DM122 has good anti-HCV effect in Huh7.5 cultured cell.

By using similar assay method, other compounds of the present invention were assayed, and the results similar to those of DM122 were obtained.

Compounds (6b, 7b, 9f, 9g, 10) were emphatically assayed in their in vivo activity on anti-HCV. Specifically, HCV-infected Huh7.5 cells were administered with compounds (6b, 7b, 9f, 9g, 10, wherein 6b was referred to 13-ethoxy matrine, 7b was referred to 13-methylaminomatrine,9g was referred to 14-hydroxyl-13-2-chloropropionyloxy matrine, 10 was referred to 14-methoxysophocarpine) in a concentration of 200 µg/mL, for 96 h. The HCV nucleoprotein level and Hsc70 protein level in positive control Huh7.5 cells were determined with α-interferon in advance. Huh7.5 cells were infected with HCV for 24 h, the drug (200µg/mL) was medicated, and after 48 h, intracellular HCV nucleoprotein level and Hsc70 protein level were determined by Western blotting method, and HCV RNA level was evaluated by real-time RT-PCT. This experiment was repeated for 3 times, * indicated p < 0.5 in comparison with control, ** indicated p < 0.01 in comparison with control.

The results were shown in Fig.7B, α-interferon was capable of inhibiting HCV replication, but was not via Hsc70 inhibition mechanism. Compounds 6b and 9g had significant activity on anti-HCV via down-regulation of Hsc70.

The results of protein levels were further confirmed with HCV RNA level, as shown in Fig.7B, Compound 6b had best activity against HCV, which was equivalent to that of α-interferon and which mechanism was via down-regulation of Hsc70 expression.

### Test Example 7: Inhibition effects of compounds on HCV Core protein in virus particles in supernatant from HCV-infected cultured cell liquid

Huh7.5 cells were inoculated in an amount of 3×10⁴/cm², cultured in 10cm culture dish (58.1cm²/well) for 6h, infected with HCV and simultaneously added with DM-122 drug solutions with different concentration and positive control Intron A, after action for 96h, the supernatant from cultured cell liquid was subjected to ultra-high speed centrifugation to separate virus particles, Western blotting was used to analyze the contents of Hsc70 and GAPDH proteins in virus particles. The results showed Compound DM122 reduced the Hsc70 protein content in virus particles in supernatant from Huh7.5 cultured cell liquid after HCV infection (the results were shown in Fig.8), and thus Compound DM122 reduced reduced the infection efficiency of virus.

Other compounds of the present invention were assayed by similar method, and similar results were obtained as well.

### Test Example 8: Acute toxicity of compound in mice

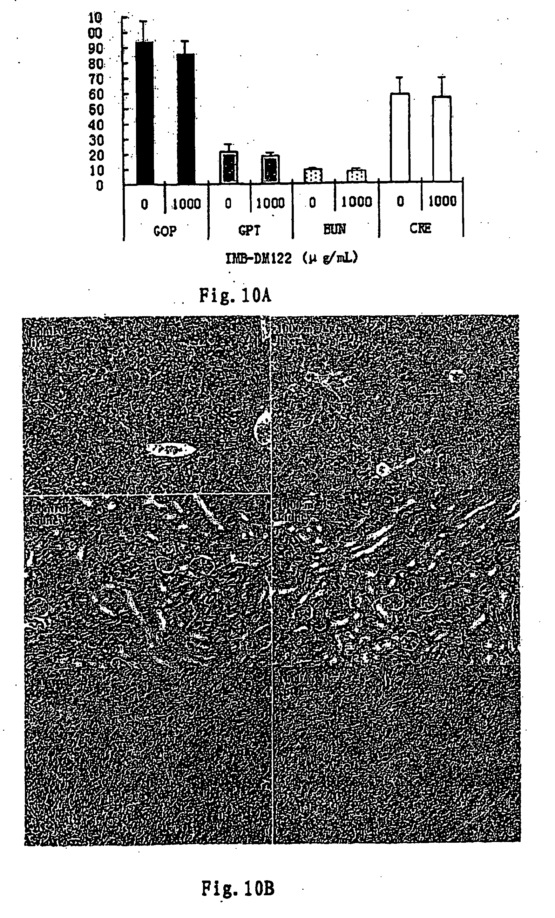

Kunming mice (purchased from Institute of Animal, Chinese Academy of Medical Sciences),18-20 g, were weighed and randomly divided in groups, 10 mice per group, half male and half female, intraperitoneally injected with DM122 solution, in concentration of 0, 250mg/kg, 500mg/kg and 1000mg/kg once. The death of animals was observed. On the 7^{th} day, the animals were weighed, blood samples were taken and functional indexes (GOT, GPT, BUN and CRE) of liver and kidney in blood were determined. The results showed the compound in various concentrations had no influence on body weight of mice (the results were shown in Fig.9), and had no influence on liver and kidney functions at the highest dose (the results were shown in Fig.10A, 10B), which indicated that the compound had good safety and had not significant toxic and side effects.

By using the same method, other compounds of the present invention were evaluated on safety, and their results were similar to those of DM122.

The safety of 13-methoxy matrine (6b) was emphatically monitored.

Since Compound 6b had relatively high activity on anti-HBV and anti-HCV. The inventors had used it as a candidate of anti-hepatitis drug to evaluate its safety in animal body. Kunming mice were used for test of safety and acute toxicity of Compound 6b (13-methoxy matrine), via oral administration, administration doses were separately 250, 500 and 750 mg/kg once, then the mice were strictly monitored for 7 days.

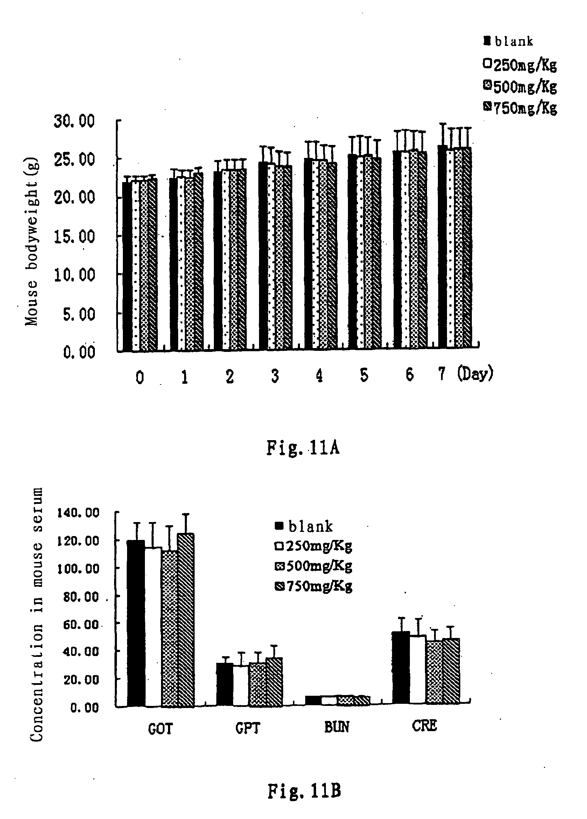

The results showed that during the test period of 14 days, no mouse died, which indicated that 6b had an oral LD₅₀ greater than 1000 mg/kg; and no significant change of bodyweight was observed in mice as well, and the results were shown in Fig.11A. In the last phase of test, blood samples were taken for assays of liver and kidney functions, and even in 750 mg/kg dose group, no significant abnormality was observed in AST, ALT, BUN and CRE indexes in blood (Fig.11B). Hence, Compound 6b was safe in body.

### Test Example 9: Inhibition effects on wild type HBV and drug resistant type HBV

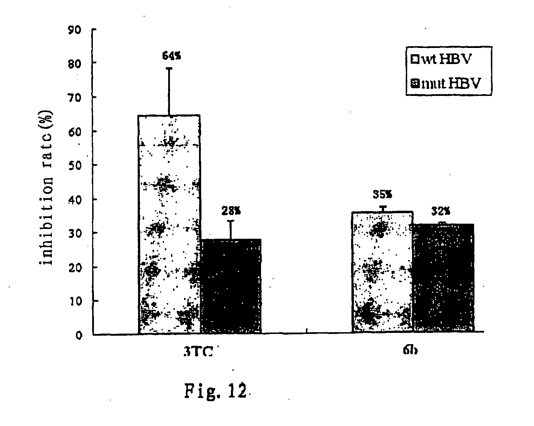

Lamivudine-resistant strain (LRS M204V + L180M) was used to transfect Huh-7.5 cells, lamivudine was used as positive control, lamivudine (0.16µg/mL) and compound 6b(37µg/mL) were separately used to treat wild type and LRS type HBV, after 36 h, Real-time PCR was used to determine HBV DNA levels, respectively, and the results were shown in Fig.12.

The results showed: lamivudine had an inhibition rate of 64% on wild type HBV, and merely an inhibition rate of 28% on drug-resistant HBV, while Compound 6b has equivalent inhibition rates on wild type HBV and drug-resistant HBV(35% vs 32%). This indicated that Compound 6b was effective to both wild type and drug-resistant HBV, and was identical to OMTR.

Other compounds of the present invention were subjected to the same test, and similar results were obtained, but all of them were inferior to Compound 6b.

In order to exhibit the specific activities of various compounds, Table 3 gave the test results of a lot of single compound.

**Table 3**

| No | Code | Hsc70 down-regulation rate (%) | HCV inhibition rate (%) | EC50 (µg/ml) | CC50 (µg/ml) | SI |
|---|---|---|---|---|---|---|
| | DM-122 | 36.8±1.4 | 77.2±4.8 | 252.35 | >1000 | >3.96 |
| | | - | 88.0±4.0 | | | |
| 1 | DM-162 | - | 96.2±0.3 | 34.06 | 431.6 | 12.67 |
| | | - | 99.1±0.1 | | | |
| 2 | DM-151 | - | 20.5±3.2 | | | |
| | | 11.5+2.4 | 53.4±5.6 | | | |
| 3 | DM-103 | 74.8±5.6 | 69.1±14.5 | 135.71 | >1000 | >7.37 |
| | | - | 91.4±1.8 | | | |
| 4 | L6 | 16.7±5.0 | 11.1±1.1 | | | |
| | | - | 74.4±7.9 | | | |
| 5 | DM-140 | 53.8±7.6 | 83.4±1.8 | 18.68 | >1000 | >53.5 |
| | | - | 96.0±1.2 | | | |
| 6 | DM-139 | 20.5±24.8 | 88.5±4.8 | 98.65 | 885.1 | 8.97 |
| | | - | 98.1±1.0 | | | |
| 7 | DM-138 | 34.8±2.1 | 91.5±7.4 | 82.49 | 418.3 | 5.07 |
| | | 21.1±12.0 | 95.1±2.9 | | | |
| 8 | DM-153 | 38.6±15.8 | 47.8±9.4 | | | |
| | | 32.6±14. | 29.2±29.5 | | | |
| 9 | L7 | 63.3±1.2 | 89.5±2.0 | 291.73 | >1000 | >3.42 |
| | | - | 95.7±1.1 | | | |
| 10 | DM-132 | 80.1±5.7 | 67.7±5.8 | 52.86 | 903.2 | 17.09 |
| | | 32.8±26.8 | 82.7±5.6 | | | |
| 11 | DM-135 | 0.1±5.6 | 74.3±8.2 | 29.86 | 620.68 | 20.8 |
| | | - | 90.5±2.0 | | | |
| 12 | DM-133 | - | 96.4±0.8 | 67.33 | 527.8 | 7.84 |
| | | - | 98.6±0.8 | | | |
| 13 | DM-123 | 65.9±6.9 | 74.0±7.8 | 104.61 | 455.7 | 4.36 |
| | | 70.5±2.3 | 81.1±6.6 | | | |
| 14 | L13 | - | 40.0±9.4 | | | |
| | | - | 74.9±5.6 | | | |
| 15 | DM-137 | - | 99.8±0.1 | <12.34 | 471.59 | >38.2 |
| | | - | 99.7±0.0 | | | |
| 16 | DM-121 | - | | | | |
| | | - | 14.9±9.0 | | | |
| 17 | L9 | 20.1±11.1 | 62.5±0.3 | | | |
| | | - | 68.9±3.8 | | | |
| 18 | DM-143 | 1.1 | | 12.46 | 374.5 | 30.06 |
| 19 | DM-146 | 0.92 | | 63.99 | >1000 | >15.6 |
| 20 | DM-126 | 0.62 | | 21.11 | >1000 | >47.4 |
| 21 | DM-124 | 0.32 | | 68.27 | 504.6 | 7.39 |
| 22 | DM-125 | -67.97 | | 34.35 | >1000 | >29.1 |
| 23 | DM-127 | -2.92 | | 113.64 | 427.8 | 3.76 |
| 24 | DM-129 | 0.27 | | 192.02 | 245.5 | 1.28 |
| 25 | DM-128 | -5.78 | | 51.66 | 535.1 | 10.36 |
| 26 | DM-136 | 0.76 | | 1.80 | 70.49 | 39.2 |
| 27 | DM-134 | 0.57 | | 52.18 | >1000 | >19.16 |
| 28 | DM-131 | -0.86 | | 89.31 | >1000 | >11.2 |
| 29 | DM-152 | | | 125.65 | >1000 | >7.96 |
| 30 | DM-154 | | | >333.33 | >1000 | 3 |
| 31 | DM-155 | | | >333.33 | >1000 | 3 |
| 32 | L3 | 78.9±1.4 | 52.5±4.4 | | | |
| | | 53.8±8.6 | 84.1±7.9 | | | |
| 33 | L2 | 76.6±2.5 | 27.9±16.3 | | | |
| | | 32.7±5.0 | 5.3±5.1 | | | |
| 34 | L17 | | | 76.79 | >1000 | >13 |
| 35 | DM-148 | | | >333.3 | 529.86 | <1.6 |
| 36 | DM-144 | | | 31.8 | >1000 | >31.4 |
| 37 | DM-145 | | | 117.2 | >1000 | >8.5 |
| 38 | L11 | 31.4±12.2 | 46.7±12.7 61.6±3.9 | | | |
| | | - | | | | |
| 39 | SC-84a | | | 7.54 | 530.07 | 70.3 |
| 40 | SC-89b | | | 3.98 | 123.08 | 30.9 |

| | | | | | | |
|---|---|---|---|---|---|---|
| Notation: EC50 in activity data was refered to half effective concentration, CC50 was refered to the concentration to cause poisoning in half cells, SI value was selectivity index and was a value of CC50/EC50; in the column of HCV inhibition rate, the upper data was inhibition rate at concentration of 200ug/ml, and the lower data was inhibition rate at concentration of 400ug/ml. In the column of Hsc70 inhibition rate, when there was two data, the upper data was inhibition rate at concentration of 200ug/ml, and the lower data was inhibition rate at concentration of 400ug/ml, "-"represents no significant inhibition rate, and blank without data meant the activity test was not performed (nd). | | | | | | |

In sum, the inventors find that the compounds of other Examples of the present invention can achieve results similar to those of DM122 or 6b in the above experiments. Oxymatrine merely has moderate activity against hepatitis B with large clinical dose, and low bioavailability in oral administration. The compounds of the present invention have significantly better activity against hepatitis B than oxymatrine, and theire activities against hepatitis C and ADIS viruses are also found. In addition, the compounds of the present invention are not easily metabolized and have high bioavailability, and thus their oral doses can be significantly reduced.

## Claims

1. A compound of Formula (I) or Formula (II), or a pharmaceutically acceptable salt, geometric isomer, stereoisomer, solvate, ester or prodrug thereof, 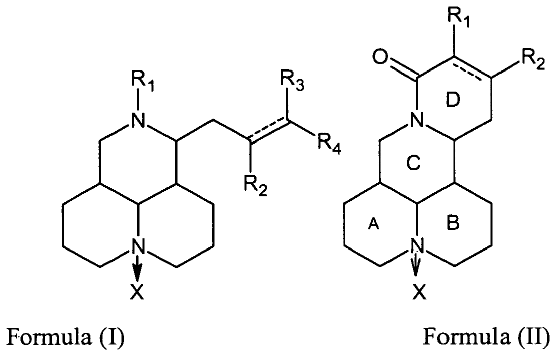 wherein:
X is unsubstituted or O;
R₁ is selected from
(1) C₁₋₆ alkyl-CO-, C₂₋₆ alkenyl-CO-, aryl-C₁₋₆ alkyl-CO-, aryl-C₂₋₆ alkenyl-CO-, aryloxy-C₁₋₆ alkyl-CO-, or aryloxy-C₂₋₆ alkenyl-CO-, wherein the alkyl, alkenyl and aryl are optionally substituted with 1-3 groups independently selected from the group consisting of: hydroxyl, halogen, nitro and C₁₋₆ alkyl,
(2) C₁₋₆ alkyl-SO₂- or aryl-SO₂-, wherein the alkyl and aryl is optionally substituted with 1-3 groups independently selected from the group consisting of: hydroxyl, halogen, nitro and C₁₋₆ alkyl,
(3) one or two C₁₋₆ alkyl, or one or two C₂₋₆ alkenyl, wherein the alkyl and alkenyl are optionally substituted with 1-3 groups independently selected from the group consisting of: hydroxyl, halogen and C₁₋₆ alkyl,
(4) aryl-C₁₋₆ alkyl-, or heteroaryl-C₁₋₆ alkyl-, wherein the aryl and heteroaryl are optionally substituted with 1-3 groups independently selected from the group consisting of: C₁₋₆ alkyloxy, nitro, halogen, cyano, C₁₋₆alkyl, C₂₋₆ alkenyl, and wherein the ring of the heteroaryl contains 1 or 2 heteroatoms selected from N, O and S,
(5) aryl, which is optionally substituted with 1-3 groups independently selected from the group consisting of: C₁₋₆ alkyl, nitro, halogen and cyano,
(6) C₁₋₁₂ alkyl, C₅₋₁₂ aryl, C₂₋₁₂ alkenyl, C₂₋₁₂ alkynyl, C₇₋₁₂ alkenylaryl, C₇-C₁₂ alkynylaryl, or C₆-C₁₂alkylaryl, which is optionally substituted with 1-3 groups independently selected from the group consisting of: hydroxyl, C₁₋₆ alkyloxy and halogen;
R₂ represents 1 or 2 groups selected from the group consisting of: -H, hydroxyl, C₁₋₁₂ alkyloxy, C₁₋₁₂ alkyl optionally substituted with nitro or amino, and C₂₋₁₂ ester group;
if present, R₃ represents 1 or 2 groups selected from the group consisting of: -H, hydroxyl, C₁₋₁₂ alkyloxy and C₂₋₁₂ ester group;
if present, R₄ is selected from the group consisting of: -COOH, -CHO, -CH=NOH, -CH=N-NH₂, hydroxyl-C₁₋₆ alkyl-, C₂-C₁₂ ester group, C₂-C₁₂ acylamino, C₂-C₁₂ ether group and C₂-C₁₂ oxime ether group;
--------- represents single bond or double bond.

2. The compound of claim 1, which is a compound of Formula (Ia), or a pharmaceutically acceptable salt, geometric isomer, stereoisomer, solvate, ester or prodrug thereof, 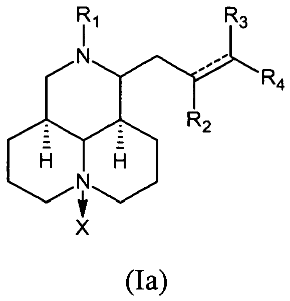 wherein:
X, R₁, R₂, R₃ and R₄ have same definitions as those of Formula (I) in claim 1.

3. The compound of claim 1, which is a compound of Formula (Ib), or a pharmaceutically acceptable salt, geometric isomer, stereoisomer, solvate, ester or prodrug thereof, 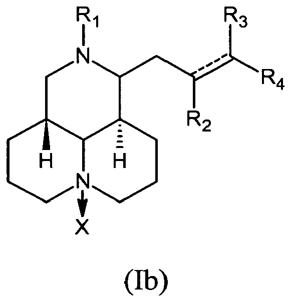 wherein:
X, R₁, R₂, R₃ and R₄ have same definitions as those of Formula (I) in claim 1.

4. The compound according to any one of claims 1-3, **characterized by** any one or more of the following items (a) to (h):
(a) X is unsubstituted;
(b) X is oxygen;
(c) R₁ is selected from :
(1) C₁₋₆ alkyl-CO-, C₂₋₆ alkenyl-CO-, aryl-C₁₋₆ alkyl-CO-, aryl-C₂₋₆ alkenyl-CO-, aryloxy-C₁₋₆ alkyl-CO-, or aryloxy-C₂₋₆ alkenyl-CO-, wherein the alkyl, alkenyl and aryl are optionally substituted with 1-3 groups independently selected from the group consisting of: hydroxyl, halogen, nitro and C₁₋₆ alkyl,
(2) C₁₋₆ alkyl-SO₂-, or aryl-SO₂-, wherein the alkyl and aryl are optionally substituted with 1-3 groups independently selected from the group consisting of: hydroxyl, halogen, nitro and C₁₋₆ alkyl,
(3) one or two C₁₋₆ alkyl, or one or two C₂₋₆ alkenyl, wherein the alkyl and alkenyl are optionally substituted with 1-3 groups independently selected from the group consisting of: hydroxyl, halogen and C₁₋₆ alkyl,
(4) aryl-C₁₋₆ alkyl-, or heteroaryl-C₁₋₆ alkyl-, wherein the aryl and heteroaryl are optionally substituted with 1-3 groups independently selected from the group consisting of: C₁₋₆ alkyloxy, nitro, halogen, cyano, C₁₋₆ alkyl, C₂₋₆ alkenyl, and wherein the ring of the heteroaryl contains 1 or 2 heteroatoms selected from N, O and S,
(5) aryl, which is optionally substituted with 1-3 groups independently selected from the group consisting of: C₁₋₆ alkyl, nitro, halogen and cyano,
(6) C₁₋₁₂ alkyl, C₅₋₁₂ aryl, C₂₋₁₂ alkenyl, C₂₋₁₂ alkynyl, C₇₋₁₂ alkenylaryl, C₇-C₁₂ alkynylaryl, or C₆-C₁₂alkylaryl, which is optionally substituted with 1-3 groups independently selected from the group consisting of: hydroxyl, C₁₋₆ alkyloxy and halogen;
(d) R₂ represents 1 or 2 groups selected from the group consisting of: -H, hydroxyl, C₁₋₁₂ alkyloxy, C₁₋₁₂ alkyl optionally substituted with nitro or amino, and C₂₋₁₂ ester group;
(e) if present, R₃ represents 1 or 2 groups selected from the group consisting of: -H, hydroxyl, C₁₋₁₂ alkyloxy and C₂₋₁₂ ester group;
(f) if present, R₄ is selected from the group consisting of: -COOH, hydroxyl-C₁₋₆ alkyl-, C₂-C₁₂ ester group, C₂-C₁₂ acylamino, C₂-C₁₂ alcohol group, C₂-C₁₂ ether group, C₂-C₁₂ aldehyde group, C₂-C₁₂ hydrazone group, C₂-C₁₂ oxime group and C₂-C₁₂ oxime ether group;
(g) --------- represents single bond;
(h) --------- represents double bond.

5. The compound according to any one of claims 1-4, wherein:
X is unsubstituted or O;
R₁ is selected from
(1) C₁₋₄ alkyl-CO-, C₂₋₄ alkenyl-CO-, phenyl-C₁₋₄ alkyl-CO-, phenyl-C₂₋₄ alkenyl-CO-, phenoxy-C₁₋₄ alkyl-CO-, or phenoxy-C₂₋₄ alkenyl-CO-, wherein the alkyl, alkenyl and phenyl are optionally substituted with 1-3 groups independently selected from the group consisting of: hydroxyl, halogen, nitro and C₁₋₄ alkyl,
(2) C₁₋₄ alkyl-SO₂-, or phenyl-SO₂-, wherein the alkyl and phenyl are optionally substituted with 1-2 groups independently selected from the group consisting of: hydroxyl, halogen, nitro and C₁₋₄ alkyl,
(3) one or two C₁₋₄ alkyl, or one or two C₂₋₄ alkenyl, wherein the alkyl and alkenyl are optionally substituted with 1-2 groups independently selected from the group consisting of: hydroxyl, halogen and C₁₋₄ alkyl,
(4) phenyl-C₁₋₄ alkyl-, or heteroaryl-C₁₋₆ alkyl-, wherein the phenyl and heteroaryl are optionally substituted with 1-2 groups independently selected from the group consisting of: C₁₋₄ alkyloxy, nitro, halogen, cyano, C₁₋₄ alkyl, C₂₋₄ alkenyl, and wherein the heteroaryl is selected from pyridyl and thiazolyl,
(5) phenyl, which is optionally substituted with 1-2 groups independently selected from the group consisting of: C₁₋₄ alkyl, nitro, halogen and cyano,
(6) C₁₋₆ alkyl, C₅₋₈ aryl, C₂₋₆ alkenyl, C₂₋₆ alkynyl, C₂₋₆ alkenylaryl-, C₂-C₆ alkynyl- aryl-, or C₁-C₆ alkyl- aryl-, which is optionally substituted with 1-3 groups independently selected from the group consisting of: hydroxyl, C₁₋₄ alkyloxy and halogen;
R₂ represents 1 or 2 groups selected from the group consisting of: -H, hydroxyl, C₁₋₆ alkyloxy, C₁₋₆ alkyl optionally substituted with nitro or amino, and C₂₋₆ ester group;
if present, R₃ represents 1 or 2 groups selected from the group consisting of: -H, hydroxyl, C₁₋₆ alkyloxy and C₂₋₆ ester group;
if present, R₄ is selected from the group consisting of: -COOH, -CHO, -CH=NOH, -CH=N-NH₂, hydroxyl-C₁₋₆ alkyl-, C₂-C₁₂ ester group, C₂-C₁₂ acylamino, C₂-C₁₂ ether group and C₂-C₁₂ oxime ether group;
--------- represents single bond or double bond.

6. The compound according to any one of claims 1-4, wherein:
X is unsubstituted or O;
R₁ is selected from
(1) C₁₋₄ alkyl-CO-, C₂₋₄ alkenyl-CO-, phenyl-C₁₋₄ alkyl-CO-, phenyl-C₂₋₄ alkenyl-CO-, phenoxy-C₁₋₄ alkyl-CO-, or phenoxy-C₂₋₄ alkenyl-CO-, wherein the alkyl, alkenyl and phenyl are optionally substituted with 1-2 groups independently selected from the group consisting of: hydroxyl, halogen, nitro and C₁₋₄ alkyl,
(2) C₁₋₄ alkyl-SO₂-, or phenyl-SO₂-, wherein the alkyl and phenyl are optionally substituted with 1-2 groups independently selected from the group consisting of: hydroxyl, halogen, nitro and C₁₋₄ alkyl,
(3) one or two C₁₋₄ alkyl, or one or two C₂₋₄ alkenyl, wherein the alkyl and alkenyl are optionally substituted with 1-2 groups independently selected from the group consisting of: hydroxyl, halogen and C₁₋₄ alkyl,
(4) phenyl-C₁₋₄ alkyl-, or heteroaryl-C₁₋₆ alkyl-, wherein the phenyl and heteroaryl are optionally substituted with 1-2 groups independently selected from the group consisting of: C₁₋₄ alkyloxy, nitro, halogen, cyano, C₁₋₄ alkyl, C₂₋₄ alkenyl, and wherein the heteroaryl is selected from pyridyl and thiazolyl,
(5) phenyl, which is optionally substituted with 1-2 groups independently selected from the group consisting of: C₁₋₄ alkyl, nitro, halogen and cyano;
R₂ represents 1 or 2 groups selected from the group consisting of: -H, hydroxyl, C₁₋₄ alkyloxy, C₁₋₄ alkyl optionally substituted with nitro or amino, and C₂₋₄ ester group;
if present, R₃ represents 1 or 2 groups selected from the group consisting of: -H, hydroxyl, C₁₋₄ alkyloxy and C₂₋₄ ester group;
if present, R₄ is selected from the group consisting of: -COOH, -CHO, -CH=NOH, -CH=N-NH₂, hydroxyl-C₁₋₆ alkyl-, C₂-C₁₂ ester group, C₂-C₁₂ acylamino, C₂-C₁₂ ether group and C₂-C₁₂ oxime ether group;
--------- represents single bond or double bond.

7. The compound of claim 1, which is a compound of following Formula (Ia-1): 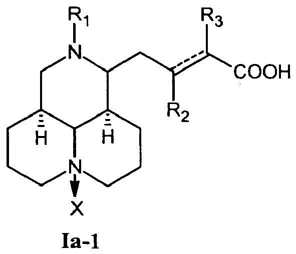 or a pharmaceutically acceptable salt, geometric isomer, stereoisomer, solvate, ester or prodrug thereof, wherein:
R₁ is selected from
(1) C₁₋₄ alkyl-CO-, C₂₋₄ alkenyl-CO-, phenyl-C₁₋₄ alkyl-CO-, phenyl-C₂₋₄ alkenyl-CO-, phenoxy-C₁₋₄ alkyl-CO-, or phenoxy-C₂₋₄ alkenyl-CO-, wherein the alkyl, alkenyl and phenyl are optionally substituted with 1-2 groups independently selected from the group consisting of: hydroxyl, halogen, nitro and C₁₋₄ alkyl,
(2) C₁₋₄ alkyl-SO₂-, or phenyl-SO₂-, wherein the alkyl and phenyl are optionally substituted with 1-2 groups independently selected from the group consisting of: hydroxyl, halogen, nitro and C₁₋₄ alkyl,
(3) one or two C₁₋₄ alkyl, or one or two C₂₋₄ alkenyl, wherein the alkyl and alkenyl are optionally substituted with 1-2 groups independently selected from the group consisting of: hydroxyl, halogen and C₁₋₄ alkyl,
(4) phenyl-C₁₋₄ alkyl-, or heteroaryl-C₁₋₆ alkyl-, wherein the phenyl and heteroaryl are optionally substituted with 1-2 groups independently selected from the group consisting of: C₁₋₄ alkyloxy, nitro, halogen, cyano, C₁₋₄ alkyl, C₂₋₄ alkenyl, and wherein the heteroaryl is selected from pyridyl and thiazolyl,
(5) phenyl, which is optionally substituted with 1-2 groups independently selected from the group consisting of: C₁₋₄ alkyl, nitro, halogen and cyano;
R₂ represents 1 or 2 groups selected from the group consisting of: -H, hydroxyl, C₁₋₄ alkyloxy, C₁₋₄ alkyl optionally substituted with nitro or amino, and C₂₋₄ ester group;
R₃ represents 1 or 2 groups selected from the group consisting of: -H, hydroxyl, C₁₋₄ alkyloxy and C₂₋₄ ester group;
R₄ is selected from the group consisting of: -COOH, -CHO, -CH=NOH, -CH=N-NH₂, hydroxyl-C₁₋₆ alkyl-, C₂-C₁₂ ester group, C₂-C₁₂ acylamino, C₂-C₁₂ ether group and C₂-C₁₂ oxime ether group;
--------- represents single bond or double bond.

8. The compound of claim 1, which is a compound of following Formula (Ia-2): 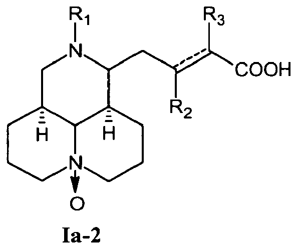 or a pharmaceutically acceptable salt, geometric isomer, stereoisomer, solvate, ester or prodrug thereof, wherein:
R₁ is selected from
(1) C₁₄ alkyl-CO-, C₂₋₄ alkenyl-CO-, phenyl-C₁₋₄ alkyl-CO-, phenyl-C₂₋₄ alkenyl-CO-, phenoxy-C₁₋₄ alkyl-CO-, or phenoxy-C₂₋₄ alkenyl-CO-, wherein the alkyl, alkenyl and phenyl are optionally substituted with 1-2 groups independently selected from the group consisting of: hydroxyl, halogen, nitro and C₁₋₄ alkyl,
(2) C₁₋₄ alkyl-SO₂-, or phenyl-SO₂-, wherein the alkyl and phenyl are optionally substituted with 1-2 groups independently selected from the group consisting of: hydroxyl, halogen, nitro and C₁₋₄ alkyl,
(3) one or two C₁₋₄ alkyl, or one or two C₂₋₄ alkenyl, wherein the alkyl and alkenyl are optionally substituted with 1-2 groups independently selected from the group consisting of: hydroxyl, halogen and C₁₋₄ alkyl,
(4) phenyl-C₁₋₄ alkyl-, or heteroaryl-C₁₋₆ alkyl-, wherein the phenyl and heteroaryl are optionally substituted with 1-2 groups independently selected from the group consisting of: C₁₋₄ alkyloxy, nitro, halogen, cyano, C₁₋₄ alkyl, C₂₋₄ alkenyl, and wherein the heteroaryl is selected from pyridyl and thiazolyl,
(5) phenyl, which is optionally substituted with 1-2 groups independently selected from the group consisting of: C₁₋₄ alkyl, nitro, halogen and cyano;
R₂ represents 1 or 2 groups selected from the group consisting of: -H, hydroxyl, C₁₋₄ alkyloxy, C₁₋₄ alkyl optionally substituted with nitro or amino, and C₂₋₄ ester group;
R₃ represents 1 or 2 groups selected from the group consisting of: -H, hydroxyl, C₁₋₄ alkyloxy and C₂₋₄ ester group;
R₄ is selected from the group consisting of: -COOH, -CHO, -CH=NOH, -CH=N-NH₂, hydroxyl-C₁₋₆ alkyl-, C₂-C₁₂ ester group, C₂-C₁₂ acylamino, C₂-C₁₂ ether group and C₂-C₁₂ oxime ether group;
--------- represents single bond or double bond.

9. The compound according to any one of claims 1-8, wherein R₁ is selected from the groups as shown in Table 1 and Table 2 in the specification, or is selected from following groups:
ClCH₂CO-, CH₃CO-, OHCH₂CO-, CH₃CHOHCO-, (*m*-CH₃C₆H₄O)CH₂CO-, C₆H₅CH=CHCO-, CH₃(*m*-CH₃C₆H₄O)CH₂CO-, C₆H₅CO-, *m*-NO₂C₆H₄CO-, 2-CH₃-5-NO₂C₆H₃CO-, *p*-CH₃C₆H₄SO₂-, C₆H₅SO₂-, CH₃SO₂-, CH₃CH₃⁺-, CH₃CH₂-, CH₃CH₂CH₂-, (CH₂CH₂CH)CH₂-, (CH₂cH₂CH)CH₂-, OHCH₂CH₂-, C₆H₅CH₂-, *p*-CH₃OC₆H₄CH₂-, *p*-CH₃OC₆H₄CH₂-, *p*-NO₂C₆H₄CH₂-, *o*-ClC₆H₄CH₂-, *m*-ClC₆H₄CH₂-, *p*-ClC₆H₄CH₂-, 3-Cl-4-ClC₆H₃CH₂-, *p*-BrC₆H₄CH₂-, 2-Cl-4-ClC₆H₃CH_{2,}*p*-FC₆H₄CH₂-, *m*-FC₆H₄CH₂-, *p*-CNC₆H₄CH₂-, *o*-FC₆H₄CH₂-, (*p*-CH₂=CH)C₆H₄CH₂-, *m*-NO₂C₆H₄CH₂-, *o*-CH₃C₆H₄CH₂-, *m*-CH₃C₆H₄CH₂-, *p*-CH₃C₆H₄CH₂-, *m*-CH₃OC₆H₄CH₂-, 2-F-4-BrC₆H₃CH₂-, 2-C₅H₄NCH₂-, 4-C₃H₂SNCH₂-, CH₃CH₂CH₂-, CH₃CH₂CH₂-, CH₃CH₂CH₂CH₂CH₂-, CH₃CH₂CH₂CH₂CH₂-, *p*-NO₂C₆H₅-, 2-CH₃C₆H₅-, 2-CH₃C₆H₅-, 4-BrC₆H₅-, 4-BrC₆H₅-, 3,5-(CH₃)₂C₆H₅-, *p*-CNC₆H₅-, *p*-CNC₆H₅-.

10. The compoundaccording to any one of claims 1-9, which is selected from:
N-acetyl matrinic acid;
N-p-tosyl matrinic acid;
N-chloroacetyl matrinic acid;
N-(2-m-methylphenoxy)acetyl matrinic acid;
N-(2-hydroxyl)acetyl matrinic acid;
N-(2-hydroxyl)propionyl matrinic acid;
N-(2-m-methylphenoxy)propionyl matrinic acid;
N-benzoyl matrinic acid;
N-(3-nitrobenzoyl) matrinic acid;
N-(2-methyl-5-nitrobenzoyl) matrinic acid;
N-benzyl matrinic acid;
N-benzyl oxy matrinic acid;
N-benzenesulfonyl matrinic acid;
N-cinnamoyl matrinic acid;
N,N'-dimethyl matrinic acid;
N,N'-dimethyl oxymatrinic acid;
N-ethyl matrinic acid;
N-propyl matrinic acid;
N-cyclopropylmethyl matrinic acid;
N-cyclopropylmethyl oxymatrinic acid;
N-hydroxylethyl matrinic acid;
N-(4-methoxybenzyl) matrinic acid;
N-(4-methoxybenzyl) oxymatrinic acid;
N-(4-nitrobenzyl) matrinic acid;
N-(2-chlorobenzyl) matrinic acid;
N-(3-chlorobenzyl) matrinic acid;
N-(4-chlorobenzyl) matrinic acid;
N-(3,4-dichlorobenzyl) matrinic acid;
N-(4-bromobenzyl) matrinic acid;
N-(2,4-dichlorobenzyl) matrinic acid;
N-(4-fluorobenzyl) matrinic acid;
N-(3-fluorobenzyl) matrinic acid;
N-(4-cyanobenzyl) matrinic acid;
N-(2-fluorobenzyl) matrinic acid;
N-(4-ethenylbenzyl) matrinic acid;
N-(3-nitrobenzyl) matrinic acid;
N-(2-methylbenzyl) matrinic acid;
N-(3-methylbenzyl) matrinic acid;
N-(4-methylbenzyl) matrinic acid;
N-(3-methoxy)benzyl matrinic acid;
N-(2-pyridinylbenzyl) matrinic acid;
N-(3-pyridinylbenzyl) matrinic acid;
N-(4-pyridinylbenzyl) matrinic acid;
N-(4-thiazolylbenzyl) matrinic acid;
N-(1-naphthylbenzyl) matrinic acid;
N-(4-methylbenzoyl) matrinic acid;
N-(4-methoxybenzoyl) matrinic acid;
N-(4-cyanobenzoyl) matrinic acid;
N-(4-fluorobenzoyl) matrinic acid;
N-(4-trifluoromethylbenzoyl) matrinic acid;
N-p-methoxybenzenesulfonyl matrinic acid;
N-(4-trifluoromethoxybenzyl) matrinic acid;
sophocarpinic acid;
N-benzoyl sophocarpinic acid;
N-benzyl sophocarpinic acid;
N-acetyloxy sophocarpinic acid;
N-propyl sophocarpinic acid;
N-(2-fluoro-4-bromobenzyl) sophocarpinic acid;
N-pivaloyl sophocarpinic acid;
N-pentyl sophocarpinic acid;
N-(4-cyanobenzyl) sophocarpinic acid;
N-(4-nitrobenzyl) sophocarpinic acid;
N-(2-methylbenzyl) sophocarpinic acid;
N-(4-bromobenzyl) sophocarpinic acid;
N-(4-trifluoromethylbenzyl) sophocarpinic acid;
N-(p-trifluoromethylbenzenesulfonyl) sophocarpinic acid;
N-(m-cyanobenzenesulfonyl) sophocarpinic acid;
N-benzyl-13-hydroxyl matrinic acid;
N-4-cyanobenzyl-13-hydroxyl matrinic acid;
N-2-methylbenzyl-13-hydroxyl matrinic acid;
N-4-bromobenzyl-13-hydroxyl matrinic acid;
N-3, 5-dimethylbenzyl-13-hydroxyl matrinic acid;
N-4-trifluoromethylbenzyl-13-hydroxyl matrinic acid;
N-acetyl-(5R)-matrinic acid;
N-tert-butyryl-(5R)-matrinic acid;
N-bromoacetyl-(5R)-matrinic acid;
N-formylethyl ester group-(5R)-matrinic acid;
N-formylmethyl ester group-(5R)-matrinic acid;
N-formylbenzyl ester group-(5R)-matrinic acid;
N-benzenesulfonyl-(5R)-matrinic acid;
N-p-tosyl-(5R)-matrinic acid;
N-benzoyl(5R)-matrinic acid;
N-(3-nitrobenzoyl)-(5R)-matrinic acid;
N-(p-methylbenzoyl)-(5R)-matrinic acid;
N-(p-methoxybenzoyl)-(5R)-matrinic acid;
N-(p-fluorobenzoyl)-(5R)-matrinic acid;
N-(2-methyl-5-nitrobenzoyl) -(5R)-matrinic acid;
N-(3-nitro-4-fluorobenzoyl) -(5R)-matrinic acid;
N-(3-nitro-4-methoxybenzoyl)-(5R)-matrinic acid,
13-hydroxyl matrine;
13-methoxy matrine;
13-benzyloxy matrine;
13-ethoxy matrine;
13-benzoyloxy matrine;
13-nitromethyl matrine;
13-methylaminomatrine;
13,14-dihydroxyl matrine;
13,14-dimethoxy matrine;
13,14-dibenzyloxy matrine;
13,14-diacetyloxy matrine;
13,14-di(4-fluoro-3-nitro) benzoyloxy matrine;
14-hydroxyl-13-acetyloxy matrine;
14-hydroxyl-13-chloroacetyloxy matrine;
14-hydroxyl-13-2-chloropropionyloxy matrine;
14-hydroxyl-13-benzoyloxy matrine;
14-hydroxyl-13-(4-fluoro-3-nitro)benzoyloxy matrine;
14-hydroxyl-13-acetyloxy matrine;
14-methoxysophocarpine,
or a pharmaceutically acceptable salt, geometric isomer, stereoisomer, solvate, ester or prodrug thereof.

11. A method for preparing the compound of any one of claims 1-10, comprising the following steps:
(1) in an aqueous solution of alkaline, subjecting a compound of Formula (ia): 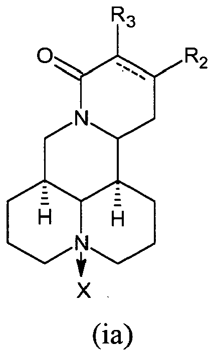 to refluxing with stirring for 2-20 h, then reacting at 10-40°C (for example, 15-35°C, such as room temperature) for a time (for example 5-30 h, such as 5-15 h), regulating pH value with an acid to 4-7 (for example 5-6), concentrating to dryness, to obtain an acid of Formula (ii): 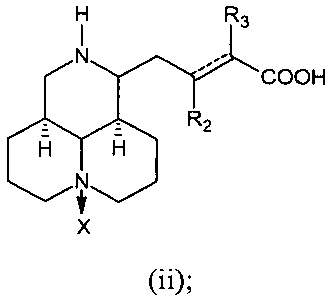
(2) in an organic solvent (e.g., petroleum ether, for example a petroleum ether having a boiling range of 60-90°C), reacting benzophenone hydrazone with yellow mercury oxide at 10-40°C (for example 15-35°C, such as room temperature) for a time (for example 2-20 h, such as 5-15 h), to obtain a solution of diphenyldiazomethane;
(3) mixing a solution of the acid of Formula (ii) in a solvent (for example alcohol, such as methanol) with the solution obtained in step (2), reacting the mixture solution at 10-40°C (for example 15-35°C, such as room temperature), to obtain an ester of Formula (iii): 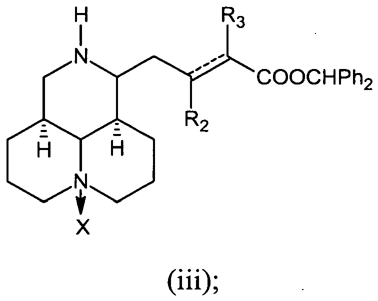
(4) in the presence of an alkaline (for example, alkali metal hydroxide or alkali metal carbonate, such as potassium hydroxide, potassium carbonate), reacting the ester of Formula (iii) with a compound represented by Formula R₁-Y at 10-40°C (for example 15-35°C, such as room temperature) for a time (for example 1-50 h, such as 2-30 h), to obtain a compound of Formula (iv): 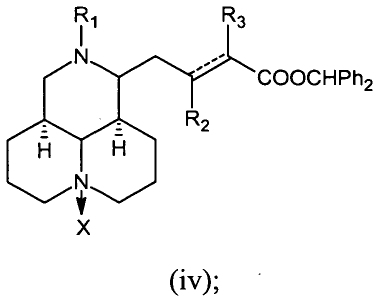
(5) in the presence of metacresol, reacting the compound of Formula (iv) at 70-90°C (for example, about 80°C) for a time (for example, 2-20 h, such as 5-15 h), to obtain a compound of Formula (Ia) 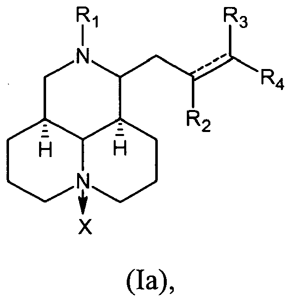 wherein Y represents halogen(for example, fluorine, chlorine, bromine or iodine), R₁, R₂, R₃, R₄, X and bond --------- have the same meanings as mentioned in any one of claims 1-9.

12. A pharmaceutical composition, comprising a therapeutically and/or prophylactically effective amount of the compound or a pharmaceutically acceptable salt, geometric isomer, stereoisomer, solvate, ester or prodrug thereof according to any one of claims 1-10, and optionally one or more pharmaceutically acceptable carriers or excipients.

13. Use of the compound or a pharmaceutically acceptable salt, geometric isomer, stereoisomer, solvate, ester or prodrug thereof according to any one of claims 1-10 or the pharmaceutical composition according to claim 12 in manufacture of a medicament for treatment and/or prophylaxis of a disease or disorder associated with viral infection.

14. The use according to claim 13, wherein the disease or disorder associated with viral infection is selected from inflammatory liver diseases (for example, hepatitis B, hepatitis C, hepatitis A) and AIDS.

15. Use of the compound or a pharmaceutically acceptable salt, geometric isomer, stereoisomer, solvate, ester or prodrug thereof according to any one of claims 1-10 or the pharmaceutical compositionaccording to claim 12 in manufacture of Hsc70 inhibitor.

16. A method for inhibition of Hsc70, treatment and/orprophylaxis of a disease or disorder associated with viral infection, the method comprising administering a subject in need an therapeutically and/or prophylactically effective amount of the compoundor a pharmaceutically acceptable salt, geometric isomer, stereoisomer, solvate, ester or prodrug thereof according to any one of claims 1-10 or the pharmaceutical composition according to claim 12.

17. The methodaccording to claim 17, wherein the disease or disorder associated with viral infection is selected from hepatitis B, hepatitis C, hepatitis A and AIDS.
